(19) Europäisches Patentamt
European Patent Office
Office européen des brevets

(11) EP 3 514 545 B1

(12) EUROPEAN PATENT SPECIFICATION

(45) Date of publication and mention
of the grant of the patent:
07.10.2020 Bulletin 2020/41

(51) Int Cl.:
$G01N\ 33/574^{(2006.01)}$        $G01N\ 33/92^{(2006.01)}$

(21) Application number: 18152687.2

(22) Date of filing: 22.01.2018

(54) **A METHOD OF DIAGNOSING PANCREATIC CANCER BASED ON LIPIDOMIC ANALYSIS OF A BODY FLUID**

VERFAHREN ZUR DIAGNOSE VON BAUCHSPEICHELDRÜSENKREBS AUF BASIS VON LIPIDOMISCHER ANALYSE EINER KÖRPERFLÜSSIGKEIT

PROCÉDÉ DE DIAGNOSTIC DU CANCER DU PANCRÉAS BASÉ SUR L'ANALYSE LIPIDOMIQUE D'UN FLUIDE CORPOREL

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(43) Date of publication of application:
24.07.2019 Bulletin 2019/30

(73) Proprietor: Univerzita Pardubice
532 10 Pardubice (CZ)

(72) Inventors:
• Holcapek, Michal
533 52 Staré Hradisté (CZ)
• Cifkova, Eva
756 63 Krhova (CZ)
• Lisa, Miroslav
535 01 Bezdekov (CZ)
• Jirasko, Robert
530 02 Pardubice (CZ)
• Wolrab, Denise
1030 Wien (AT)
• Hrnciarová, Tereza
140 00 Praha 4 (CZ)

(74) Representative: Hartvichova, Katerina et al
HARBER IP s.r.o.
Dukelskych hrdinu 567/52
170 00 Praha 7 (CZ)

(56) References cited:
US-A1- 2012 202 188    US-A1- 2013 109 592

• P. G. LOKHOV ET AL: "Mass spectrometry analysis of blood plasma lipidome as the method of disease diagnostics, evalution of effectiveness and optimization of drug therapy", BIOCHEMISTRY (MOSCOW). SUPPLEMENT SERIES B: BIOMEDICAL CHEMISTRY, vol. 9, no. 2, 1 April 2015 (2015-04-01), pages 95-105, XP055455969, RU ISSN: 1990-7508, DOI: 10.1134/S1990750815020109
• LÍSA MIROSLAV ET AL: "Lipidomic analysis of biological samples: Comparison of liquid chromatography, supercritical fluid chromatography and direct infusion mass spectrometry methods", JOURNAL OF CHROMATOGRAPHY A, vol. 1525, 8 October 2017 (2017-10-08), pages 96-108, XP085274447, ISSN: 0021-9673, DOI: 10.1016/J.CHROMA.2017.10.022
• Michal Holcapek: "Clinical lipidomic quantiation based on mass spectrometry: case study of pancreatic cancer", , 24 January 2018 (2018-01-24), XP055455975, Retrieved from the Internet: URL:https://www.ilmexhibitions.com/htc/abs tract/Clinical+Lipidomic+Quantitation+Base d+on+Mass+Spectrometry:+Case+Study+of+Pa nc reatic+Cancer/503/ [retrieved on 2018-03-02]

**Description**

Field of Art

The present invention relates to a method of diagnosing pancreatic cancer based on lipidomic analysis of a body fluid which is suitable for high throughput screening with a high selectivity and specificity.

Background Art

Pancreatic cancer belongs among the most lethal tumors with the lowest survival rate of all cancers. It is expected to become the second leading cause of cancer-related death in the US as well as Europe by the year 2020. Pancreatic cancer is very hard to be diagnosed at early stages. The pancreas is deep inside the body, so early tumors cannot be seen or felt by health care providers during routine physical examinations. People usually have no symptoms until the cancer has already spread to other organs. If people are at an increased risk of pancreatic cancer due to genetic predispositions or show signs and symptoms that can be associated with this disease, specific examinations and tests can be done. Imaging tests are most often performed in case of serious suspicion of pancreatic cancer: computed tomography, magnetic resonance imaging, abdominal or endoscopic ultrasound, cholangiopancreatography, somatostatin receptor scintigraphy, positron emission tomography, or angiography. Screening tests or examinations may be used to look for a disease in people who have no symptoms, and who have not had that disease before. However, the imaging tests used for patients with an increased risk or a suspicion of pancreatic cancer are not applicable for large-scale population screening, and moreover imaging tests may have sensitivity limitations in the detection of tumors at an early stage. For a conclusive diagnosis of pancreatic cancer, percutaneous, endoscopic, or surgical biopsy can be done, but again these invasive methods are not applicable for routine population screening.

So far, several types of blood tests have been considered to diagnose pancreatic cancer, such as carbohydrate antigen 19-9 (CA19-9), carcinoembryonic antigen (CEA), or Kirsten-ras (KRAS), but unfortunately low sensitivity and low specificity do not allow their use in the clinical practice for early diagnosis. No screening tests have so far been developed that would allow the detection of sufficiently early stages to lower the risk of dying from pancreatic cancer. Reliable detection of early stage pancreatic cancer is urgently needed, but at this time, no major professional groups recommend any routine screening for pancreatic cancer in people at risk.

Lipidomic analysis using older semi-quantitative hydrophilic liquid chromatography approach was applied for breast tumor tissues [E. Cífková, M. Holčapek, M. Lísa, D. Vrána, J. Gatek, B. Melichar, Anal. Bioanal. Chem. 407 (2015) 991-1002. Determination of lipidomic differences between human breast cancer and surrounding normal tissues using HILIC-HPLC/ESI-MS and multivariate data analysis] and breast tumor cell lines [E. Cífková, M. Lísa, R. Hrstka, D. Vrána, J. Gatek, B. Melichar, M. Holčapek, Rapid Commun. Mass Spectrom. 31 (2017) 253-263. Correlation of lipidomic composition of cell lines and tissues of breast cancer patients using hydrophilic interaction liquid chromatography - electrospray ionization mass spectrometry and multivariate data analysis] compared to surrounding normal tissues or normal cell lines, respectively, and it suggests that cancer induced lipidomic changes of tumor cells and tissues can be used for differentiation of tumors from normal tissues. These studies did not relate to body fluids. Two first attempts to differentiate pancreatic cancer patients from healthy volunteers have been presented using SM 38:2 and other lipids [US 2013/0140452] or a combination of selected lipids, metabolites, and protein CA 19-9 [WO 2016/207391], but unfortunately the selectivities and sensitivities achieved in these documents (significantly less than 90 % for samples with known classification; no data provided for samples with unknown classification) do not reach the level required for high-throughput screening programs of pancreatic cancer, so the presented approaches are not yet applicable for reliable and high-throughput population screening of pancreatic cancer.

US 2012/0202188 proposes to diagnose pancreatic cancer by analyzing a sample from the patient by high resolution mass spectrometry to obtain accurate mass intensity data, comparing the data to corresponding data obtained from one or more reference samples to identify an increase or decrease in accurate mass intensity, and using this increase or decrease for diagnosing pancreatic cancer. Internal standards are used in this document, but they are added only before the step of mass spectroscopy. The data are also not evaluated separately based on gender.

Until now, an effective method for high-throughput early diagnosis screening programs with a sufficiently high selectivity and sensitivity has not been proposed.

The principal problem of body fluid analysis is that early stage tumor may have too small impact on the dysregulation of monitored metabolites and lipids, which may result in the situation that the effect of biological variability is larger than the effect on studied cancer type, and then no statistically relevant differences can be discovered in body fluids.

The present invention aims at overcoming the problems and challenges of the current state of the art, and to provide a method of diagnosing pancreatic cancer even in early stages, said method being usable for routine high-throughput population screening.

## Summary of the Invention

The present invention provides a method of diagnosing pancreatic cancer based on lipidomic analysis of a serum taken from the body of a human patient, characterized in that it comprises the steps of:

- spiking of the sample with a set of internal standards comprising at least one internal standard for each lipid class present in the sample,
- subsequently processing the sample by liquid-liquid lipidomic extraction,
- measurement of the processed sample by a mass spectrometry method,
- determining concentrations for at least 60, preferably at least 120 or at least 230, more preferably for all lipids present at a level above detection threshold of the mass spectrometry method, using the internal standards for the corresponding lipid classes for the quantitation, or using relative concentrations of lipids, or using ratios of concentrations and/or signal intensities,

wherein the lipids for which the concentrations are determined include the following lipids:

HexCer d18:1/16:0; HexCer d18:1/15:1 (1OH)
SM 34:1
HexCer d18:1/24:1; HexCer d18:1/23:2 (1OH)
PE 34:1; PE P-35:0
PC 32:0; PC O-33:0
PE 36:4; PE P-37:3
PC 34:1; PC P-35:0
PE 34:2; PE P-35:1
PC P-34:0; PC 33:1
DG 36:2
Cer d18:1/24:1; Cer d18:1/23:2 (1OH)
CE 16:0
SM 41:1
SM 41:2
PC 34:4; PC P-35:3
HexCer d18:1/24:0; HexCer d18:1/23:1 (1OH)
PE P-36:3; PE 35:4
PE P-38:4; PE 37:5
PE P-38:5; PE 37:6
PE P-36:2; PE 35:3
SM 32:1
HexCer d18:1/22:0; HexCer d18:1/21:1 (1OH)
PE P-38:3; PE 37:4
PC P-38:2; PC 37:3
PE P-40:5; PE 39:6
PE 36:0; PE P-38:6
PE 38:0; PE P-40:6, PE O-39:0
PE P-36:3; PE 35:4
PC P-34:1; PC 33:2
PC P-36:2; PC 35:3
DG 34:3

- statistical evaluation of the determined concentrations of the lipids, said statistical evaluation determining the level of probability of the patient suffering from pancreatic cancer based on the determined lipid concentrations compared to a cancerous pattern and a non-cancerous pattern, wherein the cancerous pattern and the non-cancerous pattern are determined by statistical analysis of the lipid concentrations for a group of known cancer samples and non-cancer samples, wherein the statistical evaluation is done separately for males and females.

Preferably, the internal standards are exogenous lipid compounds, having the polar head structure typical for the relevant lipid class and containing fatty acyls with shorter chains than naturally occurring lipids, preferably chains 12:0 or 14:0, or fatty acyls with odd number of carbon atoms, preferably chains 17:0, 17:1 or 19:1, or the internal standards are isotopically labelled analogues of the lipids of the relevant lipid class, preferably D7-Chol, D7-CE 16:0; wherein the notation of lipid compounds is as follows: abbreviation of the class of the compounds, number of carbons: number of double bonds, preceded by information about isotopic labeling.

More preferably, a set of internal standards contains:

| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

wherein the notation of lipid compounds is as follows: abbreviation of the class of the compounds, number of carbons: number of double bonds, preceded by information about isotopic labeling.

The internal standards are lipid-type compounds, structurally close to the relevant lipid class. Typically, the internal standards are compounds having the structure (in particular the polar head structure) typical for the relevant lipid class but containing fatty acyls with shorter chains than naturally occurring lipids (e.g. chains 12:0 or 14:0) or fatty acyls with odd number of carbon atoms (e.g. chains 17:0, 17:1 or 19:1) or isotopically labelled analogues (e.g. D7-Chol, D7-CE 16:0).

The lipid classes are as follows:

| | |
|---|---|
| Cholesterylesters (CE) | Lysophosphatidylserines (LPS) |
| Ceramides (Cer) | Monoacylglycerols (MG) |
| Diacylglycerols (DG) | Phosphatidic acids (PA) |
| Dihexosylceramides (Hex2Cer) | Phosphatidylcholines (PC) |
| Hexosylceramides (HexCer) | Phosphatidylethanolamines (PE) |
| Cholesterol | Phosphatidylglycerols (PG) |
| Lysophosphatidic acids (LPA) | Phosphatidylserines (PS) |
| Lysophosphatidylcholines (LPC) | Sphingomyelins (SM) |
| Lysophosphatidylethanolamines (LPE) | Sulfohexosylceramides (SulfoHexCer) |
| Lysophosphatidylglycerols (LPG) | Triacylglycerols (TG) |

The notation of lipid compounds in this text is as follows: abbreviation of the class of the compounds, number of carbons: number of double bonds. The abbreviation of the class may be preceded by information about isotopic labeling, if relevant.

Due to the addition of the internal standard before sample processing, slight differences due to for instance pipetting errors can be compensated as the internal standard is affected in the same way as the target compounds. This ensures a better reliability of the quantitation of individual lipids.

Liquid-liquid lipidomic extractions are known in the art. Solvents may be used, selected from chlorinated alkanes, dialkyl ethers, alcohols, and water mixtures, which forms bilayer systems containing organic and aqueous phases. Preferably, chloroform, methyl-tert-butyl ether, methanol, ethanol, propanol, and/or butanol are used. Most preferably, chloroform - methanol - water system is used, as described e.g. in E. Cífková, M. Holčapek, M. Lísa, D. Vrána, J. Gatek, B. Melichar, Anal. Bioanal. Chem. 407 (2015) 991-1002; or in E. Cífková, M. Lísa, R. Hrstka, D. Vrána, J. Gatek, B. Melichar, M. Holčapek, Rapid Commun. Mass Spectrom. 31 (2017) 253-263. The organic phase of liquid-liquid extraction system containing lipids is collected and used for further processing. As the migration and solubility of the components of the sample are time-dependent, it is highly preferred that the extraction step is always performed in the same way for all the samples measured in one batch, in particular that the extraction is performed over the same period of time for each sample. The organic phase is typically processed by evaporation of the organic solvent and re-dissolving the residue in the solvent for mass spectrometry measurement. Suitable solvents for mass spectrometry measurements include chlorinated alkanes, alkanes and alcohols, preferably chloroform or dichloromethane, hexane and C1-C4 alcohols, most preferably a mixture of chloroform and 2-propanol (e.g., in volume ratio 1:1) or hexane : 2-propanol : chloroform 7:1.5:1.5 or chloroform - methanol - 2-propanol (1:2:4, v/v/v). Further components may be added, e.g., ammonium acetate, acetic acid, etc. Alkanes, unless defined otherwise, mean C1-C6 alkanes, preferably C1-C4 alkanes.

It is preferred to remove proteins from the sample during or after the liquid-liquid extraction, as proteins may undesirably interfere with the analysis of lipids.

The mass spectrometry method is selected from shotgun mass spectrometry, ultrahigh-performance liquid chromatography - mass spectrometry (UHPLC/MS), ultrahigh-performance supercritical fluid chromatography - mass spectrometry (UHPSFC/MS), and matrix-assisted laser desorption/ionization mass spectrometry (MALDI/MS).

In a preferred embodiment, a pooled sample is prepared by mixing identical volumes of several samples which include samples from pancreatic cancer patients and healthy volunteers, and said pooled sample is used and processed in the same way as the measured samples;

preferably the pooled sample is used for observing intra-day accuracy and intra-day precision, as well as inter-day accuracy and inter-day precision, and/or for determining the lower limit of quantitation and the upper limit of quantitation, and/or for quality control during measurements of sample set.

The method of the present invention benefits from the use of a pooled sample. A pooled sample is a sample prepared by mixing identical volumes of several samples, wherein the mixed samples include samples from pancreatic cancer patients and healthy volunteers. The ratio of samples originating from males to samples originating from females should be approximately the same as in the measured batch of samples. The term "approximately the same" refers to deviation from the ratio of male:female in the measured batch at most by 20 %, preferably at most by 10 %, more preferably at most by 5 %. The pooled sample is preferably obtained by pooling 50-100 samples.

The pooled sample, processed in the same way as any other sample, i.e., spiked with internal standard mixture and subjected to liquid-liquid extraction and any other sample processing steps, is used for the full validation and quality control during the mass spectrometry measurements. The order of samples is randomized in sample measurement sequences to avoid measurements of non-cancerous and cancerous samples in a certain portion of the measurement sequence.

The pooled sample may be used for observing intra-day accuracy and intra-day precision, as well as inter-day accuracy and inter-day precision.

Furthermore, it is advantageous to prepare test samples or pooled samples spiked with varying concentrations of the internal standard mixture.

Preferably, the order of samples is randomized in sample measurement sequences.

The limit of detection (LOD) of a lipid is preferably determined based on signal to noise ratio, e.g., such as S/N = 3.

The pooled sample spiked with varying concentrations of the internal standard mixture is used for determining the lower limit of quantitation (LLOQ) and the upper limit of quantitation (ULOQ), said limits corresponding to the first and the last points of linearity range of the calibration curve. The calibration curve is the dependence of signal on the concentration of a lipid. The linearity range of calibration curve is an interval from LLOQ to ULOQ, where the concentration of lipids can be determined.

The concentrations for all lipids present at a level above LLOQ of the mass spectrometry method are also commonly called "lipidomic profile".

The quantitation (determining concentrations) for all lipids present at a level above LLOQ of the mass spectrometry method, using the internal standards for the corresponding lipid classes for the quantitation, preferably comprises the following procedures and corrections:

- isotopic correction (deisotoping), i.e., correction of signal intensity of a lipid having due to isotopic contribution M+2 of another lipid with a molecular weight being 2 mass units lower than said lipid (e.g., isotopic interference from the lipid with one additional double bond) or due to isotopic contribution M+1 of another lipid with a molecular weight 1 mass unit lower than said lipid (e.g., interferences between PC and SM subgroups). The correction is done by subtracting calculated M+2 (or M+1) relative intensity based on known intensity in measured spectra.

- zero filling procedure in which the signals of lipid species which are not detected for particular sample, are replaced by 50 to 100 %, preferably by 70 to 100 %, more preferably by 80 to 90 % or 80 %, of the minimum concentration observed for said lipid species in all samples.

The accuracy and reliability of quantitation is ensured or improved by the following features:

- use of at least one exogenous lipid class internal standard for the quantitation of lipid species in each class,
- coelution and coionization of lipid (sub)class internal standards and analytes from the same lipid class using either lipid class separation chromatography method coupled to mass spectrometry (MS) or MS without any chromatographic separation. This co-processing is ensured by the addition of the internal standard before the sample is processed,

- use of the pooled sample spiked with the internal standards as quality control sample injected after a predetermined number of samples,
- zero filling procedure,
- isotopic correction.

These features prevent one or more of: low reproducibility, lack of robustness, signal shifts over time, ion suppression/enhancement, carry-over effects, and response change of mass spectrometer due to contamination.

When several hundreds of lipids are determined in hundreds of samples, it may happen that the expected signal is not detected for some lipid molecules due to various reasons, and then the concentration should be reported as "below LLOQ" in accordance with established analytical practice. This well-established analytical approach does not work well in case of lipidomic quantitation, and the MDA based on such approach would provide poor resolution of healthy / disease groups, and may not provide acceptable sensitivity and selectivity for the detection of pancreatic cancer or other disease. This approach is adapted to cases in which all missing signals correspond to the situation that the true concentration of the analyte is below LLOQ, for example the determination of low number of analytes well separated by chromatography. However, the situation in lipidomic quantitation is very different, because quantify ca. 150-400 lipids per sample are quantified and many of them (or all in case of shotgun) are co-eluting, which may result in the absence of signal due to other reasons than the concentration below LLOQ, mainly due to ion suppression or other reasons causing the signal drop, including the system contamination, mobile phase impurity, bleeding of chromatographic column, etc. The complexity of lipidomic analysis is enormous, and it is a natural situation that some signals are not properly recorded and processed regardless of the best analytical practice and careful analytical work. Therefore, we have developed a new zero filling approach where the values which are missing for any reason are replaced by 75 to 85 %, preferably by 80%, of the minimum concentration observed for this lipid species in all samples. This approach significantly improves the quality of subsequent statistical analysis. Our preliminary results show that when the zero-filling approach is neglected, the quality of cancer detection is significantly worse or impossible. Afterwards the average and the standard deviation of the duplicates are calculated.

The statistical evaluation is preferably performed by multivariate data analysis (MDA). The MDA methods may be non-supervised statistical methods such as principal component analysis (PCA) or supervised statistical methods such as orthogonal projections to latent structures discriminant analysis (OPLS-DA).

The "cancerous pattern" means the pattern of lipid concentrations typical for samples of a body fluid obtained from patients suffering from pancreatic cancer. The term "cancerous sample" or "cancer sample" refers to the sample of a body fluid obtained from patients suffering from pancreatic cancer.

The "non-cancerous pattern" means the pattern of lipid concentrations typical for samples of a body fluid obtained from healthy volunteers, i.e., from subjects not suffering from pancreatic cancer. The term "non-cancerous sample" or "non-cancer sample" refers to a sample of a body fluid obtained from healthy volunteers, i.e., from subjects not suffering from pancreatic cancer.

Preferably, the statistical evaluation involves

- data pre-processing such as centering, scaling, and/or transformation. The centering correlates the changes relative to the average of the data set. The scaling unifies the impact of different concentration ranges on the model (UV and Pareto scaling) so that low concentrations (maybe equally important) are not masked by very abundant concentrations. The logarithmic transformation assembles the data to the normal distribution. A proper pre-processing has a significant influence on the quality of results.
- PCA analysis used for identification of outliers, measurement failures as well as other influential factors. The PCA analysis may also visualize differences between groups as well as cluster the quality control (QC) samples in comparison to other samples.
- discrimination analysis (OPLS-DA) for group separation of pancreatic cancer patients and healthy volunteers. The discrimination analysis is performed for males and females separately.
- assignment of the statistical parameters as well as the evaluation of the prediction power.

The statistical methods are first used to build statistical models (cancerous patterns and non-cancerous patterns) based on lipidomic profiles of body fluids of healthy volunteers and diagnosed pancreatic cancer patients. These statistical models are then used for the determination of the level of probability of the patient suffering from pancreatic cancer based on the determined lipid concentrations compared to a cancerous pattern and a non-cancerous pattern.

The sample throughput of the methodology of the invention is at least 4000 samples per year and one mass spectrometry apparatus at current level of automation of standard mass spectrometry labs. Known methods of multiplexing and higher automation may further increase the sample throughput.

Brief description of Drawings

Figure 1. OPLS-DA statistical model for males from UHPSFC/MS and shotgun data, labeled by tumor stage.
Figure 2. OPLS-DA statistical model for females from UHPSFC/MS and shotgun data, labeled by tumor stage

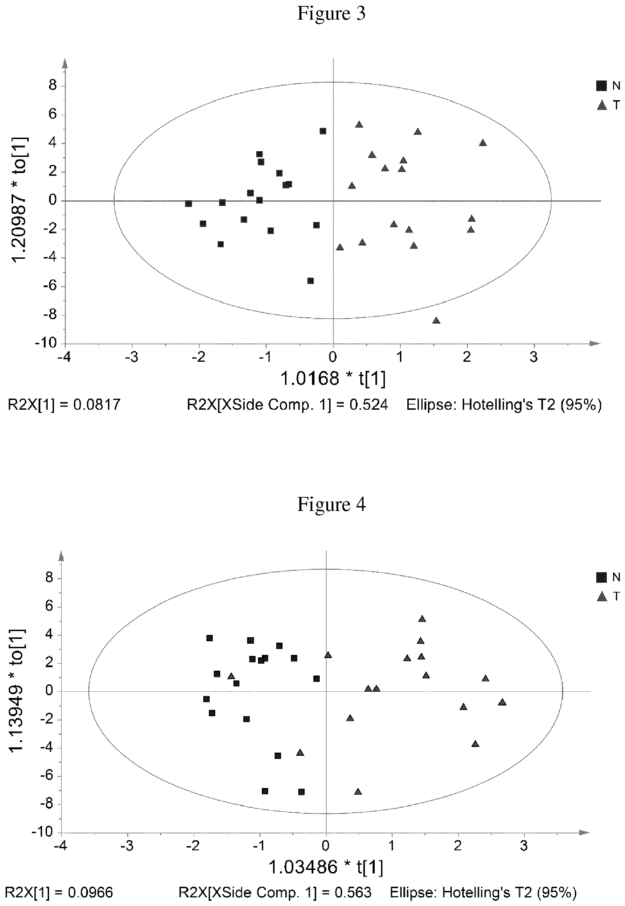

Figure 3. OPLS-DA statistical model for males from MALDI data.
Figure 4. OPLS-DA statistical model for females from MALDI data.

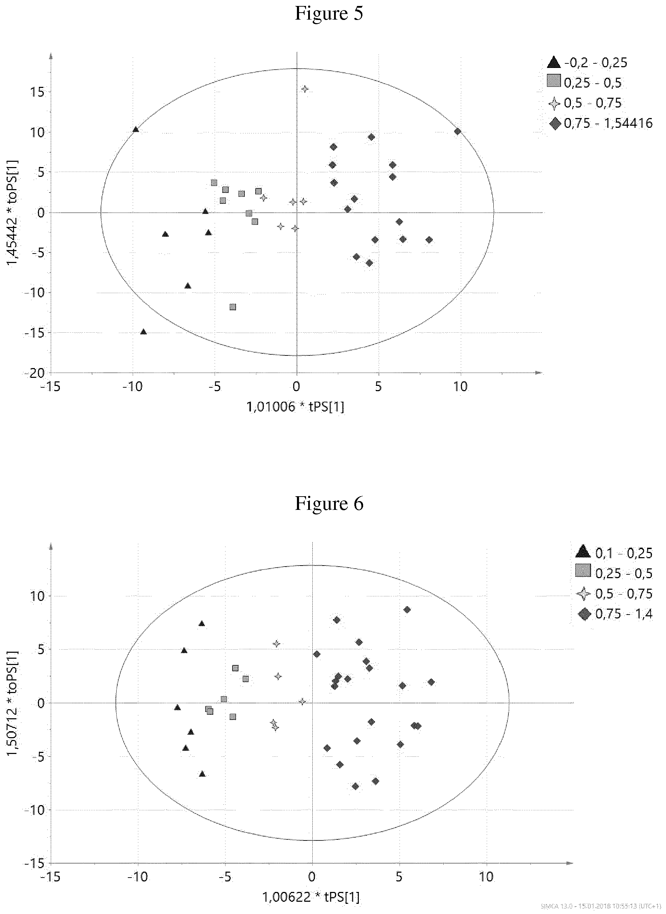

Figure 5. OPLS-DA statistical model prediction of being pancreatic cancer patient for females from UHPSFC/MS and shotgun data for unknown samples.
Figure 6. OPLS-DA statistical model prediction of being pancreatic cancer patient for males from UHPSFC/MS and shotgun data for unknown samples.

Detailed description of the Invention

*Sample collection:*

Human body fluid samples of pancreatic cancer patients and healthy volunteers are collected in the hospital. The blood of human subjects is collected in the standard and well established way used in hospitals into EDTA tubes, then serum is isolated using standardized protocols well known to a person skilled in the art, storage at -80°C at the clinic, transport from the clinic to the analytical laboratory using biological transport bags with dry ice (-20°C), storage again at -80°C until the analysis.

*Preparation of a set of internal standards (mixture of internal standards):*

Generally, the internal standard mixture is used for quantitation of lipid species. The internal standard for each lipid class behaves in the same way as the target compounds belonging to this lipid class. Due to the addition of the internal standard before sample processing, slight differences due to for instance pipetting errors can be compensated as the internal standard is affected in the same way as the target compounds.

In a particular embodiment, 2-4 mg of each standard is weighed into 2 mL HPLC glass vials using an analytical balance and dissolved in corresponding volume (chloroform : 2-propanol - 2:8) in order to obtain a final concentration of 2, 2.1 or 0.25 $\mu$g/$\mu$L. Standards for each lipid class are mixed together to form internal standard mixtures as described in Table 2 for all herein shown types of MS analysis.

Preferably, one mixture of internal standards is prepared in a sufficient amount for the whole experiment, in order to avoid variances in quantitation due to slight differences in concentration of the internal standards when the mixture is prepared in several batches. Aliquots may be prepared of this internal standard mixture and stored at -20°C.

Table 2. Preparation of internal standard mixtures

| Internal Standard | Stock concentration [$\mu$g/$\mu$L] | UHPSFC/MS and MALDI-MS for serum samples V [$\mu$L] | Shotgun MS for serum samples V [$\mu$L] |
|---|---|---|---|
| D7-CE 16:0 | 2 | 71.4 | 85 |
| Cer d18:1/12:0 | 2 | 3 | 2.5 |
| DG 12:1/12:1 | 2 | 71.4 | 5 |
| Hex2Cer d18:1/12:0 | 2 | - | 5 |
| HexCer d18:1/12:0 | 2.1 | - | 5 |
| D7-Chol | 2 | 142.8 | 170 |
| LPC 17:0 | 2.1 | 12.6 | 15 |
| LPE 14:0 | 2 | 4.2 | 5 |
| LPG 14:0 | 2.1 | - | 10 |
| LPS 17:1 | 2.1 | - | 5 |
| LPA 14:0 | 2.1 | - | 5 |
| MG 19:1 | 2 | 40.74 | 10 |
| PA 14:0/14:0 | 2 | - | 5 |

(continued)

| Internal Standard | Stock concentration [$\mu$g/$\mu$L] | UHPSFC/MS and MALDI-MS | Shotgun MS |
| --- | --- | --- | --- |
| | | for serum samples V [$\mu$L] | for serum samples V [$\mu$L] |
| PC 14:0/14:0 | 2 | 21 | 25 |
| PE 14:0/14:0 | 2 | 2.52 | 5 |
| PG 14:0/14:0 | 2 | - | 2.5 |
| PS 14:0/14:0 | 2 | - | 5 |
| SM d18:1/12:0 | 2 | 12.6 | 15 |
| SulfoHexCer d18:1/12:0 | 0.25 | 4.2 | 2.5 |
| TG 19:1/19:1/19:1 | 2 | 12.6 | 170 |
| Solvent: Chloroform: 2-propanol 2:8 (v/v) | | 867.24 | 632.5 |
| Total Volume | | 1266.3 | 1507.5 |

*Sample processing:*

All samples are spiked before the extraction with the appropriate internal standard mixture (depending on which mass spectrometry method is used).

The lipidomic extractions were performed by well-established procedures using chloroform - methanol - water extraction systems as published in our previous works [E. Cífková, M. Holčapek, M. Lísa, D. Vrána, J. Gatek, B. Melichar, Anal. Bioanal. Chem. 407 (2015) 991-1002; E. Cífková, M. Lísa, R. Hrstka, D. Vrána, J. Gatek, B. Melichar, M. Holčapek, Rapid Commun. Mass Spectrom. 31 (2017) 253-263]. The order of samples for extractions is randomized.

25 $\mu$L of serum and 17.5 $\mu$L of internal standard mixture and 2 mL chloroform (2x 1 mL) and 1 mL methanol are transferred into a glass vial (4 mL). Biological samples contain also proteins, which may lead to sticky and slimy samples. It must be ensured that the pipette draws the 25 $\mu$L of serum and is not blocked with the slimy components during drawing. The glass vials are closed and put for 10 min into the ultrasonic bath at 40°C for homogenization. Afterwards, the samples are allowed to reach room temperature in order to ensure no evaporation of the organic solvents when opening the vials. 600 $\mu$L of water are added to each sample. The samples are closed and vortexed for 1 min. It is important to do the extraction step always in the same way for all samples of one batch (one study). The target lipids for mass spectrometry are better soluble in the organic layer (bottom layer). Afterwards, the samples are centrifuged for 3 min at 3000 rpm in order to separate the organic and aqueous layer (in between these two layers a protein layer in the form of white precipitate is formed). The aqueous layer (upper layer) is removed via a glass pipette. The organic solvent containing the target compounds is evaporated under a stream of nitrogen at 30 °C and the glass vials containing the residue (target compounds) are stored at -80°C.

*Sample preparation for UHSFC/MS analysis*

Before analysis, the samples are allowed to reach ambient temperature (so that they cannot draw water from the air when opening the vials) and then the residue from the extraction and evaporation (previous step) is dissolved in 500 $\mu$L chloroform : 2-propanol (1:1, v/v). It is advisable to prepare enough solvent mixture of chloroform : 2-propanol (1:1, v/v) mixture for all samples to avoid variations between individual batches of the solvent mixture. The samples are vortexed carefully for 1 min to ensure that the target compounds are dissolved. The solution is filtered using a 0.2 $\mu$m syringe filter in order to get rid of undissolved components, which may block the UHPSFC column and compromise the analysis. 475 $\mu$L of hexane : 2-propanol: chloroform 7:1.5:1.5 is transferred into an HPLC vial and 25 $\mu$L of the diluted sample is added. Close the vials containing the filtrate with normal PTFE caps and store them at -80°C. Close the diluted sample vials with slit caps for analysis with UHPSFC/MS and place them in the autosampler.

*Sample preparation for shotgun MS analysis*

Lipid residues from the extraction and evaporation with appropriate internal standard mixtures are diluted 10 times depending on the samples by chloroform - methanol - 2-propanol (1:2:4, v/v/v) mixture containing 7.5 mmol/L of ammonium acetate and 1% of acetic acid.

*Sample preparation for MALDI-MS analysis*

MALDI matrix 9-aminoacridine (Sigma-Aldrich, St. Louis, MO, USA) is dissolved in methanol - water mixture (4:1, v/v) to provide the concentration of 5 mg/ml and mixed with particular diluted lipid extracts (1:1, v/v). The deposited amount of extract/matrix mixture is 1 $\mu$l and the dried droplet crystallization is used for the sample deposition on the target plate. The deposition of small aliquot of chloroform on MALDI plate spots before the application of diluted extract/matrix mixture is applied to avoid the drop spreading.

*Mass spectrometry (MS) method development and validation*

Three major MS based methods for lipidomic quantitation were developed in particular, which are described in more detail here. The method benefits from the use of pooled sample, which is a mixture of identical volumes of all samples for smaller studies with less than 100 subjects. In this study, the pooled sample is prepared from equal volumes of 60 randomly selected pancreatic cancer patients and healthy volunteers samples, keeping the ratio of males and females in the same proportion as in the sample set. The pooled sample is used for the method development and optimization. The pooled sample with added internal standard mixture per each lipid class to be quantified is used for the full validation and QC during the measurements. The order of samples is always randomized in sample sequences to avoid measurements of non-cancerous and cancerous samples in certain portion of sequence.

The system suitability test was carried out before the validation procedure at three concentration levels typically reported as low, medium and high concentration levels. All concentration levels must be within the linear dynamic range. The low concentration level is close to the lower limit of quantitation (LLOQ), the middle concentration level is in the middle of the linear dynamic range, and the high concentration level is close to the upper limit of quantitation (ULOQ). In a particular embodiment, we use 5, 17.5, and 30 $\mu$l of IS mixture prepared according to Table 2 for low, medium and high concentration levels, respectively. Validation parameters such as selectivity, accuracy, precision, calibration curve, limits of detection and quantitation, matrix effect, carry-over and stability were determined. Individual parameters were determined for IS representing properties of the lipid class. The selectivity was determined using 3 extracts of the pooled serum sample spiked before extraction with the IS mixture at low, middle and high concentration level and 6 extracts of appropriate non-spiked serum samples. The accuracy and precision were studied using the pooled serum sample spiked after the extraction at low, medium and high concentration levels. The intra-day accuracy and intra-day precision were studied using three samples per concentration level. The inter-day accuracy and inter-day precision were evaluated among three independent runs on two different days using three samples at the low, medium and high concentration level. The LLOQ and ULOQ corresponded to the first and the last points of linearity range, respectively.

The limit of detection (LOD) was determined based on signal to noise ratio ($S/N = 3$) observed from reconstructed ion chromatogram or neutral loss (NL) and precursor ion (PI) mass spectra (shotgun MS) of internal standard mixture. The extraction recovery was determined by calculating the ratio of the signal response of samples spiked before and after extraction for low, medium and high concentration. The process efficiency was determined by calculating the ratio of the signal response of the spiked samples before extraction and the neat standard at different concentrations. The matrix effect was calculated from the ratio of the signal response of samples spiked after extraction and the neat standard. The carry-over was evaluated for each IS by the injection of blank sample with the pure solvent after the calibration sample at high concentration level (dilution factor of 10). The reliability of results obtained within analysis of large sample sets was evaluated by on-instrument and freeze-and-thaw stability tests. The stability of spiked plasma extract at middle concentration level was measured in autosampler at certain time intervals: 0, 4, 8, 12, 16, and 24 hours (note: plasma samples do not fall into the scope of the invention). Sample for freeze-and-thaw experiment was analyzed immediately after complete unassisted thawing in autosampler.

Table 3. Dilution scheme for calibration with pooled sample (volumes in $\mu$l)

| Dilution | Mixture of IS | Pooled sample | Hexane : 2-propanol : chloroform (7:1.5:1.5, v/v/v) | Total Volume |
|---|---|---|---|---|
| 10x | 40 | 20 | 340 | 400 |
| 25x | 20 | 25 | 455 | 500 |
| 50x | 10 | 25 | 465 | 500 |
| 75x | 6.6 | 25 | 468.4 | 500 |
| 100x | 5 | 25 | 470 | 500 |
| 500x | 2 | 50 | 948 | 1000 |
| 1000x | 50 (diluted 100x) | 25 | 425 | 500 |
| 5000x | 10 (diluted 100x) | 25 | 465 | 500 |
| 10,000x | 5 (diluted 100x) | 25 | 470 | 500 |

(continued)

| Dilution | Mixture of IS | Pooled sample | Hexane : 2-propanol : chloroform (7:1.5:1.5, v/v/v) | Total Volume |
|---|---|---|---|---|
| 25,000x | 2 (diluted 100x) | 25 | 473 | 500 |
| 50,000x | 1 (diluted 100x) | 25 | 474 | 500 |
| 75,000x | 1 (diluted 100x) | 37.5 | 711.5 | 750 |
| 100,000x | 1 (diluted 100x) | 50 | 949 | 1000 |

*UHPSFC/MS*

Supercritical fluid chromatography is a tool for the separation of compounds of different polarity employing supercritical carbon dioxide (mobile phase) as main component for removing the compounds from an adsorbent (column-stationary phase). The addition of an organic solvent (typically methanol) to the supercritical carbon dioxide broadens the application range of UHPSFC and allows the removal of more polar compounds from the column. Generally, compounds can be differentiated if they are better soluble in water or alcohols, than they are of polar nature or if they are better soluble for instance in hexane than they are of nonpolar nature. Depending on the nature of the stationary phase, the mobile phase and the target compounds, like polar or nonpolar, interactions can be forced. For instance, polar compounds prefer to interact with polar stationary phases and in order to remove those from the stationary phase, a polar mobile phase can be used to remove the compounds from the stationary phase. A careful adjustment of these interactions by optimization of the mobile phase properties using a certain stationary phase allows the separation of compounds. The optimization of dimensions and properties of the stationary phase, such as smaller particle size and spherical particles of the sorbents, allows for a higher efficiency and is therefore called ultrahigh performance supercritical fluid chromatography (UHPSFC).

The chromatographic separation can be optimized for the separation of lipid classes, taking into account in particular the following. A lipid class has a dominant structural moiety (polar head group) in common which is mainly responsible for the interaction governing the retention mechanism. A lipid class can comprise numerous lipid species varying in the hydrocarbon chain length and structure (e.g., double bonds). Internal standards are added for each lipid (sub)class, therefore it is possible to identify and quantify all lipid species within a particular lipid (sub)class by comparing it to the class internal standard. MS-Analysis: Using a high-resolution, accurate-mass spectrometer as a detector allows the identification and quantification of lipids, as each lipid species has a defined m/z value and gives a signal response depending on the concentration in the sample.

In one particular embodiment, a detailed description of all parameters applied for the UHPSFC/MS method to obtain the results presented herein below is as follows: Instrument - Acquity Ultra Performance Convergence Chromatography (UPC$^2$) System hyphenated to the hybrid quadrupole-traveling wave ion mobility- time of flight mass spectrometer Synapt G2 Si from Waters. Chromatographic settings - stationary phase: Acquity BEH UPC$^2$ column (100 $\times$ 3 mm, 1.7 $\mu$m, Waters), the flow rate was 1.9 mL/min, the injection volume 1 $\mu$L, the column temperature 60°C ,the active back pressure regulator (ABPR) was set to 1800 psi, gradient mode : $CO_2$ and methanol with 30 mM ammonium acetate and 1 % water. The gradient started at 1% modifier and increased to 51 % in 5 min, afterwards kept constant for 1 min and flushed back to starting conditions with a total run time of 7.5 min. injection needle wash: a mixture of hexane - 2-propanol-water (1:2:0.5, v/v) column wash after each biological sample injection: a blank was injected using a fast gradient: 0 min - 1%, 1.4 min - 51%, 1.6 min - 51%, 1.8 min - 1%, and 4.8 min - 1% modifier., make-up effluent: HPLC 515 pump (Waters), make-up flow rate 0.25 mL/min methanol with 1 % water. ESI - MS settings: a capillary voltage of 3 kV, a sampling cone of 20 V, the source offset of 90 V, a source temperature of 150 °C, a drying temperature of 500°C, the cone gas flow of 50 L h$^{-1}$, the drying gas flow of 1000 L h$^{-1}$ and the nebulizer gas of 4 bar. Resolution mode in positive ion mode and a mass range of m/z 50-1200. The scan time was 0.15 s, and measurements were performed in continuum mode. The peptide leucine enkephaline was used as the lock mass with a scan time of 0.1 s and interval of 30 s.

During the analysis, the samples in the sequence should be randomized so that not the same type of samples, such as only healthy (non-cancerous) samples, are measured in a row. This guarantees that in case of an error at a certain time not only one type of sample is affected. Furthermore, it is important to measure QC samples after a predetermined amount of samples in order to verify the instrument performance.

Before measuring biological samples, no injection, blank and QC samples are measured to check the instrument performance and afterwards it is continued with biological samples. All samples are measured in duplicates and after 20 samples or 40 + 40 injections (sample + wash) respectively, QC and blank samples are measured. During the whole study, measurement and sample preparation control is performed by evaluating the peak areas of each internal standard of each sample and exporting results in the Microsoft Excel file. The QC samples are aliquots of an extract of a mixture of serum samples.

The lock mass is continuously measured during analysis, however the lock mass correction is not applied online, as preliminary results showed that the mass accuracy is worse using online correction. Furthermore, continuum mode is applied so that it is possible to monitor the resolution of the instrument. After measurements, the raw data get noise reduced using the MassLynx software from Waters. This improves the mass spectra as well as significantly reduces the file size, which allows easier handling of the files for further processing. The files are further processed by applying the lock mass correction and converting the files from profile to centroid mode, which enhances the mass accuracy and further reduces the file size. (The file size is important for data processing. The processing software can only hardly deal with huge file sizes and sample numbers, resulting in continuous errors making the processing time-consuming and cumbersome.)

All investigations regarding measurement control, profile mode and offline lock mass correction as well as the noise reduction improved data quality.

*Shotgun MS*

Experiments as presented herein below were performed on a quadrupole-linear ion trap mass spectrometer 6500 QTRAP (Sciex, Concord, ON, Canada) equipped by ESI probe with the following setting of tuning parameters: the ionspray voltage 5200 V, the curtain gas 20 psi, the source temperature 50°C, the ion source gas(*1*) 15 psi, and the ion source gas(2) 10 psi. MS/MS scans are measured with the scan rate 1000 Da/s, the declustering potential 80 V, the entrance potential 10 V, and the collision energy specified in the Table 4. Samples are introduced by a flow injection using a liquid chromatograph Agilent 1290 Series (Agilent Technologies) consisted of Agilent 1290 binary pump and Agilent 1260 autosampler. 50 $\mu$L of sample was injected into the flow rate 3 $\mu$L/min of chloroform - methanol - 2-propanol (1:2:4, v/v/v) mixture containing 7.5 mmol/L of ammonium acetate and 1% of acetic acid with the analysis time 12 min, and the autosampler temperature 20°C. LC/MS system is washed after each analysis with methanol - 2-propanol - water (2:2:1, v/v/v) mixture containing 7.5 mmol/L of ammonium acetate and 1% of acetic acid. Measured data experiments are extracted using LipidView software with the mass tolerance 0.3 Da, the minimum $S/N = 5$ and the minimum intensity 1%. Raw data characterized by type of scans, m/z values and peak intensities are exported as *.txt* data and further processed using our Microsoft Excel macro script for the detection and quantitation of lipids. Lipid classes are characterized using type of scans, and individual lipid species in selected MS/MS scan are detected according to *m/z* values with the mass tolerance 0.3 Da based on the database compiled from identified lipids in the pooled sample followed by the isotopic correction of ion intensities. Concentration of lipid species are calculated from corrected ion intensity related to the intensity of lipid class internal standards.

Table 4. Characterization of shotgun MS/MS scans and their parameters for individual lipid classes. PI - precursor ion scan and NL - neutral loss scan.

| Lipid class | Type of MS/MS scan | Collision energy | Observed ions |
|---|---|---|---|
| CE | PI 369; PI 376 | 12 | $[M+NH_4]^+$ |
| Cer | PI 264; PI 266 | 35 | $[M+H]^+$ |
| DG | NL 35 | 20 | $[M+NH_4]^+$ |
| Hex2Cer | PI 264; PI 266 | 35 | $[M+H]+$ |
| HexCer | PI 264; PI 266 | 35 | $[M+H]^+$ |
| Cholesterol | PI 369; PI 376 | 12 | $[M+NH_4]^+$ |
| LPA | NL 115 | 25 | $[M+NH_4]^+$ |
| LPC | PI 184 | 35 | $[M+H]^+$ |
| LPE | NL 141 | 30 | $[M+H]^+$ |
| LPG | NL 189 | 30 | $[M+NH_4]^+$ |
| LPS | NL 185 | 30 | $[M+H]^+$ |
| MG | NL 35 | 20 | $[M+NH_4]^+$ |
| PA | NL 115 | 25 | $[M+NH_4]^+$ |
| PC | PI 184 | 35 | $[M+H]^+$ |
| PE | NL 141 | 30 | $[M+H]^+$ |
| PG | NL 189 | 30 | $[M+NH_4]^+$ |

(continued)

| Lipid class | Type of MS/MS scan | Collision energy | Observed ions |
|---|---|---|---|
| PS | NL 185 | 30 | $[M+H]^+$ |
| SM | PI 184 | 35 | $[M+H]^+$ |
| SulfoHexCer | NL 98 | 25 | $[M+H]^+$ |
| TG | NL 17 | 30 | $[M+NH_4]^+$ |

*MALDI mass spectrometry*

Mass spectra were measured using ultrahigh-resolution MALDI mass spectrometer LTQ Orbitrap XL (Thermo Fisher Scientific, Waltham, MA, USA) equipped with the nitrogen UV laser (337 nm, 60 Hz) with a beam diameter of about 80 $\mu$m x 100 $\mu$m. The LTQ Orbitrap instrument is operated in the negative-ion mode over a normal mass range m/z 400 - 2000 and the mass resolution is set to R = 100,000 (full width at half maximum definition, at *m/z* 400). The zig-zag (or spiral outwards) sample movement with 250 $\mu$m step size is used during the individual data acquisition. The laser energy corresponds to 15% of maximum and 2 microscans/scan with 2 laser shots per microscan at 36 different positions are accumulated for each measurement to achieve a reproducible signal. Each sample (spotted matrix and body fluid extract mixture) is spotted five times. The total acquisition time of one sample including five consecutive spots is around ten minutes. Each measurement is represented by one average MALDI-MS spectrum with thousands of *m/z* values. The automatic peak assignment is subsequently performed and particular *m/z* peaks are matched with deprotonated molecules from a database created during the identification procedure using the Excel macro script. This peak assignment results in the generation of the list of present *m/z* of studied lipids with the average intensities in particular spectra for each samples that is used for further statistical evaluation.

The values of intensities below LLOQ are subsequently replaced by 80% of lowest quantified value in order to unify statistically insignificant or zero values and enable further processing based on logarithmic calculations. Corrected intensities are subsequently normalized to IS or particular ratios of intensities within one lipid class are generated. Finally, the statistical evaluation and comparison of normalized data set is performed.

*Data processing and quantitation*

The data processing starts with the data export from MS vendor software (e.g., Waters, Sciex or Thermo Scientific) into a data-processing software which may be Microsoft Excel for further steps to be done semiautomatically using e.g. advanced Excel script, in particular isotopic correction (Tables 5 and 6) and zero-filling. Quality control (QC) samples should be regularly injected to check the right and constant response of mass spectrometer. The typical QC sample is a pooled sample containing internal standards for all lipid classes to be quantified, which is injected after every 20 injections, and responses of individual internal standards are plotted versus the time. If responses of the internal standards are reduced too much, then it is the indication of an instrumental problem, typically the mass spectrometer requires cleaning due to the injection of too many samples. The typical cleaning interval is about several hundreds of samples, but it may strongly depend on the quality of prepared sample extracts, geometry of ion source, and system configuration.

The following example describes the example of UHPSFC/MS measurement on Synapt G2Si instrument from Waters, but similar approach is also applicable for other MS methods and different MS platforms from any instrumental vendor. The noise reduced, lock mass corrected and converted files are further processed with the MarkerLynx software. The reduced sequence table only including serum samples and QC samples has to be prepared in MassLynx with the corresponding suffix in the sample name (sample_nr20_AFAMM). Then the time scan range for each lipid class over the whole sequence has to be determined (for example m/z 250-350 is used for CE). For each lipid class, the method is created in MarkerLynx with the corresponding scan range, which will be combined, the mass peak separation of 50 mDa and marker intensity threshold of 3000. The method for each lipid class is applied for the sequence and MarkerLynx table with m/z values against the combined intensities is created. This table is exported into a text file and imported into a homemade database for the identification and quantitation of lipid species using Microsoft Excel. The m/z values obtained from MarkerLynx are compared to the accurate m/z values deposited as database for hundreds of lipid species for the identification. The database was created by evaluating present species in tissue and plasma samples. The identified lipid species are isotopically corrected and quantified by calculating the concentration in relation to the intensity and concentration of the (sub)class IS obtaining the table of lipid species concentrations vs. individual samples. Zero-filling procedure is performed. The final data matrix, where the columns are individual subjects and lines are individual lipids, are used for further MDA statistical evaluation.

Table 5. Database of lipid species monitored during the identification step in the positive-ion mode together with the calculation of isotopic correction for M+1 and M+2 isotopic peaks (so called deisotoping).

| Lipid | m/z | Isotopic correction | |
| --- | --- | --- | --- |
| | | M+2 | M+1 |
| CE 16:0 D7 (IS) | 376.3955 | 0.00% | 0.00% |
| CE 10:0 | 558.5244 | 0.00% | 0.00% |
| CE 12:0 | 586.5557 | 0.00% | 0.00% |
| CE 14:1 | 612.5714 | 0.00% | 0.00% |
| CE 14:0 | 614.5870 | 10.59% | 0.00% |
| CE 15:1 | 626.5532 | 0.00% | 0.00% |
| CE 15:0 | 628.6027 | 11.09% | 0.00% |
| CE 16:1 | 640.6027 | 0.00% | 0.00% |
| CE 16:0 | 642.6184 | 11.61% | 0.00% |
| CE 16:0 D7 (IS) | 649.6623 | 0.00% | 0.00% |
| CE 17:1 | 654.6183 | 0.00% | 0.00% |
| CE 17:0 | 656.6340 | 12.14% | 0.00% |
| CE 18:4 | 662.5870 | 0.00% | 0.00% |
| CE 18:3 | 664.6027 | 12.64% | 0.00% |
| CE 18:2 | 666.6184 | 12.65% | 0.00% |
| CE 18:1 | 668.6340 | 12.67% | 0.00% |
| CE 18:0 | 670.6497 | 12.68% | 0.00% |
| CE 19:1 | 682.6497 | 0.00% | 0.00% |
| CE 19:0 | 684.6653 | 13.23% | 0.00% |
| CE 20:5 | 688.6027 | 0.00% | 0.00% |
| CE 20:4 | 690.6184 | 13.75% | 0.00% |
| CE 20:3 | 692.6340 | 13.76% | 0.00% |
| CE 20:2 | 694.6497 | 13.77% | 0.00% |
| CE 20:1 | 696.6653 | 13.78% | 0.00% |
| CE 20:0 | 698.6810 | 13.80% | 0.00% |
| CE 21:1 | 710.6809 | 0.00% | 0.00% |
| CE 21:0 | 712.6966 | 14.37% | 0.00% |
| CE 22:6 | 714.6184 | 14.39% | 0.00% |
| CE 22:5 | 716.6340 | 14.90% | 0.00% |
| CE 22:4 | 718.6496 | 14.91% | 0.00% |
| CE 22:3 | 720.6653 | 14.93% | 0.00% |
| CE22:2 | 722.6810 | 14.94% | 0.00% |
| CE 22:1 | 724.6966 | 14.95% | 0.00% |
| CE 22:0 | 726.7123 | 14.96% | 0.00% |
| CE 23:1 | 738.7122 | 0.00% | 0.00% |
| CE 23:0 | 740.7279 | 15.57% | 0.00% |
| CE 24:5 | 744.6653 | 0.00% | 0.00% |
| CE 24:4 | 746.6809 | 16.13% | 0.00% |
| CE 24:3 | 748.6966 | 16.14% | 0.00% |
| CE 24:2 | 750.7122 | 16.15% | 0.00% |
| CE 24:1 | 752.7279 | 0.00% | 0.00% |
| CE 24:0 | 754.7436 | 16.18% | 0.00% |
| CE 25:0 | 768.7592 | 0.00% | 0.00% |
| CE 26:5 | 772.6966 | 0.00% | 0.00% |
| CE 26:4 | 774.7122 | 17.39% | 0.00% |
| CE 26:3 | 776.7279 | 17.40% | 0.00% |
| CE 26:0 | 782.7748 | 0.00% | 0.00% |

(continued)

| Lipid | m/z | Isotopic correction | |
|---|---|---|---|
| | | M+2 | M+1 |
| CE 27:0 | 796.7905 | 0.00% | 0.00% |
| CE 28:0 | 810.8061 | 0.00% | 0.00% |
| TG 35:0 | 642.5667 | 0.00% | 0.00% |
| TG 36:1 | 654.5667 | 0.00% | 0.00% |
| TG 36:0 | 656.5823 | 10.53% | 0.00% |
| TG 38:3 | 678.5667 | 0.00% | 0.00% |
| TG 38:2 | 680.5823 | 11.49% | 0.00% |
| TG 38:1 | 682.5980 | 11.50% | 0.00% |
| TG 38:0 | 684.6136 | 11.51% | 0.00% |
| TG 40:4 | 704.5823 | 0.00% | 0.00% |
| TG 40:3 | 706.5980 | 12.50% | 0.00% |
| TG 40:2 | 708.6136 | 12.51% | 0.00% |
| TG 40:1 | 710.6293 | 12.52% | 0.00% |
| TG 40:0 | 712.6449 | 12.54% | 0.00% |
| TG 41:1 | 724.6449 | 0.00% | 0.00% |
| TG 41:0 | 726.6606 | 13.07% | 0.00% |
| TG 42:4 | 732.6136 | 0.00% | 0.00% |
| TG 42:3 | 734.6293 | 0.00% | 0.00% |
| TG 42:2 | 736.6449 | 13.59% | 0.00% |
| TG 42:1 | 738.6606 | 13.60% | 0.00% |
| TG 42:0 | 740.6763 | 13.61% | 0.00% |
| TG 43:1 | 752.6763 | 0.00% | 0.00% |
| TG 43:0 | 754.6919 | 14.17% | 0.00% |
| TG 44:4 | 760.6449 | 0.00% | 0.00% |
| TG 44:3 | 762.6606 | 0.00% | 0.00% |
| TG 44:2 | 764.6763 | 14.71% | 0.00% |
| TG 44:1 | 766.6919 | 14.72% | 0.00% |
| TG 44:0 | 768.7076 | 14.73% | 0.00% |
| TG O-46:5/P-46:4 | 772.6813 | 0.00% | 0.00% |
| TG O-46:4/P-46:3 | 774.6970 | 15.64% | 0.00% |
| TG 45:3 | 776.6762 | 0.00% | 0.00% |
| TG 45:2 | 778.6919 | 15.29% | 0.00% |
| TG 45:1 | 780.7076 | 15.30% | 0.00% |
| TG 45:0 | 782.7232 | 15.31% | 0.00% |
| TG 46:4 | 788.6763 | 0.00% | 0.00% |
| TG 46:3 | 790.6919 | 15.87% | 0.00% |
| TG 46:2 | 792.7076 | 15.58% | 0.00% |
| TG 46:1 | 794.7232 | 15.89% | 0.00% |
| TG 46:0 | 796.7389 | 15.91% | 0.00% |
| TG 47:3 | 804.7076 | 0.00% | 0.00% |
| TG 47:2 | 806.7232 | 16.48% | 0.00% |
| TG 47:1 | 808.7389 | 16.50% | 0.00% |
| TG 47:0 | 810.7545 | 16.51% | 0.00% |
| TG 48:5 | 814.6919 | 0.00% | 0.00% |
| TG 48:4 | 816.7076 | 0.00% | 0.00% |
| TG 48:3 | 818.7232 | 17.09% | 0.00% |
| TG 48:2 | 820.7389 | 17.10% | 0.00% |
| TG 48:1 | 822.7545 | 17.11% | 0.00% |

(continued)

| Lipid | m/z | Isotopic correction | |
|---|---|---|---|
| | | M+2 | M+1 |
| TG 48:0 | 824.7702 | 17.13% | 0.00% |
| TG 49:3 | 832.7389 | 0.00% | 0.00% |
| TG 49:2 | 834.7545 | 17.73% | 0.00% |
| TG 49:1 | 836.7702 | 17.74% | 0.00% |
| TG 49:0 | 838.7858 | 17.76% | 0.00% |
| TG 50:6 | 840.7076 | 18.32% | 0.00% |
| TG 50:5 | 842.7232 | 18.33% | 0.00% |
| TG 50:4 | 844.7389 | 18.34% | 0.00% |
| TG 50:3 | 846.7545 | 18.36% | 0.00% |
| TG 50:2 | 848.7702 | 18.37% | 0.00% |
| TG 50:1 | 850.7858 | 18.38% | 0.00% |
| TG 50:0 | 852.8015 | 18.40% | 0.00% |
| TG 51:6 | 854.7232 | 18.41% | 0.00% |
| TG 51:5 | 856.7389 | 18.98% | 0.00% |
| TG 51:4 | 858.7545 | 18.99% | 0.00% |
| TG 51:3 | 860.7702 | 19.01% | 0.00% |
| TG 51:2 | 862.7858 | 19.02% | 0.00% |
| TG 51:1; TG 52:8 | 864.8015 | 19.04% | 0.00% |
| TG 52:7 | 866.7232 | 0.00% | 0.00% |
| TG 51:0 | 866.8171 | 19.04% | 0.00% |
| TG 52:6 | 868.7389 | 19.06% | 0.00% |
| TG 52:5 | 870.7545 | 19.65% | 0.00% |
| TG 52:4 | 872.7702 | 19.66% | 0.00% |
| TG 52:3 | 874.7858 | 19.67% | 0.00% |
| TG 52:2 | 876.8015 | 19.69% | 0.00% |
| TG 52:1 | 878.8171 | 19.70% | 0.00% |
| TG 52:0 | 880.8328 | 19.72% | 0.00% |
| TG 53:6 | 882.7545 | 19.73% | 0.00% |
| TG 53:5 | 884.7702 | 20.32% | 0.00% |
| TG 53:4 | 886.7858 | 20.34% | 0.00% |
| TG 53:3; TG 54:10 | 888.8015 | 20.35% | 0.00% |
| TG 53:2; TG 54:9 | 890.8171 | 20.36% | 0.00% |
| TG 54:8 | 892.7388 | 0.00% | 0.00% |
| TG 53:1 | 892.8327 | 20.38% | 0.00% |
| TG 54:7 | 894.7545 | 20.98% | 0.00% |
| TG 53:0 | 894.8484 | 20.39% | 0.00% |
| TG 54:6 | 896.7702 | 20.41% | 0.00% |
| TG 54:5 | 898.7858 | 21.01% | 0.00% |
| TG 54:4 | 900.8015 | 21.02% | 0.00% |
| TG 54:3 | 902.8171 | 21.04% | 0.00% |
| TG 54:2 | 904.8328 | 21.05% | 0.00% |
| TG 54:1 | 906.8484 | 21.07% | 0.00% |
| TG 54:0 | 908.8641 | 21.08% | 0.00% |
| TG 55:6 | 910.7858 | 21.10% | 0.00% |
| TG 55:5 | 912.8015 | 21.71% | 0.00% |
| TG 55:4 | 914.8171 | 21.73% | 0.00% |
| TG 55:3; TG 56:10 | 916.8328 | 21.74% | 0.00% |
| TG 56:9 | 918.7545 | 0.00% | 0.00% |

(continued)

| Lipid | m/z | Isotopic correction | |
|---|---|---|---|
| | | M+2 | M+1 |
| TG 55:2 | 918.8484 | 21.75% | 0.00% |
| TG 56:8 | 920.7701 | 22.38% | 0.00% |
| TG 55:1 | 920.8640 | 21.76% | 0.00% |
| TG 56:7 | 922.7858 | 22.39% | 0.00% |
| TG 55:0; TG 56:7 | 922.8797 | 21.79% | 0.00% |
| TG 56:6 | 924.8015 | 21.80% | 0.00% |
| TG 56:5 | 926.8171 | 22.42% | 0.00% |
| TG 56:4 | 928.8328 | 22.44% | 0.00% |
| TG 56:3 | 930.8484 | 22.45% | 0.00% |
| TG 56:2 | 932.8641 | 22.47% | 0.00% |
| TG 56:1 | 934.8797 | 22.48% | 0.00% |
| TG 57:7 | 936.8014 | 0.00% | 0.00% |
| TG 56:0 | 936.8954 | 22.50% | 0.00% |
| TG 57:6 | 938.8171 | 22.51% | 0.00% |
| TG 58:12 | 940.7388 | 0.00% | 0.00% |
| TG 57:5 | 940.8328 | 23.15% | 0.00% |
| TG 58:11 | 942.7545 | 23.79% | 0.00% |
| TG 57:4 | 942.8484 | 23.16% | 0.00% |
| TG 58:10 | 944.7701 | 23.81% | 0.00% |
| **TG 57:3 (IS)** | 944.8640 | 23.18% | 0.00% |
| TG 58:9 | 946.7858 | 23.82% | 0.00% |
| TG 57:2 | 946.8797 | 23.20% | 0.00% |
| TG 58:8 | 948.8014 | 23.84% | 0.00% |
| TG 57:1; TG 58:8 | 948.8954 | 23.21% | 0.00% |
| TG 58:7 | 950.8171 | 23.86% | 0.00% |
| TG 57:0; TG 58:7 | 950.9110 | 23.23% | 0.00% |
| TG 58:6 | 952.8328 | 23.24% | 0.00% |
| TG 58:5 | 954.8484 | 23.89% | 0.00% |
| TG 58:4 | 956.8641 | 23.90% | 0.00% |
| TG 58:3 | 958.8797 | 23.92% | 0.00% |
| TG 58:2 | 960.8954 | 23.93% | 0.00% |
| TG 59:8 | 962.8171 | 0.00% | 0.00% |
| TG 58:1 | 962.9110 | 23.95% | 0.00% |
| TG 59:7 | 964.8327 | 24.60% | 0.00% |
| TG 58:0 | 964.9267 | 23.96% | 0.00% |
| TG 60:13 | 966.7545 | 0.00% | 0.00% |
| TG 59:6 | 966.8484 | 23.98% | 0.00% |
| TG 60:12 | 968.7701 | 25.29% | 0.00% |
| TG 59:5 | 968.8641 | 24.64% | 0.00% |
| TG 60:11 | 970.7858 | 25.30% | 0.00% |
| TG 59:4 | 970.8797 | 24.65% | 0.00% |
| TG 60:10 | 972.8014 | 25.32% | 0.00% |
| TG 59:3 | 972.8953 | 24.67% | 0.00% |
| TG 60:9 | 974.8171 | 25.33% | 0.00% |
| TG 59:2 | 974.9110 | 24.68% | 0.00% |
| TG 60:8 | 976.8327 | 25.35% | 0.00% |
| TG 59:1 | 976.9266 | 24.70% | 0.00% |
| TG 59:1; TG 60:8 | 976.9267 | 24.70% | 0.00% |

(continued)

| Lipid | m/z | Isotopic correction | |
|---|---|---|---|
| | | M+2 | M+1 |
| TG 60:7 | 978.8484 | 25.37% | 0.00% |
| TG 59:0; TG 60:7 | 978.9423 | 24.72% | 0.00% |
| TG 60:6 | 980.8641 | 24.73% | 0.00% |
| TG 60:5 | 982.8797 | 25.40% | 0.00% |
| TG 60:4 | 984.8954 | 25.41% | 0.00% |
| TG 60:3 | 986.9110 | 25.43% | 0.00% |
| TG 60:2 | 988.9267 | 25.45% | 0.00% |
| TG 60:1 | 990.9423 | 25.46% | 0.00% |
| TG 60:0 | 992.9580 | 25.48% | 0.00% |
| TG 62:12 | 996.8014 | 0.00% | 0.00% |
| TG 62:11 | 998.8171 | 26.86% | 0.00% |
| TG 61:4 | 998.9110 | 0.00% | 0.00% |
| TG 62:10 | 1000.8327 | 26.88% | 0.00% |
| TG 61:3 | 1000.9266 | 26.20% | 0.00% |
| TG 62:9 | 1002.8484 | 26.89% | 0.00% |
| TG 61:2 | 1002.9423 | 26.22% | 0.00% |
| TG 62:8 | 1004.8640 | 26.91% | 0.00% |
| TG 62:7 | 1006.8797 | 26.92% | 0.00% |
| TG 62:6 | 1008.8953 | 26.94% | 0.00% |
| TG 62:5 | 1010.9110 | 26.96% | 0.00% |
| TG 62:4 | 1012.9266 | 26.97% | 0.00% |
| TG 62:3 | 1014.9423 | 26.99% | 0.00% |
| TG 62:2 | 1016.9579 | 27.01% | 0.00% |
| TG 62:1 | 1018.9736 | 27.02% | 0.00% |
| TG 64:12 | 1024.8327 | 0.00% | 0.00% |
| TG 64:11 | 1026.8484 | 28.46% | 0.00% |
| TG 63:4 | 1026.9423 | 0.00% | 0.00% |
| TG 64:10 | 1028.8640 | 28.48% | 0.00% |
| TG 63:3 | 1028.9579 | 27.79% | 0.00% |
| TG 64:9 | 1030.8797 | 28.50% | 0.00% |
| TG 64:8 | 1032.8953 | 28.52% | 0.00% |
| TG 64:7 | 1034.9110 | 28.53% | 0.00% |
| TG 64:6 | 1036.9266 | 28.55% | 0.00% |
| TG 64:5 | 1038.9423 | 28.57% | 0.00% |
| TG 64:4 | 1040.9579 | 28.58% | 0.00% |
| TG 66:12 | 1052.8640 | 0.00% | 0.00% |
| TG 66:11 | 1054.8797 | 30.12% | 0.00% |
| TG 66:10 | 1056.8953 | 30.14% | 0.00% |
| TG 66:9 | 1058.9110 | 30.15% | 0.00% |
| TG 66:8 | 1060.9266 | 30.17% | 0.00% |
| TG 66:7 | 1062.9423 | 30.19% | 0.00% |
| TG 66:6 | 1064.9579 | 30.21% | 0.00% |
| MG 10:0 | 264.2169 | 0.00% | 0.00% |
| MG 12:0 | 292.2482 | 0.00% | 0.00% |
| MG 14:1 | 318.2639 | 0.00% | 0.00% |
| MG 14:0 | 320.2795 | 2.59% | 0.00% |
| MG 15:1 | 332.2795 | 0.00% | 0.00% |
| MG 15:0 | 334.2952 | 2.80% | 0.00% |

(continued)

| Lipid | m/z | Isotopic correction | |
|---|---|---|---|
| | | M+2 | M+1 |
| MG 16:1 | 346.2952 | 0.00% | 0.00% |
| MG 16:0 | 348.3108 | 3.03% | 0.00% |
| MG 16:0 | 353.2662 | 0.00% | 0.00% |
| MG 17:1 | 360.3108 | 0.00% | 0.00% |
| MG 17:0 | 362.3265 | 3.27% | 0.00% |
| MG 17:0 | 367.2819 | 0.00% | 0.00% |
| MG 18:4 | 368.2795 | 0.00% | 0.00% |
| Chol | 369.3516 | 0.00% | 0.00% |
| **Chol D7 (IS)** | 376.3955 | 0.00% | 0.00% |
| Chol | 404.3887 | 0.00% | 0.00% |
| **Chol D7 (IS)** | 411.4326 | 0.00% | 0.00% |
| MG 18:3 | 370.2952 | 3.50% | 0.00% |
| MG 18:2 | 372.3108 | 3.51% | 0.00% |
| MG 18:1 | 374.3265 | 3.51% | 0.00% |
| MG 18:0 | 376.3421 | 3.52% | 0.00% |
| MG 18:1 | 379.2819 | 0.00% | 0.00% |
| MG 18:0 | 381.2975 | 3.42% | 0.00% |
| **MG 19:1 (IS)** | 388.3421 | 0.00% | 0.00% |
| MG 19:0 | 390.3578 | 3.78% | 0.00% |
| **MG 19:1 (IS)** | 393.2975 | 0.00% | 0.00% |
| MG 20:5 | 394.2952 | 0.00% | 0.00% |
| MG 20:4 | 396.3108 | 4.03% | 0.00% |
| MG 20:3 | 398.3265 | 4.04% | 0.00% |
| MG 20:2 | 400.3421 | 4.04% | 0.00% |
| MG 20:1 | 402.3578 | 4.05% | 0.00% |
| MG 21:1 | 416.3734 | 0.00% | 0.00% |
| MG 21:0 | 418.3891 | 4.34% | 0.00% |
| MG 22:6 | 420.3108 | 4.35% | 0.00% |
| MG 22:5 | 422.3265 | 4.61% | 0.00% |
| MG 22:4 | 424.3421 | 4.61% | 0.00% |
| MG 22:3 | 426.3587 | 4.62% | 0.00% |
| MG22:2 | 428.3734 | 4.63% | 0.00% |
| MG 22:1 | 430.3891 | 4.63% | 0.00% |
| MG 22:0 | 432.4047 | 4.64% | 0.00% |
| **DG 24:2 (IS)** | 435.3469 | 0.00% | 0.00% |
| MG 23:1 | 444.4047 | 0.00% | 0.00% |
| MG 23:0 | 446.4204 | 4.95% | 0.00% |
| MG 24:1 | 458.4204 | 0.00% | 0.00% |
| MG 24:0 | 460.4360 | 5.27% | 0.00% |
| **DG 24:2 (IS)** | 470.3840 | 0.00% | 0.00% |
| DG 28:0 | 530.4779 | 0.00% | 0.00% |
| DG 29:0 | 544.4935 | 0.00% | 0.00% |
| DG 30:2 | 554.4779 | 0.00% | 0.00% |
| DG 30:1 | 556.4935 | 7.67% | 0.00% |
| DG 30:0 | 558.5092 | 7.68% | 0.00% |
| DG 31:2 | 568.4935 | 0.00% | 0.00% |
| DG 31:1 | 570.5092 | 8.08% | 0.00% |
| DG 31:0 | 572.5248 | 8.09% | 0.00% |

(continued)

| Lipid | m/z | Isotopic correction | |
|---|---|---|---|
| | | M+2 | M+1 |
| DG 32:3 | 580.4936 | 0.00% | 0.00% |
| DG 32:2 | 582.5092 | 8.50% | 0.00% |
| DG 32:1 | 584.5249 | 8.50% | 0.00% |
| DG 32:0 | 586.5405 | 8.51% | 0.00% |
| DG 33:3 | 594.5092 | 0.00% | 0.00% |
| DG 33:2 | 596.5248 | 8.93% | 0.00% |
| DG 33:1 | 598.5405 | 8.94% | 0.00% |
| DG 33:0 | 600.5561 | 8.95% | 0.00% |
| DG 34:4 | 606.5092 | 0.00% | 0.00% |
| DG 34:3 | 608.5249 | 9.36% | 0.00% |
| DG 34:2 | 610.5405 | 9.37% | 0.00% |
| DG 34:1 | 612.5562 | 9.38% | 0.00% |
| DG 34:0 | 614.5718 | 9.39% | 0.00% |
| DG 35:4 | 620.5248 | 0.00% | 0.00% |
| DG 35:3 | 622.5405 | 9.82% | 0.00% |
| DG 35:2 | 624.5561 | 9.83% | 0.00% |
| DG 35:1 | 626.5718 | 9.84% | 0.00% |
| DG 35:0 | 628.5875 | 9.85% | 0.00% |
| DG 36:5 | 632.5249 | 0.00% | 0.00% |
| DG 36:4 | 634.5405 | 10.28% | 0.00% |
| DG 36:3 | 636.5561 | 10.29% | 0.00% |
| DG 36:2 | 638.5718 | 10.30% | 0.00% |
| DG 36:1 | 640.5874 | 10.31% | 0.00% |
| DG 37:7 | 642.5092 | 0.00% | 0.00% |
| DG 36:0 | 642.6031 | 10.32% | 0.00% |
| DG 37:5 | 646.5405 | 0.00% | 0.00% |
| DG 37:4 | 648.5561 | 0.00% | 0.00% |
| DG 37:3 | 650.5718 | 10.77% | 0.00% |
| DG 37:2 | 652.5874 | 10.78% | 0.00% |
| DG 37:1 | 654.6031 | 10.79% | 0.00% |
| DG 38:7 | 656.5248 | 0.00% | 0.00% |
| DG 37:0 | 656.6188 | 10.80% | 0.00% |
| DG 38:6 | 658.5405 | 11.23% | 0.00% |
| DG 38:5 | 660.5561 | 11.24% | 0.00% |
| DG 38:4 | 662.5718 | 11.26% | 0.00% |
| DG 38:3 | 664.5874 | 11.27% | 0.00% |
| DG 38:2 | 666.6031 | 11.28% | 0.00% |
| DG 38:1 | 668.6187 | 11.29% | 0.00% |
| DG 38:0 | 670.6344 | 11.30% | 0.00% |
| DG 39:6 | 672.5562 | 11.31% | 0.00% |
| DG 39:5 | 674.5718 | 11.75% | 0.00% |
| DG 39:4 | 676.5875 | 11.76% | 0.00% |
| DG 40:10; DG 39:3 | 678.5092 | 11.77% | 0.00% |
| DG 40:9; DG 39:2 | 680.5249 | 12.223% | 0.000% |
| DG 40:8; DG 39:1 | 682.5405 | 12.23% | 0.00% |
| DG 40:7; DG 39:0 | 684.5562 | 12.25% | 0.00% |
| DG 40:6 | 686.5718 | 12.26% | 0.00% |
| DG 40:5 | 688.5874 | 12.27% | 0.00% |

(continued)

| Lipid | m/z | Isotopic correction | |
| --- | --- | --- | --- |
| | | M+2 | M+1 |
| DG 40:4 | 690.6031 | 12.28% | 0.00% |
| DG 40:3 | 692.6187 | 12.29% | 0.00% |
| DG 40:2 | 694.6344 | 12.30% | 0.00% |
| DG 40:1 | 696.6500 | 12.31% | 0.00% |
| DG 40:0 | 698.6657 | 12.32% | 0.00% |
| DG 41:6 | 700.5675 | 12.33% | 0.00% |
| DG 41:5 | 702.3603 | 12.80% | 0.00% |
| DG 41:4 | 704.6188 | 12.81% | 0.00% |
| DG 42:10; DG 41:3 | 706.5405 | 12.82% | 0.00% |
| DG 42:9; DG 41:2 | 708.5562 | 13.29% | 0.00% |
| DG 42:8; DG 41:1 | 710.5718 | 13.30% | 0.00% |
| DG 42:7; DG 41:0 | 712.5875 | 13.32% | 0.00% |
| DG 42:6 | 714.6031 | 13.33% | 0.00% |
| DG 42:5 | 716.6188 | 13.34% | 0.00% |
| DG 42:4 | 718.6344 | 13.35% | 0.00% |
| DG 42:3 | 720.6501 | 13.36% | 0.00% |
| DG 42:2 | 722.6657 | 13.37% | 0.00% |
| DG 42:1 | 724.6814 | 13.39% | 0.00% |
| DG 42:0 | 726.6970 | 13.40% | 0.00% |
| DG 44:0 | 754.7283 | 0.00% | 0.00% |
| Coenzyme Q10 | 880.7177 | 0.00% | 0.00% |
| **Cer d30:1 (IS)** | 464.4462 | 0.00% | 0.00% |
| Cer d18:1/12:2 | 478.4255 | 0.00% | 0.00% |
| Cer d18:1/12:1 | 480.4411 | 6.08% | 0.00% |
| Cer d18:0/12:2 | 480.4412 | 0.00% | 0.00% |
| **Cer d18:1/12:0 (IS)** | 482.4568 | 6.08% | 0.00% |
| **Cer d18:0/12:1 (IS)** | 482.4568 | 6.08% | 0.00% |
| Cer d18:0/12:0 | 484.4725 | 6.09% | 0.00% |
| Cer d18:1/13:2 | 492.4411 | 0.00% | 0.00% |
| Cer d32:1 | 492.4775 | 0.00% | 0.00% |
| Cer d18:1/13:1; Cer d18:1/12:2 (1OH) | 494.4568 | 6.45% | 0.00% |
| Cer d18:0/13:2 | 494.4568 | 0.00% | 0.00% |
| Cer d18:1/13:0; Cer d18:1/12:1 (1OH) | 496.4724 | 6.46% | 0.00% |
| Cer d18:0/13:1; Cer d18:0/12:2 (1OH) | 496.4725 | 6.46% | 0.00% |
| Cer d18:1/12:0 (1OH) | 498.4517 | 6.46% | 0.00% |
| Cer d18:0/13:0; Cer d18:0/12:1 (1OH) | 498.4881 | 6.46% | 0.00% |
| Cer d18:0/12:0 (1OH) | 500.4674 | 6.47% | 0.00% |
| Cer d18:1/14:2 | 506.4568 | 0.00% | 0.00% |
| Cer d18:1/14:1; Cer d18:1/13:2 (1OH) | 508.4724 | 6.83% | 0.00% |
| Cer d18:0/14:2 | 508.4725 | 0.00% | 0.00% |
| Cer d18:1/14:0; Cer d18:1/13:0 (1OH) | 510.4808 | 6.84% | 0.00% |
| Cer d18:0/14:1; Cer d18:0/13:2 (1OH) | 510.4881 | 6.84% | 0.00% |
| Cer d18:1/13:0 (1OH) | 512.4674 | 6.85% | 0.00% |
| Cer d18:0/14:0; Cer d18:0/13:0 (1OH) | 512.4965 | 6.85% | 0.00% |
| Cer d18:0/13:0 (1OH) | 514.4831 | 6.86% | 0.00% |
| Cer d34:2 | 518.4932 | 0.00% | 0.00% |
| Cer d18:1/15:2 | 520.4724 | 0.00% | 0.00% |
| Cer d34:1 | 520.5088 | 7.41% | 0.00% |

(continued)

| Lipid | m/z | Isotopic correction | |
|---|---|---|---|
| | | M+2 | M+1 |
| Cer d18:0/15:2 | 522.4881 | 0.00% | 0.00% |
| Cer d18:1/15:1; Cer d18:1/14:2 (1OH) | 522.4881 | 7.23% | 0.00% |
| Cer d34:0 | 522.5245 | 7.42% | 0.00% |
| Cer d18:1/15:0; Cer d18:1/14:1 (1OH) | 524.5037 | 7.24% | 0.00% |
| Cer d18:0/15:1; Cer d18:0/14:2 (1OH) | 524.5038 | 7.24% | 0.00% |
| Cer d18:1/14:0 (1OH) | 526.4830 | 7.24% | 0.00% |
| Cer d18:0/15:0; Cer d18:0/14:1 (1OH) | 526.5194 | 7.24% | 0.00% |
| Cer d18:0/14:0 (1OH) | 528.4987 | 7.25% | 0.00% |
| Cer d18:1/16:2 | 534.4881 | 0.00% | 0.00% |
| Cer d35:1 | 534.5245 | 0.00% | 0.00% |
| Cer d18:1/16:1; Cer d18:1/15:2 (1OH) | 536.5037 | 7.63% | 0.00% |
| Cer d18:0/16:2 | 536.5038 | 0.00% | 0.00% |
| Cer d18:0/16:1; Cer d18:0/15:2 (1OH) | 538.5194 | 7.64% | 0.00% |
| Cer d18:1/16:0; Cer d18:1/15:1 (1OH) | 538.5194 | 7.64% | 0.00% |
| Cer d18:1/15:0 (1OH) | 540.4987 | 7.65% | 0.00% |
| Cer d18:0/16:0; Cer d18:0/15:1 (1OH) | 540.5351 | 7.65% | 0.00% |
| Cer d18:0/15:0 (1OH) | 542.5144 | 7.66% | 0.00% |
| Cer d36:2 | 546.5245 | 0.00% | 0.00% |
| Cer d18:1/17:2 | 548.5037 | 0.00% | 0.00% |
| Cer d36:1 | 548.5401 | 8.27% | 0.00% |
| Cer d18:0/17:2 | 550.5194 | 0.00% | 0.00% |
| Cer d18:1/17:1; Cer d18:1/16:2 (1OH) | 550.5194 | 8.05% | 0.00% |
| Cer d36:0 | 550.5558 | 8.28% | 0.00% |
| Cer d18:1/17:0; Cer d18:1/16:1 (1OH) | 552.5350 | 8.06% | 0.00% |
| Cer d18:0/17:1; Cer d18:0/16:2 (1OH) | 552.5351 | 8.06% | 0.00% |
| Cer d18:1/16:0 (1OH) | 554.5143 | 8.07% | 0.00% |
| Cer d18:0/17:0; Cer d18:0/16:1 (1OH) | 554.5507 | 8.07% | 0.00% |
| Cer d18:0/16:0 (1OH) | 556.5300 | 8.08% | 0.00% |
| Cer d18:1/18:3 | 560.5037 | 0.00% | 0.00% |
| Cer d18:1/18:2 | 562.5193 | 8.48% | 0.00% |
| Cer d37:1 | 562.5558 | 0.00% | 0.00% |
| Cer d18:0/18:2 | 564.5350 | 0.00% | 0.00% |
| Cer d18:1/18:1; Cer d18:1/17:2 (1OH) | 564.5350 | 8.49% | 0.00% |
| Cer d18:1/18:0; Cer d18:1/17:1 (1OH) | 566.5506 | 8.50% | 0.00% |
| Cer d18:0/18:1; Cer d18:0/17:2 (1OH) | 566.5507 | 8.50% | 0.00% |
| Cer d18:1/17:0 (1OH) | 568.5300 | 8.51% | 0.00% |
| Cer d18:0/18:0; Cer d18:0/17:1 (1OH) | 568.5663 | 8.51% | 0.00% |
| Cer d18:0/17:0 (1OH) | 570.5457 | 8.51% | 0.00% |
| Cer d38:2 | 574.5558 | 0.00% | 0.00% |
| Cer d18:1/19:2 | 576.5349 | 0.00% | 0.00% |
| Cer d38:1 | 576.5714 | 9.17% | 0.00% |
| Cer d18:0/19:2 | 578.5506 | 0.00% | 0.00% |
| Cer d18:1/19:1; Cer d18:1/18:2 (1OH) | 578.5506 | 8.93% | 0.00% |
| Cer d38:0 | 578.5871 | 9.18% | 0.00% |
| Cer d18:1/19:0; Cer d18:1/18:1 (1OH) | 580.5662 | 8.94% | 0.00% |
| Cer d18:0/19:1; Cer d18:0/18:2 (1OH) | 580.5663 | 8.94% | 0.00% |
| Cer d18:1/18:0 (1OH) | 582.5383 | 8.95% | 0.00% |
| Cer d18:0/19:0; Cer d18:0/18:1 (1OH) | 582.5819 | 8.95% | 0.00% |

(continued)

| Lipid | m/z | Isotopic correction | |
|---|---|---|---|
| | | M+2 | M+1 |
| Cer d18:0/18:0 (1OH) | 584.5540 | 8.96% | 0.00% |
| Cer d18:1/20:2 | 590.5506 | 0.00% | 0.00% |
| Cer d39:1 | 590.5871 | 0.00% | 0.00% |
| Cer d18:1/20:1; Cer d18:1/19:2 (1OH) | 592.5662 | 9.39% | 0.00% |
| Cer d18:0/20:2 | 592.5663 | 0.00% | 0.00% |
| Cer d18:0/20:1; Cer d18:0/19:2 (1OH) | 594.5819 | 9.40% | 0.00% |
| Cer d18:1/20:0; Cer d18:1/19:1 (1OH) | 594.5819 | 9.40% | 0.00% |
| Cer d18:1/19:0 (1OH) | 596.5613 | 9.41% | 0.00% |
| Cer d18:0/20:0; Cer d18:0/19:1 (1OH) | 596.5976 | 9.41% | 0.00% |
| Cer d18:0/19:0 (1OH) | 598.5770 | 9.42% | 0.00% |
| Cer d40:2 | 602.5871 | 0.00% | 0.00% |
| Cer d18:1/21:2 | 604.5662 | 0.00% | 0.00% |
| Cer d40:1 | 604.6027 | 10.11% | 0.00% |
| Cer d18:0/21:2 | 606.5819 | 0.00% | 0.00% |
| Cer d18:1/21:1; Cer d18:1/20:2 (1OH) | 606.5819 | 9.86% | 0.00% |
| Cer d40:0 | 606.6184 | 10.13% | 0.00% |
| Cer d18:1/21:0; Cer d18:1/20:1 (1OH) | 608.5975 | 9.87% | 0.00% |
| Cer d18:0/21:1; Cer d18:0/20:2 (1OH) | 608.5976 | 9.87% | 0.00% |
| Cer d18:1/20:0 (OH) | 610.5769 | 9.88% | 0.00% |
| Cer d18:0/21:0; Cer d18:0/20:1 (1OH) | 610.6132 | 9.88% | 0.00% |
| Cer d18:0/20:0 (1OH) | 612.5926 | 9.89% | 0.00% |
| Cer d18:1/22:2 | 618.5818 | 0.00% | 0.00% |
| Cer d41:1 | 618.6184 | 0.00% | 0.00% |
| Cer d18:0/22:2 | 620.5975 | 0.00% | 0.00% |
| Cer d18:1/22:1; Cer d18:1/21:2 (1OH) | 620.5975 | 10.34% | 0.00% |
| Cer d41:0 | 620.6340 | 10.62% | 0.00% |
| Cer d18:1/22:0; Cer d18:1/21:1 (1OH) | 622.6131 | 10.35% | 0.00% |
| Cer d18:0/22:1; Cer d18:0/21:2 (1OH) | 622.6132 | 10.35% | 0.00% |
| Cer d18:1/21:0 (1OH) | 624.5926 | 10.36% | 0.00% |
| Cer d18:0/22:0; Cer d18:0/21:1 (1OH) | 624.6288 | 10.36% | 0.00% |
| Cer d18:0/21:0 (1OH) | 626.6083 | 10.37% | 0.00% |
| Cer d42:3 | 628.6027 | 0.00% | 0.00% |
| Cer d42:2 | 630.6184 | 11.10% | 0.00% |
| Cer d18:1/23:2 | 632.5974 | 0.00% | 0.00% |
| Cer d42:1 | 632.6340 | 11.11% | 0.00% |
| Cer d18:0/23:2 | 634.6131 | 0.00% | 0.00% |
| Cer d18:1/23:1; Cer d18:1/22:2 (1OH) | 634.6131 | 10.83% | 0.00% |
| Cer d42:0 | 634.6497 | 11.12% | 0.00% |
| Cer d18:1/23:0; Cer d18:1/22:1 (1OH) | 636.6287 | 10.84% | 0.00% |
| Cer d18:0/23:1; Cer d18:0/22:2 (1OH) | 636.6288 | 10.84% | 0.00% |
| Cer d18:1/22:0 (1OH) | 638.6082 | 10.85% | 0.00% |
| Cer d18:0/23:0; Cer d18:0/22:1 (1OH) | 638.6444 | 10.85% | 0.00% |
| HexCer d18:1/12:2 | 640.4784 | 10.86% | 0.00% |
| Cer d18:0/22:0 (1OH) | 640.6239 | 10.86% | 0.00% |
| HexCer d18:1/12:1 | 642.4940 | 9.58% | 0.00% |
| HexCer d18:0/12:2 | 642.4941 | 10.87% | 0.00% |
| **HexCer d18:1/12:0 (IS)** | 644.5097 | 9.59% | 0.00% |
| **HexCer d18:0/12:1 (IS)** | 644.5097 | 9.59% | 0.00% |

(continued)

| Lipid | m/z | Isotopic correction | |
| --- | --- | --- | --- |
| | | M+2 | M+1 |
| Cer d43:2 | 644.6340 | 0.00% | 0.00% |
| HexCer d18:0/12:0 | 646.5254 | 9.60% | 0.00% |
| Cer d18:1/24:2 | 646.6131 | 9.60% | 0.00% |
| Cer d43:1 | 646.6497 | 11.63% | 0.00% |
| Cer d18:1/24:1; Cer d18:1/23:2 (1OH) | 648.6287 | 11.34% | 0.00% |
| Cer d18:0/24:2 | 648.6288 | 9.61% | 0.00% |
| Cer d43:0 | 648.6653 | 11.64% | 0.00% |
| Cer d18:0/24:1; Cer d18:0/23:2 (1OH) | 650.6444 | 11.35% | 0.00% |
| Cer d18:1/24:0; Cer d18:1/23:1 (1OH) | 650.6444 | 11.35% | 0.00% |
| Cer d18:1/23:0 (1OH) | 652.6239 | 11.36% | 0.00% |
| Cer d18:0/24:0; Cer d18:0/23:1 (1OH) | 652.6601 | 11.36% | 0.00% |
| HexCer d18:1/13:2 | 654.4940 | 11.07% | 0.00% |
| Cer d18:0/23:0 (1OH) | 654.6396 | 11.07% | 0.00% |
| HexCer d18:1/13:1; HexCer d18:1/12:2 (1OH) | 656.5097 | 10.03% | 0.00% |
| HexCer d18:0/13:2 | 656.5097 | 11.08% | 0.00% |
| Cer d44:3 | 656.6340 | 0.00% | 0.00% |
| HexCer d18:1/13:0; HexCer d18:1/12:1 (1OH) | 658.5253 | 10.04% | 0.00% |
| HexCer d18:0/13:1; HexCer d18:0/12:2 (1OH) | 658.5254 | 10.04% | 0.00% |
| Cer d44:2 | 658.6497 | 12.15% | 0.00% |
| HexCer d18:1/12:0 (1OH); Cer d18:1/25:2 | 660.5046 | 10.05% | 0.00% |
| HexCer d18:0/13:0; HexCer d18:0/12:1 (1OH) | 660.5410 | 10.05% | 0.00% |
| Cer d44:1 | 660.6653 | 12.16% | 0.00% |
| HexCer d18:0/12:0 (1OH); Cer d18:0/25:2 | 662.5203 | 10.06% | 0.00% |
| Cer d18:1/25:1; Cer d18:1/24:2 (1OH) | 662.6444 | 10.06% | 0.00% |
| Cer d44:0 | 662.6810 | 12.17% | 0.00% |
| Cer d18:1/25:0; Cer d18:1/24:1 (1OH) | 664.6600 | 11.86% | 0.00% |
| Cer d18:0/25:1; Cer d18:0/24:2 (1OH) | 664.6601 | 11.86% | 0.00% |
| Cer d18:1/24:0 (1OH) | 666.6395 | 11.88% | 0.00% |
| Cer d18:0/25:0; Cer d18:0/24:1 (1OH) | 666.6757 | 11.88% | 0.00% |
| HexCer d18:1/14:2 | 668.5097 | 11.58% | 0.00% |
| Cer d18:0/24:0 (1OH) | 668.6552 | 11.89% | 0.00% |
| HexCer d18:1/14:1; HexCer d18:1/13:2 (1OH) | 670.5253 | 10.49% | 0.00% |
| HexCer d18:0/14:2 | 670.5254 | 11.59% | 0.00% |
| HexCer d18:1/14:0; HexCer d18:1/13:0 (1OH) | 672.5337 | 10.50% | 0.00% |
| HexCer d18:0/14:1; HexCer d18:0/13:2 (1OH) | 672.5410 | 10.50% | 0.00% |
| HexCer d18:0/14:0; HexCer d18:0/13:0 (1OH) | 674.5494 | 10.51% | 0.00% |
| Cer d18:1/26:2; HexCer d18:1/13:0 (1OH) | 674.6443 | 10.51% | 0.00% |

(continued)

| Lipid | m/z | Isotopic correction | |
|---|---|---|---|
| | | M+2 | M+1 |
| Cer d18:0/26:2; HexCer d18:0/13:0 (1OH) | 676.6600 | 10.52% | 0.00% |
| Cer d18:1/26:1; Cer d18:1/25:2 (1OH) | 676.6600 | 12.38% | 0.00% |
| Cer d18:1/26:0; Cer d18:1/25:1 (1OH) | 678.6756 | 12.39% | 0.00% |
| Cer d18:0/26:1; Cer d18:0/25:2 (1OH) | 678.6757 | 12.39% | 0.00% |
| Cer d18:1/25:0 (1OH) | 680.6552 | 12.41% | 0.00% |
| Cer d18:0/26:0; Cer d18:0/25:1 (1OH) | 680.6913 | 12.41% | 0.00% |
| HexCer d18:1/15:2 | 682.5253 | 12.11% | 0.00% |
| Cer d18:0/25:0 (1OH) | 682.6709 | 12.42% | 0.00% |
| HexCer d18:0/15:2 | 684.5410 | 12.11% | 0.00% |
| HexCer d18:1/15:1; HexCer d18:1/14:2 (1OH) | 684.5410 | 10.96% | 0.00% |
| HexCer d18:1/15:0; HexCer d18:1/14:1 (1OH) | 686.5566 | 10.97% | 0.00% |
| HexCer d18:0/15:1; HexCer d18:0/14:2 (1OH) | 686.5567 | 10.97% | 0.00% |
| HexCer d18:1/14:0 (1OH); Cer d18: 1/27:2 | 688.5359 | 10.98% | 0.00% |
| HexCer d18:0/15:0; HexCer d18:0/14:1 (1OH) | 688.5723 | 10.98% | 0.00% |
| HexCer d18:0/14:0 (1OH); Cer d18: 0/27:2 | 690.5516 | 10.99% | 0.00% |
| Cer d18:1/27:1; Cer d18:1/26:2 (1OH) | 690.6756 | 10.90% | 0.00% |
| Cer d18:1/27:0; Cer d18:1/26:1 (1OH) | 692.6912 | 12.94% | 0.00% |
| Cer d18:0/27:1; Cer d18:0/26:2 (1OH) | 692.6913 | 11.00% | 0.00% |
| Cer d18:1/26:0 (1OH) | 694.6708 | 12.95% | 0.00% |
| Cer d18:0/27:0; Cer d18:0/26:1 (1OH) | 694.7069 | 12.95% | 0.00% |
| HexCer d18:1/16:2 | 696.5410 | 12.63% | 0.00% |
| Cer d18:0/26:0 (1OH) | 696.6865 | 12.96% | 0.00% |
| HexCer d18:1/16:1; HexCer d18:1/15:2 (1OH) | 698.5566 | 11.44% | 0.00% |
| HexCer d18:0/16:2 | 698.5567 | 12.64% | 0.00% |
| HexCer d18:0/16:1; HexCer d18:0/15:2 (1OH) | 700.5723 | 11.45% | 0.00% |
| HexCer d18:1/16:0; HexCer d18:1/15:1 (1OH) | 700.5723 | 11.45% | 0.00% |
| HexCer d18:0/16:0; HexCer d18:0/15:1 (1OH) | 702.5880 | 11.46% | 0.00% |
| Cer d18:1/28:2; HexCer d18:1/15:0 (1OH) | 702.6756 | 11.46% | 0.00% |
| Cer d18:1/28:1; Cer d18:1/27:2 (1OH) | 704.6912 | 13.48% | 0.00% |
| Cer d18:0/28:2; HexCer d18:0/15:0 (1OH) | 704.6913 | 11.47% | 0.00% |
| Cer d18:0/28:1; Cer d18:0/27:2 (1OH) | 706.7069 | 13.49% | 0.00% |
| Cer d18:1/28:0; Cer d18:1/27:1 (1OH) | 706.7069 | 13.49% | 0.00% |
| Cer d18:1/27:0 (1OH) | 708.6865 | 13.50% | 0.00% |
| Cer d18:0/28:0; Cer d18:0/27:1 (1OH) | 708.7226 | 13.50% | 0.00% |
| HexCer d18:1/17:2 | 710.5566 | 13.17% | 0.00% |
| Cer d18:0/27:0 (1OH) | 710.7022 | 13.51% | 0.00% |
| HexCer d18:0/17:2 | 712.5723 | 13.18% | 0.00% |

(continued)

| Lipid | m/z | Isotopic correction | |
|---|---|---|---|
| | | M+2 | M+1 |
| HexCer d18:1/17:1; HexCer d18:1/16:2 (1OH) | 712.5723 | 11.93% | 0.00% |
| HexCer d18:1/17:0; HexCer d18:1/16:1 (1OH) | 714.5879 | 11.95% | 0.00% |
| HexCer d18:0/17:1; HexCer d18:0/16:2 (1OH) | 714.5880 | 11.95% | 0.00% |
| HexCer d18:1/16:0 (1OH) | 716.5672 | 11.96% | 0.00% |
| HexCer d18:0/17:0; HexCer d18:0/16:1 (1OH) | 716.6036 | 11.96% | 0.00% |
| HexCer d18:0/16:0 (1OH) | 718.5829 | 11.97% | 0.00% |
| Cer d18:1/29:1; Cer d18:1/28:2 (1OH) | 718.7069 | 11.97% | 0.00% |
| Cer d18:1/29:0; Cer d18:1/28:1 (1OH) | 720.7225 | 14.06% | 0.00% |
| Cer d18:0/29:1; Cer d18:0/28:2 (1OH) | 720.7226 | 11.98% | 0.00% |
| Cer d18:1/28:0 (1OH) | 722.7201 | 14.07% | 0.00% |
| Cer d18:0/29:0; Cer d18:0/28:1 (1OH) | 722.7382 | 14.07% | 0.00% |
| HexCer d18:1/18:2 | 724.5722 | 13.73% | 0.00% |
| Cer d18:0/28:0 (1OH) | 724.7358 | 14.08% | 0.00% |
| HexCer d18:0/18:2 | 726.5879 | 13.74% | 0.00% |
| HexCer d18:1/18:1; HexCer d18:1/17:2 (1OH) | 726.5879 | 12.44% | 0.00% |
| HexCer d18:1/18:0; HexCer d18:1/17:1 (1OH) | 728.6035 | 12.45% | 0.00% |
| HexCer d18:0/18:1; HexCer d18:0/17:2 (1OH) | 728.6036 | 12.45% | 0.00% |
| HexCer d18:1/17:0 (1OH) | 730.5829 | 12.46% | 0.00% |
| HexCer d18:0/18:0; HexCer d18:0/17:1 (1OH) | 730.6192 | 12.46% | 0.00% |
| HexCer d18:0/17:0 (1OH) | 732.5986 | 12.47% | 0.00% |
| Cer d18:1/30:1; Cer d18:1/29:2 (1OH) | 732.7228 | 12.47% | 0.00% |
| Cer d18:1/30:0; Cer d18:1/29:1 (1OH) | 734.7381 | 14.64% | 0.00% |
| Cer d18:0/30:1; Cer d18:0/29:2 (1OH) | 734.7385 | 12.49% | 0.00% |
| Cer d18:1/29:0 (1OH) | 736.7178 | 14.65% | 0.00% |
| Cer d18:0/30:0; Cer d18:0/29:1 (1OH) | 736.7538 | 14.65% | 0.00% |
| HexCer d18:1/19:2 | 738.5878 | 14.29% | 0.00% |
| Cer d18:0/29:0 (1OH) | 738.7335 | 14.66% | 0.00% |
| HexCer d18:0/19:2 | 740.6035 | 14.31% | 0.00% |
| HexCer d18:1/19:1; HexCer d18:1/18:2 (1OH) | 740.6035 | 12.96% | 0.00% |
| HexCer d18:1/19:0; HexCer d18:1/18:1 (1OH) | 742.6191 | 12.97% | 0.00% |
| HexCer d18:0/19:1; HexCer d18:0/18:2 (1OH) | 742.6192 | 12.97% | 0.00% |
| HexCer d18:1/18:0 (1OH) | 744.5912 | 12.98% | 0.00% |
| HexCer d18:0/19:0; HexCer d18:0/18:1 (1OH) | 744.6348 | 12.98% | 0.00% |
| HexCer d18:0/18:0 (1OH) | 746.6069 | 12.99% | 0.00% |
| HexCer d18:1/20:2 | 752.6035 | 12.99% | 0.00% |
| HexCer d18:1/20:1; HexCer d18:1/19:2 (1OH) | 754.6191 | 13.49% | 0.00% |
| HexCer d18:0/20:2 | 754.6192 | 13.01% | 0.00% |

(continued)

| Lipid | m/z | Isotopic correction | |
|---|---|---|---|
| | | M+2 | M+1 |
| HexCer d18:0/20:1; HexCer d18:0/19:2 (1OH) | 756.6348 | 13.50% | 0.00% |
| HexCer d18:1/20:0; HexCer d18:1/19:1 (1OH) | 756.6348 | 13.50% | 0.00% |
| HexCer d18:1/19:0 (1OH) | 758.6142 | 13.52% | 0.00% |
| HexCer d18:0/20:0; HexCer d18:0/19:1 (1OH) | 758.6505 | 13.52% | 0.00% |
| HexCer d18:0/19:0 (1OH) | 760.6299 | 13.53% | 0.00% |
| HexCer d18:1/21:2 | 766.6191 | 0.00% | 0.00% |
| HexCer d18:0/21:2 | 768.6348 | 0.00% | 0.00% |
| HexCer d18:1/21:1; HexCer d18:1/20:2 (1OH) | 768.6348 | 14.04% | 0.00% |
| HexCer d18:1/21:0; HexCer d18:1/20:1 (1OH) | 770.6504 | 14.05% | 0.00% |
| HexCer d18:0/21:1; HexCer d18:0/20:2 (1OH) | 770.6505 | 14.05% | 0.00% |
| HexCer d18:1/20:0 (OH) | 772.6298 | 14.06% | 0.00% |
| HexCer d18:0/21:0; HexCer d18:0/20:1 (1OH) | 772.6661 | 14.06% | 0.00% |
| HexCer d18:0/20:0 (OH) | 774.6455 | 14.07% | 0.00% |
| HexCer d18:1/22:2 | 780.6347 | 0.00% | 0.00% |
| HexCer d18:0/22:2 | 782.6504 | 0.00% | 0.00% |
| HexCer d18:1/22:1; HexCer d18:1/21:2 (1OH) | 782.6504 | 14.59% | 0.00% |
| HexCer d18:1/22:0; HexCer d18:1/21:1 (1OH) | 784.6660 | 14.60% | 0.00% |
| HexCer d18:0/22:1; HexCer d18:0/21:2 (1OH) | 784.6661 | 14.60% | 0.00% |
| HexCer d18:1/21:0 (1OH) | 786.6455 | 14.62% | 0.00% |
| HexCer d18:0/22:0; HexCer d18:0/21:1 (1OH) | 786.6817 | 14.62% | 0.00% |
| HexCer d18:0/21:0 (1OH) | 788.6612 | 14.63% | 0.00% |
| HexCer d18:1/23:2 | 794.6503 | 0.00% | 0.00% |
| HexCer d18:0/23:2 | 796.6660 | 0.00% | 0.00% |
| HexCer d18:1/23:1; HexCer d18:1/22:2 (1OH) | 796.6660 | 15.16% | 0.00% |
| HexCer d18:1/23:0; HexCer d18:1/22:1 (1OH) | 798.6816 | 15.17% | 0.00% |
| HexCer d18:0/23:1; HexCer d18:0/22:2 (1OH) | 798.6817 | 15.17% | 0.00% |
| HexCer d18:1/22:0 (1OH) | 800.6611 | 15.18% | 0.00% |
| HexCer d18:0/23:0; HexCer d18:0/22:1 (1OH) | 800.6973 | 15.18% | 0.00% |
| Hex2Cer d18:1/12:2 | 802.5314 | 14.84% | 0.00% |
| HexCer d18:0/22:0 (1OH) | 802.6768 | 15.20% | 0.00% |
| Hex2Cer d18:1/12:1 | 804.5470 | 13.55% | 0.00% |
| Hex2Cer d18:0/12:2 | 804.5471 | 14.85% | 0.00% |
| **Hex2Cer d18:0/12:1 (IS)** | 806.5627 | 13.56% | 0.00% |
| **Hex2Cer d18:1/12:0 (IS)** | 806.5627 | 13.56% | 0.00% |
| Hex2Cer d18:0/12:0 | 808.5784 | 13.57% | 0.00% |

(continued)

| Lipid | m/z | Isotopic correction | |
|---|---|---|---|
| | | M+2 | M+1 |
| HexCer d18:1/24:2 | 808.6660 | 13.57% | 0.00% |
| HexCer d18:1/24:1; HexCer d18:1/23:2 (1OH) | 810.6816 | 15.74% | 0.00% |
| HexCer d18:0/24:2 | 810.6817 | 13.58% | 0.00% |
| HexCer d18:0/24:1; HexCer d18:0/23:2 (1OH) | 812.6973 | 15.75% | 0.00% |
| HexCer d18:1/24:0; HexCer d18:1/23:1 (1OH) | 812.6973 | 15.75% | 0.00% |
| HexCer d18:1/23:0 (1OH) | 814.6768 | 15.77% | 0.00% |
| HexCer d18:0/24:0; HexCer d18:0/23:1 (1OH) | 814.7130 | 15.77% | 0.00% |
| Hex2Cer d18:1/13:2 | 816.5470 | 15.41% | 0.00% |
| HexCer d18:0/23:0 (1OH) | 816.6925 | 15.78% | 0.00% |
| Hex2Cer d18:0/13:2 | 818.5627 | 15.42% | 0.00% |
| Hex2Cer d18:1/13:1; Hex2Cer d18:1/12:2 (1OH) | 818.5627 | 14.07% | 0.00% |
| Hex2Cer d18:1/13:0; Hex2Cer d18:1/12:1 (1OH) | 820.5783 | 14.08% | 0.00% |
| Hex2Cer d18:0/13:1; Hex2Cer d18:0/12:2 (1OH) | 820.5784 | 14.08% | 0.00% |
| Hex2Cer d18:1/12:0 (1OH); HexCer d18:1/25:2 | 822.5576 | 14.09% | 0.00% |
| Hex2Cer d18:0/13:0; Hex2Cer d18:0/12:1 (1OH) | 822.5940 | 14.09% | 0.00% |
| Hex2Cer d18:0/12:0 (1OH); HexCer d18:0/25:2 | 824.5733 | 14.10% | 0.00% |
| HexCer d18:1/25:1; HexCer d18:1/24:2 (1OH) | 824.6973 | 13.79% | 0.00% |
| HexCer d18:1/25:0; HexCer d18:1/24:1 (1OH) | 826.7129 | 16.35% | 0.00% |
| HexCer d18:0/25:1; HexCer d18:0/24:2 (1OH) | 826.7130 | 13.80% | 0.00% |
| HexCer d18:1/24:0 (1OH) | 828.6924 | 16.36% | 0.00% |
| HexCer d18:0/25:0; HexCer d18:0/24:1 (1OH) | 828.7286 | 16.36% | 0.00% |
| Hex2Cer d18:1/14:2 | 830.5627 | 15.99% | 0.00% |
| HexCer d18:0/24:0 (1OH) | 830.7081 | 16.37% | 0.00% |
| Hex2Cer d18:1/14:1; Hex2Cer d18:1/13:2 (1OH) | 832.5783 | 14.60% | 0.00% |
| Hex2Cer d18:0/14:2 | 832.5784 | 16.00% | 0.00% |
| Hex2Cer d18:1/14:0; Hex2Cer d18:1/13:1 (1OH) | 834.5867 | 14.61% | 0.00% |
| Hex2Cer d18:0/14:1; Hex2Cer d18:0/13:2 (1OH) | 834.5940 | 14.61% | 0.00% |
| Hex2Cer d18:0/14:0; Hex2Cer d18:0/13:1 (1OH) | 836.6024 | 14.63% | 0.00% |
| HexCer d18:1/26:2; Hex2Cer d18:1/13:0 (1OH) | 836.6972 | 14.63% | 0.00% |
| HexCer d18:0/26:2; Hex2Cer d18:0/13:0 (1OH) | 838.7129 | 14.64% | 0.00% |

(continued)

| Lipid | m/z | Isotopic correction | |
|---|---|---|---|
| | | M+2 | M+1 |
| HexCer d18:1/26:1; HexCer d18:1/25:2 (1OH) | 838.7129 | 16.94% | 0.00% |
| HexCer d18:1/26:0; HexCer d18:1/25:1 (1OH) | 840.7285 | 16.95% | 0.00% |
| HexCer d18:0/26:1; HexCer d18:0/25:2 (1OH) | 840.7286 | 16.95% | 0.00% |
| HexCer d18:1/25:0 (1OH) | 842.7081 | 16.96% | 0.00% |
| HexCer d18:0/26:0; HexCer d18:0/25:1 (1OH) | 842.7442 | 16.96% | 0.00% |
| Hex2Cer d18:1/15:2 | 844.5783 | 16.59% | 0.00% |
| HexCer d18:0/25:0 (1OH) | 844.7238 | 16.98% | 0.00% |
| Hex2Cer d18:0/15:2 | 846.5940 | 16.60% | 0.00% |
| Hex2Cer d18:1/15:1; Hex2Cer d18:1/14:2 (1OH) | 846.5940 | 15.15% | 0.00% |
| Hex2Cer d18:1/15:0; Hex2Cer d18:1/14:1 (1OH) | 848.6096 | 15.16% | 0.00% |
| Hex2Cer d18:0/15:1; Hex2Cer d18:0/14:2 (1OH) | 848.6097 | 15.16% | 0.00% |
| Hex2Cer d18:1/14:0 (1OH); HexCer d18:1/27:2 | 850.5889 | 15.17% | 0.00% |
| Hex2Cer d18:0/15:0; Hex2Cer d18:0/14:1 (1OH) | 850.6253 | 15.17% | 0.00% |
| Hex2Cer d18:0/14:0 (1OH); HexCer d18:0/27:2 | 852.6046 | 15.18% | 0.00% |
| HexCer d18:1/27:1; HexCer d18:1/26:2 (1OH) | 852.7285 | 14.85% | 0.00% |
| HexCer d18:1/27:0; HexCer d18:1/26:1 (1OH) | 854.7441 | 17.57% | 0.00% |
| HexCer d18:0/27:1; HexCer d18:0/26:2 (1OH) | 854.7442 | 14.86% | 0.00% |
| HexCer d18:1/26:0 (1OH) | 856.7237 | 17.58% | 0.00% |
| HexCer d18:0/27:0; HexCer d18:0/26:1 (1OH) | 856.7598 | 17.58% | 0.00% |
| Hex2Cer d18:1/16:2 | 858.5940 | 17.19% | 0.00% |
| HexCer d18:0/26:0 (1OH) | 858.7394 | 17.59% | 0.00% |
| Hex2Cer d18:1/16:1; Hex2Cer d18:1/15:2 (1OH) | 860.6096 | 15.71% | 0.00% |
| Hex2Cer d18:0/16:2 | 860.6097 | 17.20% | 0.00% |
| Hex2Cer d18:0/16:1; Hex2Cer d18:0/15:2 (1OH) | 862.6253 | 15.72% | 0.00% |
| Hex2Cer d18:1/16:0; Hex2Cer d18:1/15:1 (1OH) | 862.6253 | 15.72% | 0.00% |
| Hex2Cer d18:0/16:0; Hex2Cer d18:0/15:1 (1OH) | 864.6410 | 15.73% | 0.00% |
| HexCer d18:1/28:2; Hex2Cer d18:1/15:0 (1OH) | 864.7285 | 15.73% | 0.00% |
| HexCer d18:1/28:1; HexCer d18:1/27:2 (1OH) | 866.7441 | 18.18% | 0.00% |
| HexCer d18:0/28:2; Hex2Cer d18:0/15:0 (1OH) | 866.7442 | 15.74% | 0.00% |

(continued)

| Lipid | m/z | Isotopic correction | |
|---|---|---|---|
| | | M+2 | M+1 |
| HexCer d18:0/28:1; HexCer d18:0/27:2 (1OH) | 868.7598 | 18.20% | 0.00% |
| HexCer d18:1/28:0; HexCer d18:1/27:1 (1OH) | 868.7598 | 18.20% | 0.00% |
| HexCer d18:1/27:0 (1OH) | 870.7394 | 18.21% | 0.00% |
| HexCer d18:0/28:0; HexCer d18:0/27:1 (1OH) | 870.7755 | 18.21% | 0.00% |
| Hex2Cer d18:1/17:2 | 872.6096 | 17.81% | 0.00% |
| HexCer d18:0/27:0 (1OH) | 872.7551 | 18.22% | 0.00% |
| Hex2Cer d18:0/17:2 | 874.6253 | 17.82% | 0.00% |
| Hex2Cer d18:1/17:1; Hex2Cer d18:1/16:2 (1OH) | 874.6253 | 16.28% | 0.00% |
| Hex2Cer d18:1/17:0; Hex2Cer d18:1/16:1 (1OH) | 876.6409 | 16.29% | 0.00% |
| Hex2Cer d18:0/17:1; Hex2Cer d18:0/16:2 (1OH) | 876.6410 | 16.29% | 0.00% |
| Hex2Cer d18:1/16:0 (1OH) | 878.6202 | 16.30% | 0.00% |
| Hex2Cer d18:0/17:0; Hex2Cer d18:0/16:1 (1OH) | 878.6566 | 16.30% | 0.00% |
| Hex2Cer d18:0/16:0 (1OH) | 880.6359 | 16.31% | 0.00% |
| HexCer d18:1/29:1; HexCer d18:1/28:2 (1OH) | 880.7598 | 15.96% | 0.00% |
| HexCer d18:1/29:0; HexCer d18:1/28:1 (1OH) | 882.7754 | 18.84% | 0.00% |
| HexCer d18:0/29:1; HexCer d18:0/28:2 (1OH) | 882.7755 | 15.97% | 0.00% |
| HexCer d18:1/28:0 (1OH) | 884.7730 | 18.85% | 0.00% |
| HexCer d18:0/29:0; HexCer d18:0/28:1 (1OH) | 884.7911 | 18.85% | 0.00% |
| Hex2Cer d18:1/18:2 | 886.6252 | 18.44% | 0.00% |
| HexCer d18:0/28:0 (1OH) | 886.7887 | 18.87% | 0.00% |
| Hex2Cer d18:0/18:2 | 888.6409 | 18.45% | 0.00% |
| Hex2Cer d18:1/18:1; Hex2Cer d18:1/17:2 (1OH) | 888.6409 | 16.86% | 0.00% |
| Hex2Cer d18:1/18:0; Hex2Cer d18:1/17:1 (1OH) | 890.6565 | 16.87% | 0.00% |
| Hex2Cer d18:0/18:1; Hex2Cer d18:0/17:2 (1OH) | 890.6566 | 16.87% | 0.00% |
| Hex2Cer d18:1/17:0 (1OH) | 892.6359 | 16.88% | 0.00% |
| Hex2Cer d18:0/18:0; Hex2Cer d18:0/17:1 (1OH) | 892.6722 | 16.88% | 0.00% |
| Hex2Cer d18:0/17:0 (1OH) | 894.6516 | 16.90% | 0.00% |
| HexCer d18:1/30:1; HexCer d18:1/29:2 (1OH) | 894.7757 | 16.53% | 0.00% |
| HexCer d18:1/30:0; HexCer d18:1/29:1 (1OH) | 896.7910 | 19.49% | 0.00% |
| HexCer d18:0/30:1; HexCer d18:0/29:2 (1OH) | 896.7914 | 16.54% | 0.00% |
| HexCer d18:1/29:0 (1OH) | 898.7707 | 19.51% | 0.00% |

(continued)

| Lipid | m/z | Isotopic correction | |
|---|---|---|---|
| | | M+2 | M+1 |
| HexCer d18:0/30:0; HexCer d18:0/29:1 (1OH) | 898.8067 | 19.51% | 0.00% |
| Hex2Cer d18:1/19:2 | 900.6408 | 19.08% | 0.00% |
| HexCer d18:0/29:0 (1OH) | 900.7864 | 19.52% | 0.00% |
| Hex2Cer d18:0/19:2 | 902.6565 | 19.09% | 0.00% |
| Hex2Cer d18:1/19:1; Hex2Cer d18:1/18:2 (1OH) | 902.6565 | 17.45% | 0.00% |
| Hex2Cer d18:1/19:0; Hex2Cer d18:1/18:1 (1OH) | 904.6721 | 17.47% | 0.00% |
| Hex2Cer d18:0/19:1; Hex2Cer d18:0/18:2 (1OH) | 904.6722 | 17.47% | 0.00% |
| Hex2Cer d18:1/18:0 (1OH) | 906.6442 | 17.48% | 0.00% |
| Hex2Cer d18:0/19:0; Hex2Cer d18:0/18:1 (1OH) | 906.6878 | 17.48% | 0.00% |
| Hex2Cer d18:0/18:0 (1OH) | 908.6599 | 17.49% | 0.00% |
| Hex2Cer d18:1/20:2 | 914.6565 | 0.00% | 0.00% |
| Hex2Cer d18:1/20:1; Hex2Cer d18:1/19:2 (1OH) | 916.6721 | 18.06% | 0.00% |
| Hex2Cer d18:0/20:2 | 916.6722 | 0.00% | 0.00% |
| Hex2Cer d18:0/20:1; Hex2Cer d18:0/19:2 (1OH) | 918.6878 | 18.07% | 0.00% |
| Hex2Cer d18:1/20:0; Hex2Cer d18:1/19:1 (1OH) | 918.6878 | 18.07% | 0.00% |
| Hex2Cer d18:1/19:0 (1OH) | 920.6672 | 18.09% | 0.00% |
| Hex2Cer d18:0/20:0; Hex2Cer d18:0/19:1 (1OH) | 920.7035 | 18.09% | 0.00% |
| Hex2Cer d18:0/19:0 (1OH) | 922.6829 | 18.10% | 0.00% |
| Hex2Cer d18:1/21:2 | 928.6721 | 0.00% | 0.00% |
| Hex2Cer d18:0/21:2 | 930.6878 | 0.00% | 0.00% |
| Hex2Cer d18:1/21:1; Hex2Cer d18:1/20:2 (1OH) | 930.6878 | 18.68% | 0.00% |
| Hex2Cer d18:1/21:0; Hex2Cer d18:1/20:1 (1OH) | 932.7034 | 18.69% | 0.00% |
| Hex2Cer d18:0/21:1; Hex2Cer d18:0/20:2 (1OH) | 932.7035 | 18.69% | 0.00% |
| Hex2Cer d18:1/20:0 (OH) | 934.6828 | 18.70% | 0.00% |
| Hex2Cer d18:0/21:0; Hex2Cer d18:0/20:1 (1OH) | 934.7191 | 18.70% | 0.00% |
| Hex2Cer d18:0/20:0 (OH) | 936.6985 | 18.72% | 0.00% |
| Hex2Cer d18:1/22:2 | 942.6877 | 0.00% | 0.00% |
| Hex2Cer d18:0/22:2 | 944.7034 | 0.00% | 0.00% |
| Hex2Cer d18:1/22:1; Hex2Cer d18:1/21:2 (1OH) | 944.7034 | 19.31% | 0.00% |
| Hex2Cer d18:1/22:0; Hex2Cer d18:1/21:1 (1OH) | 946.7190 | 19.32% | 0.00% |
| Hex2Cer d18:0/22:1; Hex2Cer d18:0/21:2 (1OH) | 946.7191 | 19.32% | 0.00% |
| Hex2Cer d18:1/21:0 (1OH) | 948.6985 | 19.34% | 0.00% |
| Hex2Cer d18:0/22:0; Hex2Cer d18:0/21:1 (1OH) | 948.7347 | 19.34% | 0.00% |

(continued)

| Lipid | m/z | Isotopic correction | |
|---|---|---|---|
| | | M+2 | M+1 |
| Hex2Cer d18:0/21:0 (1OH) | 950.7142 | 19.35% | 0.00% |
| Hex2Cer d18:1/23:2 | 956.7033 | 0.00% | 0.00% |
| Hex2Cer d18:0/23:2 | 958.7190 | 0.00% | 0.00% |
| Hex2Cer d18:1/23:1; Hex2Cer d18:1/22:2 (1OH) | 958.7190 | 19.95% | 0.00% |
| Hex2Cer d18:1/23:0; Hex2Cer d18:1/22:1 (1OH) | 960.7346 | 19.97% | 0.00% |
| Hex2Cer d18:0/23:1; Hex2Cer d18:0/22:2 (1OH) | 960.7347 | 19.97% | 0.00% |
| Hex2Cer d18:1/22:0 (1OH) | 962.7141 | 19.98% | 0.00% |
| Hex2Cer d18:0/23:0; Hex2Cer d18:0/22:1 (1OH) | 962.7503 | 19.98% | 0.00% |
| Hex2Cer d18:0/22:0 (1OH) | 964.7298 | 19.99% | 0.00% |
| Hex2Cer d18:1/24:2 | 970.7190 | 0.00% | 0.00% |
| Hex2Cer d18:1/24:1; Hex2Cer d18:1/23:2 (1OH) | 972.7346 | 20.61% | 0.00% |
| Hex2Cer d18:0/24:2 | 972.7347 | 0.00% | 0.00% |
| Hex2Cer d18:0/24:1; Hex2Cer d18:0/23:2 (1OH) | 974.7503 | 20.62% | 0.00% |
| Hex2Cer d18:1/24:0; Hex2Cer d18:1/23:1 (1OH) | 974.7503 | 20.62% | 0.00% |
| Hex2Cer d18:1/23:0 (1OH) | 976.7298 | 20.64% | 0.00% |
| Hex2Cer d18:0/24:0; Hex2Cer d18:0/23:1 (1OH) | 976.7660 | 20.64% | 0.00% |
| Hex2Cer d18:0/23:0 (1OH) | 978.7455 | 20.65% | 0.00% |
| Hex2Cer d18:1/25:2 | 984.7346 | 0.00% | 0.00% |
| Hex2Cer d18:0/25:2 | 986.7503 | 0.00% | 0.00% |
| Hex2Cer d18:1/25:1; Hex2Cer d18:1/24:2 (1OH) | 986.7503 | 21.28% | 0.00% |
| Hex2Cer d18:1/25:0; Hex2Cer d18:1/24:1 (1OH) | 988.7659 | 21.29% | 0.00% |
| Hex2Cer d18:0/25:1; Hex2Cer d18:0/24:2 (1OH) | 988.7660 | 21.29% | 0.00% |
| Hex2Cer d18:1/24:0 (1OH) | 990.7454 | 21.30% | 0.00% |
| Hex2Cer d18:0/25:0; Hex2Cer d18:0/24:1 (1OH) | 990.7816 | 21.30% | 0.00% |
| Hex2Cer d18:0/24:0 (1OH) | 992.7611 | 21.32% | 0.00% |
| Hex2Cer d18:1/26:2 | 998.7502 | 0.00% | 0.00% |
| Hex2Cer d18:0/26:2 | 1000.7659 | 0.00% | 0.00% |
| Hex2Cer d18:1/26:1; Hex2Cer d18:1/25:2 (1OH) | 1000.7659 | 21.96% | 0.00% |
| Hex2Cer d18:1/26:0; Hex2Cer d18:1/25:1 (1OH) | 1002.7815 | 21.97% | 0.00% |
| Hex2Cer d18:0/26:1; Hex2Cer d18:0/25:2 (1OH) | 1002.7816 | 21.97% | 0.00% |
| Hex2Cer d18:1/25:0 (1OH) | 1004.7611 | 21.98% | 0.00% |
| Hex2Cer d18:0/26:0; Hex2Cer d18:0/25:1 (1OH) | 1004.7972 | 21.98% | 0.00% |
| Hex2Cer d18:0/25:0 (1OH) | 1006.7768 | 22.00% | 0.00% |
| Hex2Cer d18:1/27:2 | 1012.7658 | 0.00% | 0.00% |

(continued)

| Lipid | m/z | Isotopic correction | |
|---|---|---|---|
| | | M+2 | M+1 |
| Hex2Cer d18:0/27:2 | 1014.7815 | 0.00% | 0.00% |
| Hex2Cer d18:1/27:1; Hex2Cer d18:1/26:2 (1OH) | 1014.7815 | 22.65% | 0.00% |
| Hex2Cer d18:1/27:0; Hex2Cer d18:1/26:1 (1OH) | 1016.7971 | 22.66% | 0.00% |
| Hex2Cer d18:0/27:1; Hex2Cer d18:0/26:2 (1OH) | 1016.7972 | 22.66% | 0.00% |
| Hex2Cer d18:1/26:0 (1OH) | 1018.7767 | 22.68% | 0.00% |
| Hex2Cer d18:0/27:0; Hex2Cer d18:0/26:1 (1OH) | 1018.8128 | 22.68% | 0.00% |
| Hex2Cer d18:0/26:0 (1OH) | 1020.7924 | 22.69% | 0.00% |
| Hex2Cer d18:1/28:2 | 1026.7815 | 0.00% | 0.00% |
| Hex2Cer d18:1/28:1; Hex2Cer d18:1/27:2 (1OH) | 1028.7971 | 23.35% | 0.00% |
| Hex2Cer d18:0/28:2 | 1028.7972 | 0.00% | 0.00% |
| Hex2Cer d18:0/28:1; Hex2Cer d18:0/27:2 (1OH) | 1030.8128 | 23.37% | 0.00% |
| Hex2Cer d18:1/28:0; Hex2Cer d18:1/27:1 (1OH) | 1030.8128 | 23.37% | 0.00% |
| Hex2Cer d18:1/27:0 (1OH) | 1032.7924 | 23.38% | 0.00% |
| Hex2Cer d18:0/28:0; Hex2Cer d18:0/27:1 (1OH) | 1032.8285 | 23.38% | 0.00% |
| Hex2Cer d18:0/27:0 (1OH) | 1034.8081 | 23.40% | 0.00% |
| Hex2Cer d18:1/29:1; Hex2Cer d18:1/28:2 (1OH) | 1042.8128 | 0.00% | 0.00% |
| Hex2Cer d18:1/29:0; Hex2Cer d18:1/28:1 (1OH) | 1044.8284 | 24.08% | 0.00% |
| Hex2Cer d18:0/29:1; Hex2Cer d18:0/28:2 (1OH) | 1044.8285 | 0.00% | 0.00% |
| Hex2Cer d18:1/28:0 (1OH) | 1046.8260 | 24.10% | 0.00% |
| Hex2Cer d18:0/29:0; Hex2Cer d18:0/28:1 (1OH) | 1046.8441 | 24.09% | 0.00% |
| Hex2Cer d18:0/28:0 (1OH) | 1048.8417 | 24.11% | 0.00% |
| Hex2Cer d18:1/30:1; Hex2Cer d18:1/29:2 (1OH) | 1056.8287 | 0.00% | 0.00% |
| Hex2Cer d18:1/30:0; Hex2Cer d18:1/29:1 (1OH) | 1058.8440 | 24.81% | 0.00% |
| Hex2Cer d18:0/30:1; Hex2Cer d18:0/29:2 (1OH) | 1058.8444 | 0.00% | 0.00% |
| Hex2Cer d18:1/29:0 (1OH) | 1060.8237 | 24.83% | 0.00% |
| Hex2Cer d18:0/30:0; Hex2Cer d18:0/29:1 (1OH) | 1060.8597 | 24.83% | 0.00% |
| Hex2Cer d18:0/29:0 (1OH) | 1062.8394 | 24.84% | 0.00% |
| LPE 6:0 | 314.1363 | 0.00% | 0.00% |
| LPE 8:0 | 342.1676 | 0.00% | 0.00% |
| LPE 10:0 | 370.1989 | 0.00% | 0.00% |
| LPE 12:0 | 398.2302 | 0.00% | 0.00% |
| **LPE 14:0 (IS)** | 426.2615 | 0.00% | 0.00% |
| LPE P-16:0; LPE O-16:1 | 438.2979 | 0.00% | 0.00% |
| LPE O-16:0; LPE 15:0 | 440.2772 | 3.95% | 0.00% |

(continued)

| Lipid | m/z | Isotopic correction | |
|---|---|---|---|
| | | M+2 | M+1 |
| LPE 16:2 | 450.2615 | 0.00% | 0.00% |
| LPE 16:1 | 452.2772 | 4.15% | 0.00% |
| LPE 16:0 | 454.2928 | 4.16% | 0.00% |
| LPE P-18:0; LPE O-18:1; LPE 17:1 | 466.3298 | 0.00% | 0.00% |
| LPE O-18:0; LPE 17:0 | 468.3085 | 4.49% | 0.00% |
| LPE 18:4 | 474.2615 | 0.00% | 0.00% |
| LPE 18:3 | 476.2772 | 4.68% | 0.00% |
| LPE 18:2 | 478.2928 | 4.69% | 0.00% |
| LPE 18:1 | 480.3085 | 4.69% | 0.00% |
| LPE 18:0 | 482.3241 | 4.70% | 0.00% |
| LPE P-20:0; LPE O-20:1 | 494.3605 | 0.00% | 0.00% |
| LPE O-20:0; LPE 19:0 | 496.3398 | 5.08% | 0.00% |
| LPE 20:5 | 500.2766 | 0.00% | 0.00% |
| LPE 20:4 | 502.2923 | 5.26% | 0.00% |
| LPE 20:3 | 504.3079 | 5.27% | 0.00% |
| LPE 20:2 | 506.3241 | 5.27% | 0.00% |
| LPE 20:1 | 508.3398 | 5.28% | 0.00% |
| LPE 20:0 | 510.3554 | 5.29% | 0.00% |
| LPE P-22:0; LPE O-22:1 | 522.3918 | 0.00% | 0.00% |
| LPE O-22:0; LPE 21:0 | 524.3711 | 5.71% | 0.00% |
| LPE 22:6 | 526.2928 | 5.72% | 0.00% |
| LPE 22:5 | 528.3079 | 5.89% | 0.00% |
| LPE 22:4 | 530.3235 | 5.89% | 0.00% |
| LPE 22:1 | 536.3711 | 0.00% | 0.00% |
| LPE 22:0 | 538.3867 | 5.92% | 0.00% |
| LPE P-24:0; LPE O-24:1 | 550.4231 | 0.00% | 0.00% |
| LPE O-24:0; LPE 23:0 | 552.4024 | 6.40% | 0.00% |
| LPE 24:1 | 564.4024 | 0.00% | 0.00% |
| LPE 24:0 | 566.4180 | 6.61% | 0.00% |
| LPE 25:0 | 580.4337 | 0.00% | 0.00% |
| LPE 26:0 | 594.4493 | 0.00% | 0.00% |
| LPE 27:0 | 608.4650 | 0.00% | 0.00% |
| LPE 28:0 | 622.4806 | 0.00% | 0.00% |
| **PE 28:0** | 636.4599 | 0.00% | 0.00% |
| PE 29:0 | 650.4755 | 0.00% | 0.00% |
| PE 30:1 | 662.4755 | 0.00% | 0.00% |
| PE 30:0 | 664.4912 | 9.17% | 0.00% |
| PE P-32:1; PE-31:2 | 674.5119 | 0.00% | 0.00% |
| PE P-32:0; PE-31:1 | 676.4912 | 9.83% | 0.00% |
| PE O-32:0; PE-31:0 | 678.5068 | 9.84% | 0.00% |
| PE 32:3; PE P-33:2 | 686.4755 | 0.00% | 0.00% |
| PE 32:2; PE P-33:1 | 688.4912 | 10.03% | 0.00% |
| PE 32:1; PE P-33:0 | 690.5068 | 10.04% | 0.00% |
| PE 32:0; PE O-33:0 | 692.5225 | 10.05% | 0.00% |
| PE 32:0 | 692.5225 | 10.05% | 0.00% |
| PE O-34:3/P-34:2 | 700.5276 | 0.00% | 0.00% |
| PE P-34:1; PE 33:2 | 702.5068 | 0.00% | 0.00% |
| PE O-34:2/P-34:1 | 702.5432 | 10.75% | 0.00% |

(continued)

| Lipid | m/z | Isotopic correction | |
| | | M+2 | M+1 |
|---|---|---|---|
| PE P-34:0; PE 33:1 | 704.5225 | 10.76% | 0.00% |
| PE O-34:1/P-34:0 | 704.5589 | 10.76% | 0.00% |
| PE O-34:0;PE 33:0 | 706.5381 | 10.77% | 0.00% |
| PE 34:4; PE P-35:3 | 712.4912 | 0.00% | 0.00% |
| PE 34:3; PE P-35:2 | 714.5068 | 10.95% | 0.00% |
| PE 34:2; PE P-35:1 | 716.5225 | 10.96% | 0.00% |
| PE 34:1; PE P-35:0 | 718.5381 | 10.97% | 0.00% |
| PE 34:0; PE P-36:6 | 720.5538 | 10.98% | 0.00% |
| PE P-36:5; PE 35:6 | 722.4756 | 11.69% | 0.00% |
| PE O-36:6/P-36:5 | 722.5119 | 0.00% | 0.00% |
| PE P-36:4; PE 35:5 | 724.4912 | 11.70% | 0.00% |
| PE O-36:5/P-36:4 | 724.5276 | 11.70% | 0.00% |
| PE P-36:3; PE 35:4 | 726.5068 | 11.71% | 0.00% |
| PE O-36:4/P-36:3 | 726.5432 | 11.71% | 0.00% |
| PE P-36:2; PE 35:3 | 728.5225 | 11.72% | 0.00% |
| PE O-36:3/P-36:2 | 728.5589 | 11.72% | 0.00% |
| PE P-36:1; PE 35:2 | 730.5381 | 11.73% | 0.00% |
| PE O-36:2/P-36:1 | 730.5745 | 11.73% | 0.00% |
| PE P-36:0; PE 35:1 | 732.5538 | 11.74% | 0.00% |
| PE 36:7; PE O-36:0; PE 35:0 | 734.5694 | 11.75% | 0.00% |
| PE 36:6; PE P-37:5 | 736.4912 | 11.90% | 0.00% |
| PE 36:5; PE P-37:4 | 738.5068 | 11.91% | 0.00% |
| PE 36:4; PE P-37:3 | 740.5225 | 11.92% | 0.00% |
| PE 36:3; PE P-37:2 | 742.5381 | 11.93% | 0.00% |
| PE 36:2; PE P-37:1 | 744.5538 | 11.94% | 0.00% |
| PE 36:1; PE P-37:0 | 746.5694 | 11.95% | 0.00% |
| PE O-38:7/P-38:6 | 748.5276 | 0.00% | 0.00% |
| PE 36:0; PE P-38:6 | 748.5851 | 11.96% | 0.00% |
| PE P-38:5; PE 37:6 | 750.5063 | 12.71% | 0.00% |
| PE O-38:6/P-38:5 | 750.5432 | 12.71% | 0.00% |
| PE P-38:4; PE 37:5 | 752.5220 | 14.35% | 0.00% |
| PE O-38:5/P-38:4 | 752.5589 | 12.72% | 0.00% |
| PE P-38:3; PE 37:4 | 754.5376 | 14.36% | 0.00% |
| PE O-38:4/P-38:3 | 754.5745 | 12.73% | 0.00% |
| PE P-38:2; PE 37:3 | 756.5538 | 14.37% | 0.00% |
| PE O-38:3/P-38:2 | 756.5902 | 12.74% | 0.00% |
| PE P-38:1; PE 37:2 | 758.5694 | 14.39% | 0.00% |
| PE P-38:0; PE 37:1 | 760.5851 | 14.40% | 0.00% |
| PE 38:7; PE O-38:0; PE 37:0 | 762.6007 | 14.41 % | 0.00% |
| PE 38:6; PE P-39:5 | 764.5225 | 14.55% | 0.00% |
| PE 38:5; PE P-39:4 | 766.5382 | 14.56% | 0.00% |
| PE 38:4; PE P-40:10; PE P-29:3 | 768.5538 | 14.57% | 0.00% |
| PE 38:3; PE P-40:9; PE P-39:2 | 770.5694 | 14.586% | 0.00% |
| PE 38:2; PE P-40:8; PE P-39:1 | 772.5851 | 14.60% | 0.00% |
| PE 38:1; PE P-40:7; PE P-39:0 | 774.6007 | 14.61 % | 0.00% |
| PE O-40:7/P-40:6 | 776.5589 | 0.00% | 0.00% |
| PE 38:0; PE P-40:6, PE O-39:0 | 776.6164 | 14.62% | 0.00% |
| PE P-40:5; PE 39:6 | 778.5382 | 13.78% | 0.00% |

34

(continued)

| Lipid | m/z | Isotopic correction | |
|---|---|---|---|
| | | M+2 | M+1 |
| PE O-40:6/P-40:5 | 778.5745 | 13.78% | 0.00% |
| PE P-40:4; PE 39:5 | 780.5538 | 13.79% | 0.00% |
| PE O-40:5/P-40:4 | 780.5902 | 13.79% | 0.00% |
| PE P-40:3; PE 39:4 | 782.5694 | 15.51 % | 0.00% |
| PE 40:10; PE P-40:2; PE 39:3 | 784.4912 | 15.52% | 0.00% |
| PE 40:9; PE P-40:1; PE 39:2 | 786.5069 | 15.66% | 0.00% |
| PE 40:8; PE P-40:0; PE 39:1 | 788.5225 | 15.67% | 0.00% |
| PE 40:7; PE O-40:0; PE 39:0 | 790.5381 | 15.69% | 0.00% |
| PE 40:6; PE P-41:5 | 792.5538 | 15.70% | 0.00% |
| PE 40:5; PE P-41:4 | 794.5695 | 15.71 % | 0.00% |
| PE 40:4; PE P-42:10; PE P-41:3 | 796.5851 | 15.72% | 0.00% |
| PE 40:3; PE P-42:9; PE P-41:2 | 798.6008 | 15.73% | 0.00% |
| PE 40:2; PE P-42:8; PE P-41:1 | 800.6164 | 15.75% | 0.00% |
| PE 40:1; PE P-42:7; PE P-41:0 | 802.6320 | 15.76% | 0.00% |
| PE 40:0; PE P-42:6, PE O-41:0 | 804.6477 | 15.77% | 0.00% |
| PE P-42:5; PE 41:6 | 806.5694 | 14.90% | 0.00% |
| PE O-42:6/P-42:5 | 806.6058 | 0.00% | 0.00% |
| PE P-42:4; PE 41:5 | 808.5851 | 16.69% | 0.00% |
| PE P-42:3; PE 41:4 | 810.6007 | 16.71 % | 0.00% |
| PE 42:10; PE P-42:2; PE 41:3 | 812.5225 | 16.72% | 0.00% |
| PE 42:9; PE P-42:1; PE 41:2 | 814.5382 | 16.85% | 0.00% |
| PE 42:8; PE P-42:0; PE 41:1 | 816.5538 | 16.87% | 0.00% |
| PE 42:7; PE O-42:0 | 818.5694 | 16.88% | 0.00% |
| PE 42:6; PE P-43:5 | 820.5851 | 16.89% | 0.00% |
| PE 42:5; PE P-43:4 | 822.6007 | 16.91 % | 0.00% |
| PE 42:4; PE P-43:3 | 824.6164 | 16.92% | 0.00% |
| PE 42:3; PE P-43:2 | 826.6320 | 16.93% | 0.00% |
| PE 42:2; PE P-43:1 | 828.6477 | 16.94% | 0.00% |
| PE 42:1; PE P-43:0 | 830.6633 | 16.96% | 0.00% |
| PE 42:0; PE O-43:0 | 832.6790 | 16.97% | 0.00% |
| PE 44:0 | 860.7103 | 0.00% | 0.00% |
| LPI 6:0 | 450.1734 | 0.00% | 0.00% |
| LPI 8:0 | 478.2047 | 0.00% | 0.00% |
| LPI 10:0 | 506.2360 | 0.00% | 0.00% |
| LPI 12:0 | 534.2673 | 0.00% | 0.00% |
| LPI 14:0 | 562.2986 | 0.00% | 0.00% |
| LPI P-16:0; LPI O-16:1 | 574.3350 | 0.00% | 0.00% |
| LPI O-16:0; LPI 15:0 | 576.3143 | 6.16% | 0.00% |
| LPI 16:2 | 586.2986 | 0.00% | 0.00% |
| PI 16:0 | 587.2827 | 0.00% | 0.00% |
| LPI 16:1 | 588.3143 | 6.36% | 0.00% |
| LPI 16:0 | 590.3299 | 6.37% | 0.00% |
| LPI P-18:0; LPI O-18:1; LPI 17:1 | 602.3669 | 0.00% | 0.00% |
| **PI 16:0; LPI O-18:0; LPI 17:0 (IS)** | 604.3456 | 6.80% | 0.00% |
| LPI 18:4 | 610.2986 | 0.00% | 0.00% |
| LPI 18:3 | 612.3143 | 6.99% | 0.00% |
| LPI 18:2 | 614.3299 | 6.99% | 0.00% |
| LPI 18:1 | 616.3456 | 7.00% | 0.00% |

(continued)

| Lipid | m/z | Isotopic correction | |
|---|---|---|---|
| | | M+2 | M+1 |
| LPI 18:0 | 618.3612 | 7.01% | 0.00% |
| LPI P-20:0; LPI O-20:1 | 630.3976 | 0.00% | 0.00% |
| LPI O-20:0; LPI 19:0 | 632.3769 | 7.49% | 0.00% |
| LPI 20:5 | 636.3137 | 0.00% | 0.00% |
| LPI 20:4 | 638.3294 | 7.67% | 0.00% |
| LPI 20:3 | 640.3450 | 7.68% | 0.00% |
| LPI 20:2 | 642.3612 | 7.68% | 0.00% |
| LPI 20:1 | 644.3769 | 7.69% | 0.00% |
| LPI 20:0 | 646.3925 | 7.70% | 0.00% |
| LPI P-22:0; LPI O-22:1 | 658.4289 | 0.00% | 0.00% |
| LPI O-22:0; LPI 21:0 | 660.4082 | 8.23% | 0.00% |
| LPI 22:6 | 662.3299 | 8.23% | 0.00% |
| LPI 22:5 | 664.3450 | 8.40% | 0.00% |
| LPI 22:4 | 666.3606 | 8.41% | 0.00% |
| LPI 22:1 | 672.4082 | 0.00% | 0.00% |
| LPI 22:0 | 674.4238 | 8.44% | 0.00% |
| LPI P-24:0; LPI O-24:1 | 686.4602 | 0.00% | 0.00% |
| LPI O-24:0; LPI 23:0 | 688.4395 | 9.01% | 0.00% |
| LPI 24:1 | 700.4395 | 0.00% | 0.00% |
| LPI 24:0 | 702.4551 | 9.22% | 0.00% |
| LPI 25:0 | 716.4708 | 0.00% | 0.00% |
| LPI 26:0 | 730.4864 | 0.00% | 0.00% |
| LPI 27:0 | 744.5021 | 0.00% | 0.00% |
| LPI 28:0 | 758.5177 | 0.00% | 0.00% |
| PI 28:0 | 772.4970 | 0.00% | 0.00% |
| PI 29:0 | 786.5126 | 0.00% | 0.00% |
| PI 30:1 | 798.5126 | 0.00% | 0.00% |
| PI 30:0 | 800.5283 | 10.04% | 0.00% |
| PI P-32:1; PI-31:2 | 810.5490 | 0.00% | 0.00% |
| PI P-32:0; PI-31:1 | 812.5283 | 12.85% | 0.00% |
| PI O-32:0; PI-31:0 | 814.5440 | 12.86% | 0.00% |
| PI 32:3; PI P-33:2 | 822.5126 | 0.00% | 0.00% |
| PI 32:2; PI P-33:1 | 824.5283 | 13.05% | 0.00% |
| PI 32:1; PI P-33:0 | 826.5440 | 13.07% | 0.00% |
| PI 32:0; PI O-33:0 | 828.5596 | 13.08% | 0.00% |
| PI P-34:1; PI 33:2 | 838.5440 | 0.00% | 0.00% |
| PI P-34:0; PI 33:1 | 840.5596 | 13.88% | 0.00% |
| PI O-34:0;PI 33:0 | 842.5753 | 13.90% | 0.00% |
| PI 34:4; PI P-35:3 | 848.5283 | 0.00% | 0.00% |
| PI 34:3; PI P-35:2 | 850.5440 | 14.08% | 0.00% |
| PI 34:2; PI P-35:1 | 852.5596 | 14.09% | 0.00% |
| PI 34:1; PI P-35:0 | 854.5753 | 14.10% | 0.00% |
| PI 34:0; PI P-36:6 | 856.5909 | 14.11% | 0.00% |
| PI P-36:5; PI 35:6 | 858.5127 | 14.91% | 0.00% |
| PI 36:4 | 859.5331 | 0.00% | 0.00% |
| PI P-36:4; 35:5 | 860.5283 | 14.92% | 0.00% |
| PI P-36:3; PI 35:4 | 862.5440 | 14.93% | 0.00% |
| PI P-36:2; PI 35:3 | 864.5596 | 14.94% | 0.00% |

(continued)

| Lipid | m/z | Isotopic correction | |
|---|---|---|---|
| | | M+2 | M+1 |
| PI P-36:1; PI 35:2 | 866.5753 | 14.95% | 0.00% |
| PI P-36:0; PI 35:1 | 868.5909 | 14.96% | 0.00% |
| PI 36:7; PI O-36:0; PI 35:0 | 870.6066 | 14.97% | 0.00% |
| PI 36:6; PI P-37:5 | 872.5283 | 15.12% | 0.00% |
| PI 36:5; PI P-37:4 | 874.5440 | 15.13% | 0.00% |
| PI 36:4; PI P-37:3 | 876.5596 | 15.14% | 0.00% |
| PI 36:3; PI P-37:2 | 878.5753 | 15.15% | 0.00% |
| PI 36:2; PI P-37:1 | 880.5909 | 15.16% | 0.00% |
| PI 36:1; PI P-37:0 | 882.6066 | 15.18% | 0.00% |
| PI 36:0; PI P-38:6 | 884.6222 | 15.19% | 0.00% |
| PI 38:5 | 885.5488 | 0.00% | 0.00% |
| PI P-38:5; PI 37:6 | 886.5434 | 16.03% | 0.00% |
| PI 38:4 | 887.5644 | 0.00% | 0.00% |
| PI P-38:4; PI 37:5 | 888.5591 | 16.04% | 0.00% |
| PI 38:3 | 889.5801 | 0.00% | 0.00% |
| PI P-38:3; PI 37:4 | 890.5747 | 16.06% | 0.00% |
| PI P-38:2; PI 37:3 | 892.5909 | 16.07% | 0.00% |
| PI P-38:1; PI 37:2 | 894.6066 | 16.08% | 0.00% |
| PI P-38:0; PI 37:1 | 896.6222 | 16.09% | 0.00% |
| PI 38:7; PI O-38:0; PI 37:0 | 898.6379 | 16.10% | 0.00% |
| PI 38:6; PI P-39:5 | 900.5596 | 16.25% | 0.00% |
| PI 38:5; PI P-39:4 | 902.5753 | 16.26% | 0.00% |
| PI 38:4; PI P-40:10; PI P-29:3 | 904.5909 | 16.27% | 0.00% |
| PI 38:3; PI P-40:9; PI P-39:2 | 906.6066 | 16.28% | 0.00% |
| PI 38:2; PI P-40:8; PI P-39:1 | 908.6222 | 16.29% | 0.00% |
| PI 40:7 | 909.5488 | 0.00% | 0.00% |
| PI 38:1; PI P-40:7; PI P-39:0 | 910.6379 | 16.31% | 0.00% |
| PI 40:6 | 911.5644 | 0.00% | 0.00% |
| PI 38:0; PI P-40:6, PI O-39:0 | 912.6535 | 16.32% | 0.00% |
| PI P-40:5; PI 39:6 | 914.5753 | 17.21% | 0.00% |
| PI P-40:4; PI 39:5 | 916.5909 | 17.22% | 0.00% |
| PI P-40:3; PI 39:4 | 918.6066 | 17.23% | 0.00% |
| PI 40:10; PI P-40:2; PI 39:3 | 920.5283 | 17.25% | 0.00% |
| PI 40:9; PI P-40:1; PI 39:2 | 922.5440 | 17.38% | 0.00% |
| PI 40:8; PI P-40:0; PI 39:1 | 924.5596 | 17.40% | 0.00% |
| PI 40:7; PI O-40:0; PI 39:0 | 926.5753 | 17.41% | 0.00% |
| PI 40:6; PI P-41:5 | 928.5909 | 17.42% | 0.00% |
| PI 40:5; PI P-41:4 | 930.6066 | 17.43% | 0.00% |
| PI 40:4; PI P-42:10; PI P-41:3 | 932.6222 | 17.45% | 0.00% |
| PI 40:3; PI P-42:9; PI P-41:2 | 934.6379 | 17.46% | 0.00% |
| PI 40:2; PI P-42:8; PI P-41:1 | 936.6535 | 17.47% | 0.00% |
| PI 40:1; PI P-42:7; PI P-41:0 | 938.6692 | 17.48% | 0.00% |
| PI 40:0; PI P-42:6, PI O-41:0 | 940.6848 | 17.50% | 0.00% |
| PI P-42:5; PI 41:6 | 942.6066 | 18.43% | 0.00% |
| PI P-42:4; PI 41:5 | 944.6222 | 18.44% | 0.00% |
| PI P-42:3; PI 41:4 | 946.6379 | 18.46% | 0.00% |
| PI 42:10; PI P-42:2; PI 41:3 | 948.5596 | 18.47% | 0.00% |
| PI 42:9; PI P-42:1; PI 41:2 | 950.5753 | 18.61% | 0.00% |

(continued)

| Lipid | m/z | Isotopic correction | |
|---|---|---|---|
| | | M+2 | M+1 |
| PI 42:8; PI P-42:0; PI 41:1 | 952.5909 | 18.62% | 0.00% |
| PI 42:7; PI O-42:0 | 954.6066 | 18.63% | 0.00% |
| PI 42:6; PI P-43:5 | 956.6222 | 18.65% | 0.00% |
| PI 42:5; PI P-43:4 | 958.6379 | 18.66% | 0.00% |
| PI 42:4; PI P-43:3 | 960.6535 | 18.67% | 0.00% |
| PI 42:3; PI P-43:2 | 962.6692 | 18.68% | 0.00% |
| PI 42:2; PI P-43:1 | 964.6848 | 18.70% | 0.00% |
| PI 42:1; PI P-43:0 | 966.7005 | 18.71 % | 0.00% |
| PI 42:0; PI O-43:0 | 968.7161 | 18.72% | 0.00% |
| PI 44:0 | 996.7474 | 0.00% | 0.00% |
| LPC 6:0 | 356.1833 | 0.00% | 0.00% |
| LPC 8:0 | 384.2146 | 0.00% | 0.00% |
| LPC 10:0 | 412.2459 | 0.00% | 0.00% |
| LPC 12:0 | 440.2772 | 0.00% | 0.00% |
| LPC 14:0 | 468.3085 | 0.00% | 0.00% |
| LPC P-16:0; LPC O-16:1 | 480.3449 | 0.00% | 0.00% |
| LPC O-16:0; LPC 15:0 | 482.3241 | 4.78% | 0.00% |
| LPC 16:2 | 492.3085 | 0.00% | 0.00% |
| LPC 16:1 | 494.3241 | 4.98% | 0.00% |
| LPC 16:0 | 496.3398 | 4.99% | 0.00% |
| LPC P-18:0; LPC O-18:1; LPC 17:1 | 508.3767 | 0.00% | 0.00% |
| **LPC O-18:0; LPC 17:0 (IS)** | 510.3554 | 5.39% | 0.00% |
| LPC O-18:0 | 510.3918 | 0.00% | 0.00% |
| LPC 18:4 | 516.3085 | 0.00% | 0.00% |
| LPC 18:3 | 518.3241 | 5.58% | 0.00% |
| LPC 18:2 | 520.3398 | 5.58% | 0.00% |
| LPC 18:1 | 522.3554 | 5.59% | 0.00% |
| LPC 18:1 | 522.3554 | 5.59% | 0.00% |
| LPC 18:0 | 524.3711 | 5.60% | 0.00% |
| LPC P-20:0; LPC O-20:1 | 536.4075 | 0.00% | 0.00% |
| LPC O-20:0; LPC 19:0 | 538.3867 | 6.05% | 0.00% |
| LPC 20:5 | 542.3235 | 0.00% | 0.00% |
| LPC 20:4 | 544.3392 | 6.23% | 0.00% |
| LPC 20:3 | 546.3548 | 6.24% | 0.00% |
| LPC 20:2 | 548.3711 | 6.25% | 0.00% |
| LPC 20:1 | 550.3867 | 6.25% | 0.00% |
| LPC 20:0 | 552.4024 | 6.26% | 0.00% |
| LPC P-22:0; LPC O-22:1 | 564.4388 | 0.00% | 0.00% |
| LPC O-22:0; LPC 21:0 | 566.4180 | 6.76% | 0.00% |
| LPC 22:6 | 568.3398 | 6.77% | 0.00% |
| LPC 22:5 | 570.3548 | 6.93% | 0.00% |
| LPC 22:4 | 572.3704 | 6.94% | 0.00% |
| LPC 22:1 | 578.4180 | 0.00% | 0.00% |
| LPC 22:0 | 580.4337 | 6.97% | 0.00% |
| LPC P-24:0; LPC O-24:1 | 592.4701 | 0.00% | 0.00% |
| LPC O-24:0; LPC 23:0 | 594.4493 | 7.52% | 0.00% |
| LPC 24:1 | 606.4493 | 0.00% | 0.00% |
| LPC 24:0 | 608.4650 | 7.73% | 0.00% |

(continued)

| Lipid | m/z | Isotopic correction | |
|---|---|---|---|
| | | M+2 | M+1 |
| SM 28:2 | 617.4653 | 0.00% | 0.00% |
| SM 28:1 | 619.4809 | 8.04% | 0.00% |
| SM 28:0 | 621.4966 | 8.05% | 0.00% |
| LPC 25:0 | 622.4806 | 0.00% | 37.46% |
| SM 29:2 | 631.4809 | 0.00% | 0.00% |
| SM 29:1 | 633.4966 | 8.45% | 0.00% |
| SM 29:0 | 635.5123 | 8.46% | 0.00% |
| LPC 26:0 | 636.4963 | 0.00% | 38.57% |
| SM 30:2 | 645.4966 | 0.00% | 0.00% |
| **SM 30:1** | 647.5123 | 8.88% | 0.00% |
| SM 30:0 | 649.5279 | 8.89% | 0.00% |
| LPC 27:0 | 650.5119 | 0.00% | 39.67% |
| SM 31:2 | 659.5123 | 0.00% | 0.00% |
| SM 31:1 | 661.5279 | 9.32% | 0.00% |
| SM 31:0 | 663.5436 | 9.33% | 0.00% |
| LPC 28:0 | 664.5276 | 0.00% | 40.78% |
| SM 32:2 | 673.5279 | 0.00% | 0.00% |
| SM 32:1 | 675.5436 | 9.77% | 0.00% |
| SM d32:1 | 675.5436 | 0.00% | 0.00% |
| SM 32:0 | 677.5592 | 9.78% | 0.00% |
| PC 28:0 | 678.5068 | 0.00% | 41.88% |
| SM 33:2 | 687.5436 | 0.00% | 0.00% |
| SM 33:1 | 689.5592 | 10.23% | 0.00% |
| PC O-30:1/P-30:0 | 690.5432 | 0.00% | 0.00% |
| SM 33:0 | 691.5749 | 10.24% | 0.00% |
| PC 29:0 | 692.5225 | 0.00% | 42.99% |
| PC O-30:0 | 692.5589 | 10.30% | 0.00% |
| SM 34:2 | 701.5592 | 0.00% | 0.00% |
| SM 34:1 | 703.5749 | 10.70% | 0.00% |
| PC 30:1 | 704.5225 | 0.00% | 44.07% |
| SM 34:0 | 705.5905 | 10.71% | 42.64% |
| PC 30:0 | 706.5381 | 10.51% | 44.09% |
| SM 35:2 | 715.5749 | 0.00% | 0.00% |
| PC P-32:1; PC-31:2 | 716.5589 | 0.00% | 45.15% |
| PC O-32:2/P-32:1 | 716.5589 | 0.00% | 0.00% |
| SM 35:1 | 717.5905 | 11.19% | 44.81% |
| PC P-32:0; PC-31:1 | 718.5381 | 11.24% | 45.17% |
| PC O-32:1/P-32:0 | 718.5745 | 11.24% | 0.00% |
| SM 35:0 | 719.6062 | 11.20% | 44.83% |
| PC O-32:0; PC-31:0 | 720.5538 | 11.25% | 45.20% |
| PC O-32:0 | 720.5902 | 11.25% | 0.00% |
| PC 32:3; PC P-33:2 | 728.5225 | 0.00% | 0.00% |
| SM 36:2 | 729.5905 | 0.00% | 44.80% |
| PC 32:2; PC P-33:1 | 730.5381 | 11.44% | 46.26% |
| SM 36:1 | 731.6062 | 11.69% | 44.82% |
| PC 32:1; PC P-33:0 | 732.5538 | 11.46% | 46.28% |
| SM 36:0 | 733.6218 | 11.70% | 44.85% |
| PC 32:0; PC O-33:0 | 734.5694 | 11.47% | 46.30% |

(continued)

| Lipid | m/z | Isotopic correction | |
| --- | --- | --- | --- |
| | | M+2 | M+1 |
| PC O-34:3/P-34:2 | 742.5745 | 0.00% | 0.00% |
| SM 37:2 | 743.6062 | 0.00% | 0.00% |
| PC P-34:1; PC 33:2 | 744.5538 | 0.00% | 47.36% |
| PC O-34:2/P-34:1 | 744.5902 | 12.23% | 0.00% |
| SM 37:1 | 745.6218 | 12.20% | 47.02% |
| PC P-34:0; PC 33:1 | 746.5694 | 12.25% | 47.38% |
| PC O-34:1/P-34:0 | 746.6058 | 12.25% | 0.00% |
| SM 37:0 | 747.6375 | 12.21% | 47.04% |
| PC O-34:0;PC 33:0 | 748.5851 | 12.26% | 47.41% |
| PC 34:4; PC P-35:3 | 754.5382 | 0.00% | 0.00% |
| PC 34:3; PC P-35:2 | 756.5538 | 12.44% | 0.00% |
| SM 38:2 | 757.6218 | 0.00% | 47.01% |
| PC 34:2; PC P-35:1 | 758.5694 | 12.45% | 48.46% |
| SM 38:1 | 759.6375 | 12.72% | 47.03% |
| PC 34:1; PC P-35:0 | 760.5851 | 12.46% | 48.49% |
| SM 38:0 | 761.6531 | 12.74% | 47.06% |
| PC 34:0; PC P-36:6 | 762.6007 | 12.47% | 48.51% |
| PC P-36:5; PC 35:6 | 764.5226 | 13.24% | 0.00% |
| PC P-36:4; PC 35:5 | 766.5382 | 13.25% | 0.00% |
| PC O-36:5/P-36:4 | 766.5745 | 0.00% | 0.00% |
| PC P-36:3; PC 35:4 | 768.5538 | 13.26% | 0.00% |
| PC O-36:4/P-36:3 | 768.5902 | 13.26% | 0.00% |
| PC P-36:2; PC 35:3 | 770.5694 | 13.27% | 0.00% |
| PC O-36:3/P-36:2 | 770.6058 | 13.27% | 0.00% |
| SM 39:2 | 771.6375 | 0.00% | 49.20% |
| PC P-36:1; PC 35:2 | 772.5851 | 13.29% | 49.57% |
| SM 39:1 | 773.6531 | 13.26% | 49.23% |
| PC P-36:0; PC 35:1 | 774.6007 | 13.30% | 49.59% |
| SM 39:0 | 775.6688 | 13.27% | 49.25% |
| PC 36:7; PC O-36:0; PC 35:0 | 776.6164 | 13.31% | 49.62% |
| PC 36:6; PC P-37:5 | 778.5381 | 13.45% | 0.00% |
| PC 36:5; PC P-37:4 | 780.5538 | 13.46% | 0.00% |
| PC 36:4; PC P-37:3 | 782.5694 | 13.48% | 0.00% |
| SM 40:3 | 783.6375 | 0.00% | 49.20% |
| PC 36:3; PC P-37:2 | 784.5851 | 13.49% | 50.65% |
| SM 40:2 | 785.6531 | 13.80% | 49.22% |
| PC 36:2; PC P-37:1 | 786.6007 | 13.50% | 50.67% |
| SM 40:1 | 787.6688 | 13.81% | 49.24% |
| PC 36:1; PC P-37:0 | 788.6164 | 13.51% | 50.70% |
| SM 40:0 | 789.6844 | 13.82% | 49.26% |
| PC 36:0; PC P-38:6 | 790.6320 | 13.52% | 50.72% |
| PC P-38:5; PC 37:6 | 792.5532 | 14.34% | 0.00% |
| PC O-38:6/P-38:5 | 792.5902 | 0.00% | 0.00% |
| PC P-38:4; PC 37:5 | 794.5689 | 14.35% | 0.00% |
| PC O-38:5/P-38:4 | 794.6058 | 14.35% | 0.00% |
| PC P-38:3; PC 37:4 | 796.5845 | 14.36% | 0.00% |
| PC O-38:4/P-38:3 | 796.6215 | 14.36% | 0.00% |
| PC P-38:2; PC 37:3 | 798.6007 | 14.37% | 0.00% |

(continued)

| Lipid | m/z | Isotopic correction | |
|---|---|---|---|
| | | M+2 | M+1 |
| SM 41:2 | 799.6688 | 0.00% | 51.41% |
| PC P-38:1; PC 37:2 | 800.6164 | 14.39% | 51.78% |
| SM 41:1 | 801.6844 | 14.37% | 51.44% |
| PC P-38:0; PC 37:1 | 802.6320 | 14.40% | 51.80% |
| SM 41:0 | 803.7001 | 14.38% | 51.46% |
| PC 38:7 | 804.5538 | 0.00% | 0.00% |
| PC 38:7; PC O-38:0; PC 37:0 | 804.6477 | 14.41% | 51.82% |
| PC 38:6; PC P-39:5 | 806.5695 | 14.55% | 0.00% |
| PC 38:5; PC P-39:4 | 808.5852 | 14.56% | 0.00% |
| PC 38:4; PC P-40:10; PC P-29:3 | 810.6007 | 14.57% | 0.00% |
| SM d42:3 | 811.6688 | 0.00% | 0.00% |
| PC 38:3; PC P-40:9; PC P-39:2 | 812.6164 | 14.59% | 0.00% |
| SM 42:2 | 813.6844 | 0.00% | 51.43% |
| PC 38:2; PC P-40:8; PC P-39:1 | 814.6320 | 14.60% | 52.88% |
| SM 42:1 | 815.7001 | 14.94% | 51.45% |
| PC 38:1; PC P-40:7; PC P-39:0 | 816.6477 | 14.61% | 52.91% |
| SM 42:0 | 817.7157 | 14.95% | 51.47% |
| PC O-40:7/P-40:6 | 818.6058 | 0.00% | 0.00% |
| PC 38:0; PC P-40:6, PC O-39:0 | 818.6633 | 14.62% | 52.93% |
| PC P-40:5; PC 39:6 | 820.5852 | 15.48% | 0.00% |
| PC O-40:6/P-40:5 | 820.6215 | 15.48% | 0.00% |
| PC P-40:4; PC 39:5 | 822.6008 | 15.50% | 0.00% |
| PC O-40:5/P-40:4 | 822.6371 | 15.50% | 0.00% |
| PC P-40:3; PC 39:4 | 824.6164 | 15.51% | 0.00% |
| PC O-40:4/P-40:3 | 824.6528 | 15.51% | 0.00% |
| PC 40:10; PC P-40:2; PC 39:3 | 826.5382 | 15.52% | 0.00% |
| PC O-40:3/P-40:2 | 826.6684 | 15.52% | 0.00% |
| SM 43:2 | 827.7001 | 0.00% | 53.48% |
| PC 40:9; PC P-40:1; PC 39:2 | 828.5539 | 15.66% | 53.99% |
| PC O-40:2/P-40:1 | 828.6841 | 15.53% | 0.00% |
| SM 43:1 | 829.7157 | 15.52% | 53.50% |
| PC 40:8; PC P-40:0; PC 39:1 | 830.5695 | 15.67% | 54.01% |
| PC O-40:1/P-40:0 | 830.6997 | 15.55% | 0.00% |
| SM 43:0 | 831.7314 | 15.54% | 53.52% |
| PC 40:7; PC O-40:0; PC 39:0 | 832.5851 | 15.69% | 54.03% |
| PC 40:6; PC P-41:5 | 834.6007 | 15.70% | 0.00% |
| PC 40:5; PC P-41:4 | 836.6165 | 15.71% | 0.00% |
| PC 40:4; PC P-42:10; PC P-41:3 | 838.6321 | 15.72% | 0.00% |
| SM d44:3 | 839.7001 | 0.00% | 0.00% |
| PC 40:3; PC P-42:9; PC P-41:2 | 840.6478 | 15.73% | 0.00% |
| SM 44:2 | 841.7157 | 0.00% | 55.67% |
| PC 40:2; PC P-42:8; PC P-41:1 | 842.6633 | 15.75% | 55.09% |
| SM 44:1 | 843.7314 | 16.12% | 55.69% |
| PC 40:1; PC P-42:7; PC P-41:0 | 844.6790 | 15.76% | 55.11% |
| SM 44:0 | 845.7470 | 16.13% | 55.72% |
| PC 40:0; PC P-42:6, PC O-41:0 | 846.6946 | 15.77% | 55.14% |
| PC P-42:5; PC 41:6 | 848.6164 | 16.68% | 0.00% |
| PC O-42:6/P-42:5 | 848.6528 | 0.00% | 0.00% |

(continued)

| Lipid | m/z | Isotopic correction | |
|---|---|---|---|
| | | M+2 | M+1 |
| PC P-42:4; PC 41:5 | 850.6321 | 16.69% | 0.00% |
| PC O-42:5/P-42:4 | 850.6684 | 16.69% | 0.00% |
| PC P-42:3; PC 41:4 | 852.6477 | 16.71% | 0.00% |
| PC O-42:4/P-42:3 | 852.6841 | 16.71% | 0.00% |
| PC 42:10; PC P-42:2; PC 41:3 | 854.5695 | 16.72% | 0.00% |
| PC O-42:3/P-42:2 | 854.6997 | 16.72% | 0.00% |
| PC 42:9; PC P-42:1; PC 41:2 | 856.5852 | 16.85% | 0.00% |
| PC O-42:2/P-42:1 | 856.7154 | 16.73% | 0.00% |
| PC 42:8; PC P-42:0; PC 41:1 | 858.6008 | 16.87% | 0.00% |
| PC 42:7; PC O-42:0 | 860.6164 | 16.88% | 0.00% |
| PC 42:6; PC P-43:5 | 862.6320 | 16.89% | 0.00% |
| PC 42:5; PC P-43:4 | 864.6477 | 16.91% | 0.00% |
| PC 42:4; PC P-43:3 | 866.6633 | 16.92% | 0.00% |
| PC 42:3; PC P-43:2 | 868.6790 | 16.93% | 0.00% |
| PC 42:2; PC P-43:1 | 870.6946 | 16.94% | 0.00% |
| PC 42:1; PC P-43:0 | 872.7103 | 16.96% | 0.00% |
| PC 42:0; PC O-43:0 | 874.7259 | 16.97% | 0.00% |
| PC O-44:6/P-44:5 | 876.6841 | 0.00% | 0.00% |
| PC O-44:5/P-44:4 | 878.6997 | 17.94% | 0.00% |
| PC O-44:4/P-44:3 | 880.7154 | 17.95% | 0.00% |
| PC 44:0 | 902.7573 | 0.00% | 0.00% |
| Sulfatide 30:2 | 722.4473 | 0.00% | 0.00% |
| **Sulfatide 30:1** | 724.4630 | 15.10% | 0.00% |
| Sulfatide 30:0 | 726.4786 | 15.11% | 0.00% |
| Sulfatide 31:2 | 736.4630 | 0.00% | 0.00% |
| Sulfatide 31:1; Sulfatide 30:2 (1OH) | 738.4786 | 15.55% | 0.00% |
| Sulfatide 31:0; Sulfatide 30:1 (1OH) | 740.4943 | 15.56% | 0.00% |
| Sulfatide 30:0 (1OH) | 742.4735 | 15.57% | 0.00% |
| Sulfatide 32:2 | 750.4786 | 0.00% | 0.00% |
| Sulfatide 32:1; Sulfatide 31:2 (1OH) | 752.4946 | 16.02% | 0.00% |
| Sulfatide 32:0; Sulfatide 31:1 (1OH) | 754.5099 | 16.03% | 0.00% |
| Sulfatide 31:0 (1OH) | 756.4892 | 16.04% | 0.00% |
| Sulfatide 33:2 | 764.4943 | 0.00% | 0.00% |
| Sulfatide 33:1; Sulfatide 32:2 (1OH) | 766.5099 | 16.50% | 0.00% |
| Sulfatide 33:0; Sulfatide 32:1 (1OH) | 768.5255 | 16.51% | 0.00% |
| Sulfatide 32:0 (1OH) | 770.5048 | 16.52% | 0.00% |
| Sulfatide 34:2 | 778.5099 | 0.00% | 0.00% |
| Sulfatide 34:1; Sulfatide 33:2 (1OH) | 780.5255 | 17.00% | 0.00% |
| Sulfatide 34:0; Sulfatide 33:1 (1OH) | 782.5412 | 17.01% | 0.00% |
| Sulfatide 33:0 (1OH) | 784.5205 | 17.02% | 0.00% |
| Sulfatide 35:2 | 792.5255 | 0.00% | 0.00% |
| Sulfatide 35:1; Sulfatide 34:2 (1OH) | 794.5412 | 17.50% | 0.00% |
| Sulfatide 35:0; Sulfatide 34:1 (1OH) | 796.5569 | 17.51% | 0.00% |
| Sulfatide 34:0 (1OH) | 798.5361 | 17.52% | 0.00% |
| Sulfatide 36:2 | 806.5412 | 0.00% | 0.00% |
| Sulfatide 36:1; Sulfatide 35:2 (1OH) | 808.5569 | 18.02% | 0.00% |
| Sulfatide 36:0; Sulfatide 35:1 (1OH) | 810.5725 | 18.03% | 0.00% |
| Sulfatide 35:0 (1OH) | 812.5518 | 18.05% | 0.00% |

(continued)

| Lipid | m/z | Isotopic correction | |
|---|---|---|---|
| | | M+2 | M+1 |
| Sulfatide 37:2 | 820.5569 | 0.00% | 0.00% |
| Sulfatide 37:1; Sulfatide 36:2 (1OH) | 822.5725 | 18.55% | 0.00% |
| Sulfatide 37:0; Sulfatide 36:1 (1OH) | 824.5882 | 18.56% | 0.00% |
| Sulfatide 36:0 (1OH) | 826.5674 | 18.57% | 0.00% |
| Sulfatide 38:3 | 832.5569 | 0.00% | 0.00% |
| Sulfatide 38:2 | 834.5725 | 19.08% | 0.00% |
| Sulfatide 38:1; Sulfatide 37:2 (1OH) | 836.5882 | 19.09% | 0.00% |
| Sulfatide 38:0; Sulfatide 37:1 (1OH) | 838.6038 | 19.10% | 0.00% |
| Sulfatide 37:0 (1OH) | 840.5831 | 19.11% | 0.00% |
| Sulfatide 39:2; Sulfatide 38:3 (1OH) | 848.5882 | 0.00% | 0.00% |
| Sulfatide 39:1; Sulfatide 38:2 (1OH) | 850.6038 | 19.65% | 0.00% |
| Sulfatide 39:0; Sulfatide 38:1 (1OH) | 852.6195 | 19.66% | 0.00% |
| Sulfatide 38:0 (1OH) | 854.5987 | 19.67% | 0.00% |
| Sulfatide 40:3 | 860.5881 | 0.00% | 0.00% |
| Sulfatide 40:2 | 862.6038 | 20.20% | 0.00% |
| Sulfatide 40:1; Sulfatide 39:2 (1OH) | 864.6195 | 20.22% | 0.00% |
| Sulfatide 40:0; Sulfatide 39:1 (1OH) | 866.6351 | 20.23% | 0.00% |
| Sulfatide 39:0 (1OH) | 868.6144 | 20.24% | 0.00% |
| Sulfatide 41:2; Sulfatide 40:3 (1OH) | 876.6195 | 0.00% | 0.00% |
| Sulfatide 41:1; Sulfatide 40:2 (1OH) | 878.6351 | 20.79% | 0.00% |
| Sulfatide 41:0; Sulfatide 40:1 (1OH) | 880.6508 | 20.80% | 0.00% |
| Sulfatide 40:0 (1OH) | 882.6300 | 20.81% | 0.00% |
| Sulfatide 42:3 | 888.6195 | 0.00% | 0.00% |
| Sulfatide 42:2 | 890.6351 | 21.37% | 0.00% |
| Sulfatide 42:1; Sulfatide 41:2 (1OH) | 892.6508 | 21.38% | 0.00% |
| Sulfatide 42:0; Sulfatide 41:1 (1OH) | 894.6664 | 21.40% | 0.00% |
| Sulfatide 41:0 (1OH) | 896.6457 | 21.41% | 0.00% |
| Sulfatide 43:2; Sulfatide 42:3 (1OH) | 904.6508 | 0.00% | 0.00% |
| Sulfatide 43:1; Sulfatide 42:2 (1OH) | 906.6664 | 21.98% | 0.00% |
| Sulfatide 43:0; Sulfatide 42:1 (1OH) | 908.6821 | 22.00% | 0.00% |
| Sulfatide 42:0 (1OH) | 910.6613 | 22.01% | 0.00% |
| Sulfatide 44:3 | 916.6508 | 0.00% | 0.00% |
| Sulfatide 44:2 | 918.6664 | 22.59% | 0.00% |
| Sulfatide 44:1; Sulfatide 43:2 (1OH) | 920.6821 | 22.60% | 0.00% |
| Sulfatide 44:0; Sulfatide 43:1 (1OH) | 922.6977 | 22.62% | 0.00% |
| Sulfatide 43:0 (1OH) | 924.6770 | 22.63% | 0.00% |
| LPA 6:0 | 288.1206 | 0.00% | 0.00% |
| LPA 8:0 | 316.1519 | 0.00% | 0.00% |
| LPA 10:0 | 344.1832 | 0.00% | 0.00% |
| LPA 12:0 | 372.2145 | 0.00% | 0.00% |
| **LPA 14:0** (IS) | 400.2458 | 0.00% | 0.00% |
| LPA P-16:0; LPA O-16:1 | 412.2822 | 0.00% | 0.00% |
| LPA O-16:0; LPA 15:0 | 414.2615 | 3.46% | 0.00% |
| LPA 16:2 | 424.2458 | 0.00% | 0.00% |
| LPA 16:1 | 426.2615 | 3.67% | 0.00% |
| LPA 16:0 | 428.2771 | 3.67% | 0.00% |
| LPA P-18:0; LPA O-18:1; LPA 17:1 | 440.3140 | 0.00% | 0.00% |
| LPA O-18:0; LPA 17:0 | 442.2928 | 3.95% | 0.00% |

(continued)

| Lipid | m/z | Isotopic correction | |
|---|---|---|---|
| | | M+2 | M+1 |
| LPA 18:4 | 448.2458 | 0.00% | 0.00% |
| LPA 18:3 | 450.2615 | 4.15% | 0.00% |
| LPA 18:2 | 452.2771 | 4.15% | 0.00% |
| LPA 18:1 | 454.2928 | 4.16% | 0.00% |
| LPA 18:0 | 456.3084 | 4.16% | 0.00% |
| LPA P-20:0; LPA O-20:1 | 468.3448 | 0.00% | 0.00% |
| LPA O-20:0; LPA 19:0 | 470.3241 | 4.49% | 0.00% |
| LPA 20:5 | 474.2609 | 0.00% | 0.00% |
| LPA 20:4 | 476.2766 | 4.68% | 0.00% |
| LPA 20:3 | 478.2922 | 4.69% | 0.00% |
| LPA 20:2 | 480.3084 | 4.69% | 0.00% |
| LPA 20:1 | 482.3241 | 4.70% | 0.00% |
| LPA 20:0 | 484.3397 | 4.70% | 0.00% |
| LPA P-22:0; LPA O-22:1 | 496.3761 | 0.00% | 0.00% |
| LPA O-22:0; LPA 21:0 | 498.3554 | 5.08% | 0.00% |
| LPA 22:6 | 500.2771 | 5.09% | 0.00% |
| LPA 22:5 | 502.2922 | 5.26% | 0.00% |
| LPA 22:4 | 504.3078 | 5.27% | 0.00% |
| LPA 22:1 | 510.3554 | 0.00% | 0.00% |
| LPA 22:0 | 512.3710 | 5.29% | 0.00% |
| LPA P-24:0; LPA O-24:1 | 524.4074 | 0.00% | 0.00% |
| LPA O-24:0; LPA 23:0 | 526.3867 | 5.72% | 0.00% |
| LPA 24:1 | 538.3867 | 0.00% | 0.00% |
| LPA 24:0 | 540.4023 | 5.93% | 0.00% |
| LPA 25:0 | 554.4180 | 0.00% | 0.00% |
| LPA 26:0 | 568.4336 | 0.00% | 0.00% |
| LPA 27:0 | 582.4493 | 0.00% | 0.00% |
| LPA 28:0 | 596.4649 | 0.00% | 0.00% |
| **PA 28:0 (IS)** | 610.4442 | 0.00% | 0.00% |
| PA 29:0 | 624.4598 | 0.00% | 0.00% |
| PA 30:1 | 636.4598 | 0.00% | 0.00% |
| PA 30:0 | 638.4755 | 8.35% | 0.00% |
| PA P-32:1; PA-31:2 | 648.4962 | 0.00% | 0.00% |
| PA P-32:0; PA-31:1 | 650.4755 | 8.96% | 0.00% |
| PA O-32:0; PA-31:0 | 652.4911 | 8.97% | 0.00% |
| PA 32:3; PA P-33:2 | 660.4598 | 0.00% | 0.00% |
| PA 32:2; PA P-33:1 | 662.4755 | 9.16% | 0.00% |
| PA 32:1; PA P-33:0 | 664.4911 | 9.17% | 0.00% |
| PA 32:0; PA O-33:0 | 666.5068 | 9.18% | 0.00% |
| PA P-34:1; PA 33:2 | 676.4911 | 0.00% | 0.00% |
| PA P-34:0; PA 33:1 | 678.5068 | 9.84% | 0.00% |
| PA O-34:0;PA 33:0 | 680.5224 | 9.85% | 0.00% |
| PA 34:4; PA P-35:3 | 686.4755 | 0.00% | 0.00% |
| PA 34:3; PA P-35:2 | 688.4911 | 10.03% | 0.00% |
| PA 34:2; PA P-35:1 | 690.5068 | 10.04% | 0.00% |
| PA 34:1; PA P-35:0 | 692.5224 | 10.05% | 0.00% |
| PA 34:0; PA P-36:6 | 694.5381 | 10.06% | 0.00% |
| PA P-36:5; PA 35:6 | 696.4599 | 10.72% | 0.00% |

(continued)

| Lipid | m/z | Isotopic correction | |
|---|---|---|---|
| | | M+2 | M+1 |
| PA P-36:4; PA 35:5 | 698.4755 | 10.73% | 0.00% |
| PA P-36:3; PA 35:4 | 700.4911 | 10.74% | 0.00% |
| PA P-36:2; PA 35:3 | 702.5068 | 10.75% | 0.00% |
| PA P-36:1; PA 35:2 | 704.5224 | 10.76% | 0.00% |
| PA P-36:0; PA 35:1 | 706.5381 | 10.77% | 0.00% |
| PA 36:7; PA O-36:0; PA 35:0 | 708.5537 | 10.79% | 0.00% |
| PA 36:6; PA P-37:5 | 710.4755 | 10.93% | 0.00% |
| PA 36:5; PA P-37:4 | 712.4911 | 10.94% | 0.00% |
| PA 36:4; PA P-37:3 | 714.5068 | 10.95% | 0.00% |
| PA 36:3; PA P-37:2 | 716.5224 | 10.97% | 0.00% |
| PA 36:2; PA P-37:1 | 718.5381 | 10.98% | 0.00% |
| PA 36:1; PA P-37:0 | 720.5537 | 10.99% | 0.00% |
| PA 36:0; PA P-38:6 | 722.5694 | 11.00% | 0.00% |
| PA P-38:5; PA 37:6 | 724.4905 | 11.70% | 0.00% |
| PA P-38:4; PA 37:5 | 726.5062 | 11.71% | 0.00% |
| PA P-38:3; PA 37:4 | 728.5218 | 11.72% | 0.00% |
| PA P-38:2; PA 37:3 | 730.5381 | 11.73% | 0.00% |
| PA P-38:1; PA 37:2 | 732.5537 | 11.74% | 0.00% |
| PA P-38:0; PA 37:1 | 734.5694 | 11.75% | 0.00% |
| PA 38:7; PA O-38:0; PA 37:0 | 736.5850 | 11.76% | 0.00% |
| PA 38:6; PA P-39:5 | 738.5068 | 11.91% | 0.00% |
| PA 38:5; PA P-39:4 | 740.5225 | 11.92% | 0.00% |
| PA 38:4; PA P-40:10; PA P-29:3 | 742.5381 | 11.93% | 0.00% |
| PA 38:3; PA P-40:9; PA P-39:2 | 744.5537 | 11.941% | 0.00% |
| PA 38:2; PA P-40:8; PA P-39:1 | 746.5694 | 11.95% | 0.00% |
| PA 38:1; PA P-40:7; PA P-39:0 | 748.5850 | 11.97% | 0.00% |
| PA 38:0; PA P-40:6, PA O-39:0 | 750.6007 | 11.98% | 0.00% |
| PA P-40:5; PA 39:6 | 752.5225 | 12.72% | 0.00% |
| PA P-40:4; PA 39:5 | 754.5381 | 12.73% | 0.00% |
| PA P-40:3; PA 39:4 | 756.5537 | 12.74% | 0.00% |
| PA 40:10; PA P-40:2; PA 39:3 | 758.4755 | 12.76% | 0.00% |
| PA 40:9; PA P-40:1; PA 39:2 | 760.4912 | 12.90% | 0.00% |
| PA 40:8; PA P-40:0; PA 39:1 | 762.5068 | 12.91% | 0.00% |
| PA 40:7; PA O-40:0; PA 39:0 | 764.5224 | 12.92% | 0.00% |
| PA 40:6; PA P-41:5 | 766.5381 | 12.93% | 0.00% |
| PA 40:5; PA P-41:4 | 768.5538 | 12.95% | 0.00% |
| PA 40:4; PA P-42:10; PA P-41:3 | 770.5694 | 12.96% | 0.00% |
| PA 40:3; PA P-42:9; PA P-41:2 | 772.5851 | 12.97% | 0.00% |
| PA 40:2; PA P-42:8; PA P-41:1 | 774.6007 | 12.98% | 0.00% |
| PA 40:1; PA P-42:7; PA P-41:0 | 776.6163 | 12.99% | 0.00% |
| PA 40:0; PA P-42:6, PA O-41:0 | 778.6320 | 13.01% | 0.00% |
| PA P-42:5; PA 41:6 | 780.5537 | 13.79% | 0.00% |
| PA P-42:4; PA 41:5 | 782.5694 | 13.81% | 0.00% |
| PA P-42:3; PA 41:4 | 784.5850 | 13.82% | 0.00% |
| PA 42:10; PA P-42:2; PA 41:3 | 786.5068 | 13.83% | 0.00% |
| PA 42:9; PA P-42:1; PA 41:2 | 788.5225 | 13.97% | 0.00% |
| PA 42:8; PA P-42:0; PA 41:1 | 790.5381 | 13.98% | 0.00% |
| PA 42:7; PA O-42:0 | 792.5537 | 13.99% | 0.00% |

(continued)

| Lipid | m/z | Isotopic correction | |
|---|---|---|---|
| | | M+2 | M+1 |
| PA 42:6; PA P-43:5 | 794.5694 | 14.01% | 0.00% |
| PA 42:5; PA P-43:4 | 796.5850 | 14.02% | 0.00% |
| PA 42:4; PA P-43:3 | 798.6007 | 14.03% | 0.00% |
| PA 42:3; PA P-43:2 | 800.6163 | 14.05% | 0.00% |
| PA 42:2; PA P-43:1 | 802.6320 | 14.06% | 0.00% |
| PA 42:1; PA P-43:0 | 804.6476 | 14.07% | 0.00% |
| PA 42:0; PA O-43:0 | 806.6633 | 14.08% | 0.00% |
| PA 44:0 | 834.6946 | 0.00% | 0.00% |
| S1P d16:1 | 374.2067 | 0.00% | 0.00% |
| S1P d16:0 | 376.2223 | 2.60% | 0.00% |
| S1P d17:0 | 386.2067 | 0.00% | 0.00% |
| S1P d17:1 | 388.2223 | 2.80% | 0.00% |
| S1P d18:2 | 400.2223 | 0.00% | 0.00% |
| S1P d18:1 | 402.2380 | 3.01% | 0.00% |
| S1P d18:0 | 404.2536 | 3.02% | 0.00% |
| S1P d19:1 | 414.2380 | 0.00% | 0.00% |
| S1P d19:0 | 416.2531 | 3.24% | 0.00% |
| S1P d20:1 | 430.2693 | 0.00% | 0.00% |
| S1P d20:0 | 432.2849 | 3.48% | 0.00% |
| LPS 6:0 | 358.1261 | 0.00% | 0.00% |
| LPS 8:0 | 386.1574 | 0.00% | 0.00% |
| LPS 10:0 | 414.1887 | 0.00% | 0.00% |
| LPS 12:0 | 442.2200 | 0.00% | 0.00% |
| LPS 14:0 | 470.2513 | 0.00% | 0.00% |
| LPS P-16:0; LPS O-16:1 | 482.2877 | 0.00% | 0.00% |
| LPS O-16:0; LPS 15:0 | 484.2670 | 4.64% | 0.00% |
| LPS 16:2 | 494.2513 | 0.00% | 0.00% |
| LPS 16:1 | 496.2670 | 4.84% | 0.00% |
| LPS 16:0 | 498.2826 | 4.85% | 0.00% |
| **LPS P-18:0; LPS O-18:1; LPS 17:1 (IS)** | 510.3196 | 0.00% | 0.00% |
| LPS O-18:0; LPS 17:0 | 512.2983 | 5.20% | 0.00% |
| LPS 18:4 | 518.2513 | 0.00% | 0.00% |
| LPS 18:3 | 520.2670 | 5.39% | 0.00% |
| LPS 18:2 | 522.2826 | 5.40% | 0.00% |
| LPS 18:1 | 524.2983 | 5.40% | 0.00% |
| LPS 18:0 | 526.3139 | 5.41% | 0.00% |
| LPS P-20:0; LPS O-20:1 | 538.3503 | 0.00% | 0.00% |
| LPS O-20:0; LPS 19:0 | 540.3296 | 5.81% | 0.00% |
| LPS 20:5 | 544.2664 | 0.00% | 0.00% |
| LPS 20:4 | 546.2821 | 6.00% | 0.00% |
| LPS 20:3 | 548.2977 | 6.00% | 0.00% |
| LPS 20:2 | 550.3139 | 6.01% | 0.00% |
| LPS 20:1 | 552.3296 | 6.02% | 0.00% |
| LPS 20:0 | 554.3452 | 6.02% | 0.00% |
| LPS P-22:0; LPS O-22:1 | 566.3816 | 0.00% | 0.00% |
| LPS O-22:0; LPS 21:0 | 568.3609 | 6.48% | 0.00% |
| LPS 22:6 | 570.2826 | 6.48% | 0.00% |
| LPS 22:5 | 572.2977 | 6.65% | 0.00% |

(continued)

| Lipid | m/z | Isotopic correction | |
|---|---|---|---|
| | | M+2 | M+1 |
| LPS 22:4 | 574.3133 | 6.66% | 0.00% |
| LPS 22:1 | 580.3609 | 0.00% | 0.00% |
| LPS 22:0 | 582.3765 | 6.69% | 0.00% |
| LPS P-24:0; LPS O-24:1 | 594.4129 | 0.00% | 0.00% |
| LPS O-24:0; LPS 23:0 | 596.3922 | 7.19% | 0.00% |
| LPS 24:1 | 608.3922 | 0.00% | 0.00% |
| LPS 24:0 | 610.4078 | 7.40% | 0.00% |
| LPS 25:0 | 624.4235 | 0.00% | 0.00% |
| LPS 26:0 | 638.4391 | 0.00% | 0.00% |
| LPS 27:0 | 652.4548 | 0.00% | 0.00% |
| LPS 28:0 | 666.4704 | 0.00% | 0.00% |
| **PS 28:0 (IS)** | 680.4497 | 0.00% | 0.00% |
| PS 29:0 | 694.4653 | 0.00% | 0.00% |
| PS 30:1 | 706.4653 | 0.00% | 0.00% |
| PS 30:0 | 708.4810 | 10.04% | 0.00% |
| PS P-32:1; PS-31:2 | 718.5017 | 0.00% | 0.00% |
| PS P-32:0; PS-31:1 | 720.4810 | 10.72% | 0.00% |
| PS O-32:0; PS-31:0 | 722.4966 | 10.73% | 0.00% |
| PS 32:3; PS P-33:2 | 730.4653 | 0.00% | 0.00% |
| PS 32:2; PS P-33:1 | 732.4810 | 10.92% | 0.00% |
| PS 32:1; PS P-33:0 | 734.4966 | 10.93% | 0.00% |
| PS 32:0; PS O-33:0 | 736.5123 | 10.94% | 0.00% |
| PS P-34:1; PS 33:2 | 746.4966 | 0.00% | 0.00% |
| PS P-34:0; PS 33:1 | 748.5123 | 11.68% | 0.00% |
| PS O-34:0; PS 33:0 | 750.5279 | 11.69% | 0.00% |
| PS 34:4; PS P-35:3 | 756.4810 | 0.00% | 0.00% |
| PS 34:3; PS P-35:2 | 758.4966 | 11.87% | 0.00% |
| PS 34:2; PS P-35:1 | 760.5123 | 11.88% | 0.00% |
| PS 34:1; PS P-35:0 | 762.5279 | 11.89% | 0.00% |
| PS 34:0; PS P-36:6 | 764.5436 | 11.90% | 0.00% |
| PS P-36:5; PS 35:6 | 766.4654 | 12.67% | 0.00% |
| PS P-36:4; PS 35:5 | 768.4810 | 12.64% | 0.00% |
| PS P-36:3; PS 35:4 | 770.4966 | 12.65% | 0.00% |
| PS P-36:2; PS 35:3 | 772.5123 | 12.66% | 0.00% |
| PS P-36:1; PS 35:2 | 774.5279 | 12.67% | 0.00% |
| PS P-36:0; PS 35:1 | 776.5436 | 12.68% | 0.00% |
| PS 36:7; PS O-36:0; PS 35:0 | 778.5593 | 12.69% | 0.00% |
| PS 36:6; PS P-37:5 | 780.4810 | 12.84% | 0.00% |
| PS 36:5; PS P-37:4 | 782.4966 | 12.85% | 0.00% |
| PS 36:4; PS P-37:3 | 784.5123 | 12.86% | 0.00% |
| PS 36:3; PS P-37:2 | 786.5279 | 12.87% | 0.00% |
| PS 36:2; PS P-37:1 | 788.5436 | 12.88% | 0.00% |
| PS 36:1; PS P-37:0 | 790.5593 | 12.90% | 0.00% |
| PS 36:0; PS P-38:6 | 792.5749 | 12.91% | 0.00% |
| PS P-38:5; PS 37:6 | 794.4961 | 13.68% | 0.00% |
| PS P-38:4; PS 37:5 | 796.5118 | 13.69% | 0.00% |
| PS P-38:3; PS 37:4 | 798.5274 | 13.70% | 0.00% |
| PS P-38:2; PS 37:3 | 800.5436 | 13.71% | 0.00% |

(continued)

| Lipid | m/z | Isotopic correction | |
|---|---|---|---|
| | | M+2 | M+1 |
| PS P-38:1; PS 37:2 | 802.5593 | 13.72% | 0.00% |
| PS P-38:0; PS 37:1 | 804.5749 | 13.74% | 0.00% |
| PS 38:7; PS O-38:0; PS 37:0 | 806.5906 | 13.75% | 0.00% |
| PS 38:6; PS P-39:5 | 808.5123 | 13.89% | 0.00% |
| PS 38:5; PS P-39:4 | 810.5280 | 13.90% | 0.00% |
| PS 38:4; PS P-40:10; PS P-29:3 | 812.5436 | 13.91% | 0.00% |
| PS 38:3; PS P-40:9; PS P-39:2 | 814.5593 | 13.925% | 0.00% |
| PS 38:2; PS P-40:8; PS P-39:1 | 816.5749 | 13.94% | 0.00% |
| PS 38:1; PS P-40:7; PS P-39:0 | 818.5906 | 13.95% | 0.00% |
| PS 38:0; PS P-40:6, PS O-39:0 | 820.6062 | 13.96% | 0.00% |
| PS P-40:5; PS 39:6 | 822.5280 | 14.78% | 0.00% |
| PS P-40:4; PS 39:5 | 824.5436 | 14.79% | 0.00% |
| PS P-40:3; PS 39:4 | 826.5593 | 14.80% | 0.00% |
| PS 40:10; PS P-40:2; PS 39:3 | 828.4810 | 14.81% | 0.00% |
| PS 40:9; PS P-40:1; PS 39:2 | 830.4967 | 14.95% | 0.00% |
| PS 40:8; PS P-40:0; PS 39:1 | 832.5123 | 14.96% | 0.00% |
| PS 40:7; PS O-40:0; PS 39:0 | 834.5279 | 14.98% | 0.00% |
| PS 40:6; PS P-41:5 | 836.5436 | 14.99% | 0.00% |
| PS 40:5; PS P-41:4 | 838.5593 | 15.00% | 0.00% |
| PS 40:4; PS P-42:10; PS P-41:3 | 840.5749 | 15.01% | 0.00% |
| PS 40:3; PS P-42:9; PS P-41:2 | 842.5906 | 15.03% | 0.00% |
| PS 40:2; PS P-42:8; PS P-41:1 | 844.6062 | 15.04% | 0.00% |
| PS 40:1; PS P-42:7; PS P-41:0 | 846.6219 | 15.05% | 0.00% |
| PS 40:0; PS P-42:6, PS O-41:0 | 848.6375 | 15.06% | 0.00% |
| PS P-42:5; PS 41:6 | 850.5593 | 15.92% | 0.00% |
| PS P-42:4; PS 41:5 | 852.5749 | 15.94% | 0.00% |
| PS P-42:3; PS 41:4 | 854.5906 | 15.95% | 0.00% |
| PS 42:10; PS P-42:2; PS 41:3 | 856.5123 | 15.96% | 0.00% |
| PS 42:9; PS P-42:1; PS 41:2 | 858.5280 | 16.10% | 0.00% |
| PS 42:8; PS P-42:0; PS 41:1 | 860.5436 | 16.11% | 0.00% |
| PS 42:7; PS O-42:0 | 862.5593 | 16.12% | 0.00% |
| PS 42:6; PS P-43:5 | 864.5749 | 16.14% | 0.00% |
| PS 42:5; PS P-43:4 | 866.5906 | 16.15% | 0.00% |
| PS 42:4; PS P-43:3 | 868.6062 | 16.16% | 0.00% |
| PS 42:3; PS P-43:2 | 870.6219 | 16.18% | 0.00% |
| PS 42:2; PS P-43:1 | 872.6375 | 16.19% | 0.00% |
| PS 42:1; PS P-43:0 | 874.6532 | 16.20% | 0.00% |
| PS 42:0; PS O-43:0 | 876.6688 | 16.21% | 0.00% |
| PS 44:0 | 904.7001 | 0.00% | 0.00% |
| LPG 6:0 | 362.1574 | 0.00% | 0.00% |
| LPG 8:0 | 390.1887 | 0.00% | 0.00% |
| LPG 10:0 | 418.2200 | 0.00% | 0.00% |
| LPG 12:0 | 446.2513 | 0.00% | 0.00% |
| **LPG 14:0 (IS)** | 474.2826 | 0.00% | 0.00% |
| LPG P-16:0; LPG O-16:1 | 486.3190 | 0.00% | 0.00% |
| LPG O-16:0; LPG 15:0 | 488.2983 | 4.65% | 0.00% |
| LPG 16:2 | 498.2826 | 0.00% | 0.00% |
| LPG 16:1 | 500.2983 | 4.85% | 0.00% |

(continued)

| Lipid | m/z | Isotopic correction | |
|---|---|---|---|
| | | M+2 | M+1 |
| LPG 16:0 | 502.3139 | 4.86% | 0.00% |
| LPG P-18:0; LPG O-18:1; LPG 17:1 | 514.3508 | 0.00% | 0.00% |
| LPG O-18:0; LPG 17:0 | 516.3296 | 5.21% | 0.00% |
| LPG 18:4 | 522.2826 | 0.00% | 0.00% |
| LPG 18:3 | 524.2983 | 5.40% | 0.00% |
| LPG 18:2 | 526.3139 | 5.41% | 0.00% |
| LPG 18:1 | 528.3296 | 5.42% | 0.00% |
| LPG 18:0 | 530.3452 | 5.42% | 0.00% |
| LPG P-20:0; LPG O-20:1 | 542.3816 | 0.00% | 0.00% |
| LPG O-20:0; LPG 19:0 | 544.3609 | 5.83% | 0.00% |
| LPG 20:5 | 548.2977 | 0.00% | 0.00% |
| LPG 20:4 | 550.3134 | 6.01% | 0.00% |
| LPG 20:3 | 552.3290 | 6.02% | 0.00% |
| LPG 20:2 | 554.3452 | 6.02% | 0.00% |
| LPG 20:1 | 556.3609 | 6.03% | 0.00% |
| LPG 20:0 | 558.3765 | 6.04% | 0.00% |
| LPG P-22:0; LPG O-22:1 | 570.4129 | 0.00% | 0.00% |
| LPG O-22:0; LPG 21:0 | 572.3922 | 6.49% | 0.00% |
| LPG 22:6 | 574.3139 | 6.50% | 0.00% |
| LPG 22:5 | 576.3290 | 6.67% | 0.00% |
| LPG 22:4 | 578.3446 | 6.67% | 0.00% |
| LPG 22:1 | 584.3922 | 0.00% | 0.00% |
| LPG 22:0 | 586.4078 | 6.70% | 0.00% |
| LPG P-24:0; LPG O-24:1 | 598.4442 | 0.00% | 0.00% |
| LPG O-24:0; LPG 23:0 | 600.4235 | 7.20% | 0.00% |
| LPG 24:1 | 612.4235 | 0.00% | 0.00% |
| LPG 24:0 | 614.4391 | 7.41% | 0.00% |
| LPG 25:0 | 628.4548 | 0.00% | 0.00% |
| LPG 26:0 | 642.4704 | 0.00% | 0.00% |
| LPG 27:0 | 656.4861 | 0.00% | 0.00% |
| LPG 28:0 | 670.5017 | 0.00% | 0.00% |
| **PG 28:0** | 684.4810 | 0.00% | 0.00% |
| PG 29:0 | 698.4966 | 0.00% | 0.00% |
| PG 30:1 | 710.4966 | 0.00% | 0.00% |
| PG 30:0 | 712.5123 | 10.06% | 0.00% |
| PG P-32:1; PG-31:2 | 722.5330 | 0.00% | 0.00% |
| PG P-32:0; PG-31:1 | 724.5123 | 10.74% | 0.00% |
| PG O-32:0; PG-31:0 | 726.5279 | 10.75% | 0.00% |
| PG 32:3; PG P-33:2 | 734.4966 | 0.00% | 0.00% |
| PG 32:2; PG P-33:1 | 736.5123 | 10.94% | 0.00% |
| PG 32:1; PG P-33:0 | 738.5279 | 10.96% | 0.00% |
| PG 32:0; PG O-33:0 | 740.5436 | 10.97% | 0.00% |
| PG P-34:1; PG 33:2 | 750.5279 | 0.00% | 0.00% |
| PG P-34:0; PG 33:1 | 752.5436 | 11.70% | 0.00% |
| PG O-34:0;PG 33:0 | 754.5592 | 11.71% | 0.00% |
| PG 34:4; PG P-35:3 | 760.5123 | 0.00% | 0.00% |
| PG 34:3; PG P-35:2 | 762.5279 | 11.89% | 0.00% |
| PG 34:2; PG P-35:1 | 764.5436 | 11.90% | 0.00% |

(continued)

| Lipid | m/z | Isotopic correction | |
| --- | --- | --- | --- |
| | | M+2 | M+1 |
| PG 34:1; PG P-35:0 | 766.5592 | 11.91% | 0.00% |
| PG 34:0; PG P-36:6 | 768.5749 | 11.92% | 0.00% |
| PG P-36:5; PG 35:6 | 770.4967 | 12.65% | 0.00% |
| PG P-36:4; PG 35:5 | 772.5123 | 12.66% | 0.00% |
| PG P-36:3; PG 35:4 | 774.5279 | 12.67% | 0.00% |
| PG P-36:2; PG 35:3 | 776.5436 | 12.68% | 0.00% |
| PG P-36:1; PG 35:2 | 778.5592 | 12.69% | 0.00% |
| PG P-36:0; PG 35:1 | 780.5749 | 12.70% | 0.00% |
| PG 36:7; PG O-36:0; PG 35:0 | 782.5905 | 12.72% | 0.00% |
| PG 36:6; PG P-37:5 | 784.5123 | 12.86% | 0.00% |
| PG 36:5; PG P-37:4 | 786.5279 | 12.87% | 0.00% |
| PG 36:4; PG P-37:3 | 788.5436 | 12.88% | 0.00% |
| PG 36:3; PG P-37:2 | 790.5592 | 12.90% | 0.00% |
| PG 36:2; PG P-37:1 | 792.5749 | 12.91% | 0.00% |
| PG 36:1; PG P-37:0 | 794.5905 | 12.92% | 0.00% |
| PG 36:0; PG P-38:6 | 796.6062 | 12.93% | 0.00% |
| PG P-38:5; PG 37:6 | 798.5273 | 13.70% | 0.00% |
| PG P-38:4; PG 37:5 | 800.5430 | 13.71% | 0.00% |
| PG P-38:3; PG 37:4 | 802.5586 | 13.72% | 0.00% |
| PG P-38:2; PG 37:3 | 804.5749 | 13.73% | 0.00% |
| PG P-38:1; PG 37:2 | 806.5905 | 13.75% | 0.00% |
| PG P-38:0; PG 37:1 | 808.6062 | 13.76% | 0.00% |
| PG 38:7; PG O-38:0; PG 37:0 | 810.6218 | 13.77% | 0.00% |
| PG 38:6; PG P-39:5 | 812.5436 | 13.91% | 0.00% |
| PG 38:5; PG P-39:4 | 814.5593 | 13.92% | 0.00% |
| PG 38:4; PG P-40:10; PG P-29:3 | 816.5749 | 13.94% | 0.00% |
| PG 38:3; PG P-40:9; PG P-39:2 | 818.5905 | 13.95% | 0.00% |
| PG 38:2; PG P-40:8; PG P-39:1 | 820.6062 | 13.96% | 0.00% |
| PG 38:1; PG P-40:7; PG P-39:0 | 822.6218 | 13.97% | 0.00% |
| PG 38:0; PG P-40:6, PG O-39:0 | 824.6375 | 13.98% | 0.00% |
| PG P-40:5; PG 39:6 | 826.5593 | 14.80% | 0.00% |
| PG P-40:4; PG 39:5 | 828.5749 | 14.81% | 0.00% |
| PG P-40:3; PG 39:4 | 830.5905 | 14.82% | 0.00% |
| PG 40:10; PG P-40:2; PG 39:3 | 832.5123 | 14.84% | 0.00% |
| PG 40:9; PG P-40:1; PG 39:2 | 834.5280 | 14.98% | 0.00% |
| PG 40:8; PG P-40:0; PG 39:1 | 836.5436 | 14.99% | 0.00% |
| PG 40:7; PG O-40:0; PG 39:0 | 838.5592 | 15.00% | 0.00% |
| PG 40:6; PG P-41:5 | 840.5749 | 15.01% | 0.00% |
| PG 40:5; PG P-41:4 | 842.5906 | 15.03% | 0.00% |
| PG 40:4; PG P-42:10; PG P-41:3 | 844.6062 | 15.04% | 0.00% |
| PG 40:3; PG P-42:9; PG P-41:2 | 846.6219 | 15.05% | 0.00% |
| PG 40:2; PG P-42:8; PG P-41:1 | 848.6375 | 15.06% | 0.00% |
| PG 40:1; PG P-42:7; PG P-41:0 | 850.6531 | 15.07% | 0.00% |
| PG 40:0; PG P-42:6, PG O-41:0 | 852.6688 | 15.09% | 0.00% |
| PG P-42:5; PG 41:6 | 854.5905 | 15.96% | 0.00% |
| PG P-42:4; PG 41:5 | 856.6062 | 15.97% | 0.00% |
| PG P-42:3; PG 41:4 | 858.6218 | 15.99% | 0.00% |
| PG 42:10; PG P-42:2; PG 41:3 | 860.5436 | 16.00% | 0.00% |

(continued)

| Lipid | m/z | Isotopic correction | |
|---|---|---|---|
| | | M+2 | M+1 |
| PG 42:9; PG P-42:1; PG 41:2 | 862.5593 | 16.14% | 0.00% |
| PG 42:8; PG P-42:0; PG 41:1 | 864.5749 | 16.15% | 0.00% |
| PG 42:7; PG O-42:0 | 866.5905 | 16.16% | 0.00% |
| PG 42:6; PG P-43:5 | 868.6062 | 16.17% | 0.00% |
| PG 42:5; PG P-43:4 | 870.6218 | 16.19% | 0.00% |
| PG 42:4; PG P-43:3 | 872.6375 | 16.20% | 0.00% |
| PG 42:3; PG P-43:2 | 874.6531 | 16.21% | 0.00% |
| PG 42:2; PG P-43:1 | 876.6688 | 16.23% | 0.00% |
| PG 42:1; PG P-43:0 | 878.6844 | 16.24% | 0.00% |
| PG 42:0; PG O-43:0 | 880.7001 | 16.25% | 0.00% |
| PG 44:0 | 908.7314 | 0.00% | 0.00% |

* mass-to-charge (m/z) of $[M+H]^+$; $[M+Na]^+$; $[M+NH_4]^+$; or $[M+NH_4-H_2O]^+$ depending on individual lipid (sub)class.

Table 6. Database of lipid species monitored during the identification step in the negative-ion mode together with the calculation of isotopic correction for M+1 and M+2 isotopic peaks (so called deisotoping).

| Lipid | m/z | Isotopic correction | |
|---|---|---|---|
| | | M+2 | M+1 |
| SulfoHexCer 34:1 | 778.5145 | 0.00% | 0.00% |
| SulfoHexCer 34:2 (OH) | 792.4937 | 0.00% | 0.00% |
| SulfoHexCer 35:1 | 792.5301 | 0.00% | 0.00% |
| SulfoHexCer 34:1 (OH) | 794.5094 | 17.5% | 0.00% |
| SulfoHexCer 35:0 | 794.5458 | 17.5% | 0.00% |
| SulfoHexCer 34:0 (OH) | 796.5250 | 17.51% | 0.00% |
| SulfoHexCer 36:2 | 804.5301 | 0.00% | 0.00% |
| SulfoHexCer 36:1 | 806.5458 | 18% | 0.00% |
| SulfoHexCer 36:2 (OH) | 820.5250 | 0.00% | 0.00% |
| SulfoHexCer 37:1 | 820.5614 | 0.00% | 0.00% |
| SulfoHexCer 36:1 (OH) | 822.5407 | 18.55% | 0.00% |
| SulfoHexCer 38:2 | 832.5614 | 0.00% | 0.00% |
| SulfoHexCer 38:1 | 834.5771 | 19.01% | 0.00% |
| SulfoHexCer 39:1 | 848.5927 | 0.00% | 0.00% |
| SulfoHexCer 38:1 (OH) | 850.5720 | 19.65% | 0.00% |
| SulfoHexCer 40:2 | 860.5927 | 0.00% | 0.00% |
| SulfoHexCer 40:1 | 862.6084 | 20.19% | 0.00% |
| SulfoHexCer 39:1 (OH) | 864.5876 | 20.21% | 0.00% |
| SulfoHexCer 41:2 | 874.6084 | 0.00% | 0.00% |
| SulfoHexCer 40:2 (OH) | 876.5876 | 20.78% | 0.00% |
| SulfoHexCer 41:1 | 876.6240 | 20.78% | 0.00% |
| SulfoHexCer 40:1 (OH) | 878.6033 | 20.79% | 0.00% |
| SulfoHexCer 40:0 (OH) | 880.6189 | 20.44% | 0.00% |
| SulfoHexCer 42:4 | 884.5927 | 0.00% | 0.00% |
| SulfoHexCer 42:3 | 886.6084 | 21.34% | 0.00% |
| SulfoHexCer 42:2 | 888.6240 | 21.35% | 0.00% |
| SulfoHexCer 41:2 (OH) | 890.6033 | 21.36% | 0.00% |
| SulfoHexCer 42:1 | 890.6397 | 21.36% | 0.00% |
| SulfoHexCer 41:1 (OH) | 892.6189 | 21.37% | 0.00% |
| SulfoHexCer 41:0 (OH) | 894.6346 | 21.02% | 0.00% |

(continued)

| Lipid | m/z | Isotopic correction | |
|---|---|---|---|
| | | M+2 | M+1 |
| SulfoHexCer 40:0 (2OH) | 896.6138 | 21.03% | 0.00% |
| SulfoHexCer 42:3 (OH) | 902.6033 | 0.00% | 0.00% |
| SulfoHexCer 43:2 | 902.6397 | 0.00% | 0.00% |
| SulfoHexCer 42:2 (OH) | 904.6189 | 21.97% | 0.00% |
| SulfoHexCer 43:1 | 904.6553 | 21.97% | 0.00% |
| SulfoHexCer 42:1 (OH) | 906.6346 | 21.98% | 0.00% |
| SulfoHexCer 42:0 (OH) | 908.6502 | 21.61% | 0.00% |
| SulfoHexCer 41:0 (2OH) | 910.6295 | 21.62% | 0.00% |
| SulfoHexCer 44:3 | 914.6397 | 0.00% | 0.00% |
| SulfoHexCer 44:2 | 916.6553 | 22.57% | 0.00% |
| SulfoHexCer 43:2 (OH) | 918.6346 | 22.58% | 0.00% |
| SulfoHexCer 44:1 | 918.6710 | 22.58% | 0.00% |
| SulfoHexCer 43:1 (OH) | 920.6502 | 22.59% | 0.00% |
| SulfoHexCer 42:1 (2OH) | 922.6295 | 22.21% | 0.00% |
| SulfoHexCer 43:0 (OH) | 922.6659 | 22.21% | 0.00% |
| SulfoHexCer 42:0 (2OH) | 924.6451 | 22.22% | 0.00% |
| SulfoHexCer 44:2(OH) | 932.6502 | 0.00% | 0.00% |
| SulfoHexCer 44:1(OH) | 934.6659 | 22.81% | 0.00% |
| SulfoHexCer 43:1 (2OH) | 936.6451 | 22.82% | 0.00% |
| SulfoHexCer 44:0 (OH) | 936.6815 | 22.82% | 0.00% |
| SulfoHexCer 43:0 (2OH) | 938.6608 | 22.83% | 0.00% |
| SulfoHex$_2$Cer 32:1 | 912.5360 | 0.00% | 0.00% |
| SulfoHex$_2$Cer 33:1 | 926.5516 | 0.00% | 0.00% |
| SulfoHex$_2$Cer 34:2 | 938.5516 | 0.00% | 0.00% |
| SulfoHex$_2$Cer 34:1 | 940.5673 | 21.31% | 0.00% |
| SulfoHex$_2$Cer 35:1 | 954.5829 | 0.00% | 0.00% |
| SulfoHex$_2$Cer 34:1 (OH) | 956.5622 | 21.9% | 0.00% |
| SulfoHex$_2$Cer 36:2 | 966.5829 | 21.9% | 0.00% |
| SulfoHex$_2$Cer 36:1 | 968.5986 | 22% | 0.00% |
| SulfoHex$_2$Cer 37:1 | 982.6142 | 0.00% | 0.00% |
| SulfoHex$_2$Cer 36:1 (OH) | 984.5935 | 23.1% | 0.00% |
| SulfoHex$_2$Cer 38:2 | 994.6142 | 0.00% | 0.00% |
| SulfoHex$_2$Cer 38:1 | 996.6299 | 23.71% | 0.00% |
| SulfoHex$_2$Cer 39:1 | 1010.6455 | 0.00% | 0.00% |
| SulfoHex$_2$Cer 38:1 (OH) | 1012.6248 | 24.35% | 0.00% |
| SulfoHex$_2$Cer 40:3 | 1020.6299 | 0.00% | 0.00% |
| SulfoHex$_2$Cer 40:2 | 1022.6455 | 24.96% | 0.00% |
| SulfoHex$_2$Cer 40:1 | 1024.6612 | 24.97% | 0.00% |
| SulfoHex$_2$Cer 41:3 | 1034.6455 | 24.97% | 0.00% |
| SulfoHex$_2$Cer 41:2 | 1036.6612 | 24.98% | 0.00% |
| SulfoHex$_2$Cer 40:2 (OH) | 1038.6404 | 24.99% | 0.00% |
| SulfoHex$_2$Cer 41:1 | 1038.6768 | 24.99% | 0.00% |
| SulfoHex$_2$Cer 40:1 (OH) | 1040.6561 | 25% | 0.00% |
| SulfoHex$_2$Cer 40:0 (OH) | 1042.6717 | 25.1% | 0.00% |
| SulfoHex$_2$Cer 42:4 | 1046.6455 | 0.00% | 0.00% |
| SulfoHex$_2$Cer 42:3 | 1048.6612 | 26.27% | 0.00% |
| SulfoHex$_2$Cer 42:2 | 1050.6768 | 26.28% | 0.00% |
| SulfoHex$_2$Cer 42:1 | 1052.6925 | 26.28% | 0.00% |

(continued)

| Lipid | m/z | Isotopic correction | |
| --- | --- | --- | --- |
| | | M+2 | M+1 |
| SulfoHex$_2$Cer 42:3 (OH) | 1064.6561 | 0.00% | 0.00% |
| SulfoHex$_2$Cer 43:2 | 1064.6925 | 0.00% | 0.00% |
| SulfoHex$_2$Cer 42:2 (OH) | 1066.6717 | 26.98% | 0.00% |
| SulfoHex$_2$Cer 43:1 | 1066.7081 | 26.98% | 0.00% |
| SulfoHex$_2$Cer 42:1 (OH) | 1068.6874 | 26.99% | 0.00% |
| SulfoHex$_2$Cer 42:0 (OH) | 1070.7030 | 27% | 0.00% |
| SulfoHex$_2$Cer 44:4 | 1074.6770 | 0.00% | 0.00% |
| SulfoHex$_2$Cer 44:3 | 1076.6925 | 27.63% | 0.00% |
| SulfoHex$_2$Cer 44:2 | 1078.7081 | 27.64% | 0.00% |
| SulfoHex$_2$Cer 43:2 (OH) | 1080.6874 | 27.65% | 0.00% |
| SulfoHex$_2$Cer 44:1 | 1080.7238 | 27.65% | 0.00% |
| SulfoHex$_2$Cer 42:1 (2*OH) | 1084.6823 | 0.00% | 0.00% |
| SulfoHex$_2$Cer 44:2 (OH) | 1094.7030 | 0.00% | 0.00% |
| SulfoHexNAcHex2Cer 34:1 | 1143.6467 | 0.00% | 0.00% |
| SulfoHexNAcHex2Cer 38:1 | 1199.7093 | 0.00% | 0.00% |
| SulfoHexNAcHex2Cer 40:2 | 1225.7249 | 0.00% | 0.00% |
| SulfoHexNAcHex2Cer 40:1 | 1227.7406 | 31.93% | 0.00% |
| SulfoHexNAcHex2Cer 41:2 | 1239.7406 | 0.00% | 0.00% |
| SulfoHexNAcHex2Cer 41:1 | 1241.7562 | 32.69% | 0.00% |
| SulfoHexNAcHex2Cer 42:4 | 1249.7249 | 0.00% | 0.00% |
| SulfoHexNAcHex2Cer 42:3 | 1251.7406 | 33.43% | 0.00% |
| SulfoHexNAcHex2Cer 42:2 | 1253.7562 | 33.44% | 0.00% |
| SulfoHexNAcHex2Cer 42:1 | 1255.7719 | 33.45% | 0.00% |
| SulfoHexNAcHex2Cer 44:3 | 1279.7719 | 0.00% | 0.00% |
| SulfoHexNAcHex2Cer 44:2 | 1281.7875 | 0.00% | 0.00% |
| SulfoHexNAcHex4Cer 38:1 | 1523.8149 | 0.00% | 0.00% |
| SulfoHexNAcHex4Cer 40:2 | 1549.8306 | 0.00% | 0.00% |
| SulfoHexNAcHex4Cer 40:1 | 1551.8462 | 44.15% | 0.00% |
| SulfoHexNAcHex4Cer 41:2 | 1563.8462 | 0.00% | 0.00% |
| SulfoHexNAcHex4Cer 41:1 | 1565.8619 | 45.05% | 0.00% |
| SulfoHexNAcHex4Cer 42:3 | 1575.8462 | 0.00% | 0.00% |
| SulfoHexNAcHex4Cer 42:2 | 1577.8618 | 45.95% | 0.00% |
| SulfoHexNAcHex4Cer 42:1 | 1579.8775 | 45.97% | 0.00% |
| SulfoHexNAcHex4Cer 42:2 (OH) | 1593.8568 | 0.00% | 0.00% |
| SulfoHexNAcHex4Cer 42:1 (OH) | 1595.8724 | 46.2% | 0.00% |
| SulfoHexNAcHex3Cer 40:1 | 1389.7934 | 0.00% | 0.00% |
| SulfoHexNAcHex3Cer 42:2 | 1415.8090 | 0.00% | 0.00% |
| SulfoHexNAcHex3Cer 42:1 | 1417.8246 | 39.48% | 0.00% |
| SulfoHexNAcHex3Cer 42:2 (OH) | 1431.8039 | 0.00% | 0.00% |
| SulfoHexNAcHex3Cer 42:1 (OH) | 1433.8196 | 39.72% | 0.00% |
| GM3 32:1 | 1123.6746 | 0.00% | 0.00% |
| GM3 34:2 | 1149.6902 | 0.00% | 0.00% |
| GM3 34:1 | 1151.7059 | 24.68% | 0.00% |
| GM3 34:1 (OH) | 1167.7008 | 0.00% | 0.00% |
| GM3 36:2 | 1177.7215 | 0.00% | 0.00% |
| GM3 36:1 | 1179.7372 | 26.1% | 0.00% |
| GM3 36:1 (OH) | 1195.7321 | 0.00% | 0.00% |
| GM3 38:2 | 1205.7528 | 0.00% | 0.00% |

(continued)

| Lipid | m/z | Isotopic correction | |
|---|---|---|---|
| | | M+2 | M+1 |
| GM3 38:1 | 1207.7685 | 27.6% | 0.00% |
| GM3 38:1 (OH) | 1223.7634 | 0.00% | 0.00% |
| GM3 40:2 | 1233.7841 | 0.00% | 0.00% |
| GM3 40:1 | 1235.7998 | 29.13% | 0.00% |
| GM3 41:2 | 1247.7998 | 0.00% | 0.00% |
| GM3 41:1 | 1249.8154 | 29.91% | 0.00% |
| GM3 40:1 (OH) | 1251.7947 | 29.93% | 0.00% |
| GM3 40:0 (OH) | 1253.8104 | 29.38% | 0.00% |
| GM3 42:3 | 1259.7998 | 0.00% | 0.00% |
| GM3 42:2 | 1261.8154 | 30.7% | 0.00% |
| GM3 42:1 | 1263.8311 | 30.71% | 0.00% |
| GM3 42:3 (OH) | 1275.7947 | 0.00% | 0.00% |
| GM3 42:2 (OH) | 1277.8103 | 30.9% | 0.00% |
| GM3 42:1 (OH) | 1279.8260 | 30.95% | 0.00% |
| PS 34:2 | 758.4977 | 0.00% | 0.00% |
| PS 34:1 | 760.5134 | 11.88% | 0.00% |
| PS 36:4 | 782.4977 | 0.00% | 0.00% |
| PS 36:2 | 786.5290 | 0.00% | 0.00% |
| PS 36:1 | 788.5447 | 12.88% | 0.00% |
| PS 38:4 | 810.5290 | 0.00% | 0.00% |
| PS 38:3 | 812.5447 | 13.91% | 0.00% |
| **LPI 16:0 (IS)** | 571.2889 | 0.00% | 0.00% |
| LPI 18:1 | 597.3045 | 0.00% | 0.00% |
| LPI 18:0 | 599.3202 | 6.87% | 0.00% |
| LPI 20:4 | 619.2889 | 0.00% | 0.00% |
| **PI 32:1 (IS)** | 807.5029 | 0.00% | 0.00% |
| PI 32:0 | 809.5185 | 12.88% | 0.00% |
| PI 34:2 | 833.5185 | 0.00% | 0.00% |
| PI 34:1 | 835.5342 | 13.89% | 0.00% |
| PI 34:0 | 837.5498 | 13.9% | 0.00% |
| PI 35:2 | 847.5342 | 0.00% | 0.00% |
| PI 35:1 | 849.5498 | 14.42% | 0.00% |
| PI 36:4 | 857.5185 | 0.00% | 0.00% |
| PI 36:3 | 859.5342 | 14.94% | 0.00% |
| PI 36:2 | 861.5498 | 14.95% | 0.00% |
| PI 36:1 | 863.5655 | 14.96% | 0.00% |
| PI 37:4 | 871.5342 | 0.00% | 0.00% |
| PI 37:3 | 873.5498 | 15.49% | 0.00% |
| PI 38:6 | 881.5185 | 0.00% | 0.00% |
| PI 38:5 | 883.5342 | 16.03% | 0.00% |
| PI 38:4 | 885.5498 | 16.05% | 0.00% |
| PI 38:3 | 887.5655 | 16.06% | 0.00% |
| PI 40:6 | 909.5498 | 0.00% | 0.00% |
| PI 40:5 | 911.5655 | 17.2% | 0.00% |
| PI 40:4 | 913.5811 | 17.21% | 0.00% |
| **PG 32:0 (IS)** | 721.5025 | 0.00% | 0.00% |
| PG 34:4 | 741.4712 | 0.00% | 0.00% |
| PG 34:3 | 743.4869 | 11.7% | 0.00% |

(continued)

| Lipid | m/z | Isotopic correction | |
| --- | --- | --- | --- |
| | | M+2 | M+1 |
| PG 34:2 | 745.5025 | 11.71% | 0.00% |
| PG 34:1 | 747.5181 | 11.72% | 0.00% |
| PG 35:1 | 761.5338 | 0.00% | 0.00% |
| PG 36:4 | 769.5025 | 0.00% | 0.00% |
| PG 36:3 | 771.5181 | 12.69% | 0.00% |
| PG 36:2 | 773.5338 | 11.7% | 0.00% |
| PG 36:1 | 775.5494 | 11.71% | 0.00% |
| PG 38:5 | 795.5181 | 0.00% | 0.00% |
| PG 38:4 | 797.5338 | 13.73% | 0.00% |
| * mass-to-charge (m/z) of [M-H]$^-$. | | | |

*Statistical evaluation*

Measured concentrations of individual lipids of all measured subjects are imported into a statistical software (e.g., SIMCA from Umetrics, Sweden). Proper transformation and scaling are chosen, typically logarithmic transformation and Pareto or UV scaling. The scaling and transformation are based on PCA analysis, where normal distribution of healthy and pancreatic cancer patients is desirable. PCA analysis is also used to find potential outliers, if so, the influence of the outlier on the model is tested (remove the outlier and check the model) and measurement methods are questioned. If a technical problem in case of outlier measurement is identified, then this measurement is removed from the data set. PCA method is used for finding other influential factors, such as gender or age. QC samples should cluster closely together in PCA analysis, typically close to the middle of the PCA graph.

The next step is the use of discrimination analysis (OPLS-DA) for the group separation of pancreatic cancer patients and healthy volunteers separately for males and females. The scaling and transformation is based on PCA results, but the final model has to be found. Models are fitted on all data together and also on separate strata for influential covariates. Different groups of lipids are used to build the model and to explore the influence on the healthy vs. cancer separation. The final model is chosen based on multiple factors as good fit (all known samples are correctly classified), on the model stability (no too influential observations), good prediction ability (by cross-validation performed automatically and then manually with random groups of observations) and biological reasoning and resistance to removing unimportant lipids. The final models are used to identify unknown samples, and the sensitivity and specificity are estimated based on these predictions. For this purpose, the receiver operating characteristic (ROC) curves are plotted. The model is again tested for good prediction ability via the final classification. After this last validation of the model, the most dysregulated lipids are identified using the S-plot or the loading plot. The limits to identify the lipid as dysregulated are the ones from the S-plot with p bigger than 0.1 and pcorr bigger than 0.4. For these most dysregulated lipids, the box-plots comparing the average values in the group of pancreatic cancer and healthy patients for different strata are used to find exact biological interpretation.

*Pancreatic cancer model based on known classification using shotgun and UHPSFC/MS data:*

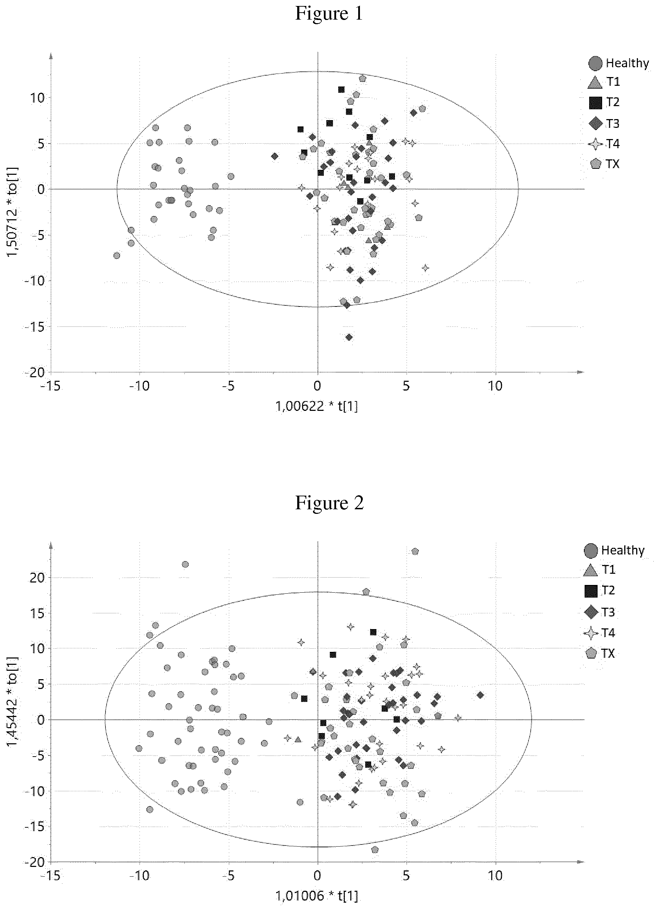

These statistical models are built based on 292 known samples both for shotgun and UHPSFC/MS data sets. First, the non-supervised PCA method is used to check the regular distribution of data, and it already shows partial separation of classes of cancer patients and healthy. Then, supervised OPLS-DA models are prepared separately for males and females. These models included all lipids used (complete lipidome) and are automatically computed by SIMCA software. Figure 1 (males) and Figure 2 (females) show the OPLS-DA statistical models from UHPSFC/MS and shotgun data. This model performed quite well with good fit (1 wrong classified from 292 observations used for model-building) and with good prediction ability estimated by simple cross-validation (1 wrong prediction from 16). It is worth noting that there is the gap between both groups, which visually highlights the reliability of the model for pancreatic cancer classification.

The list of 31 most upregulated and downregulated lipids for males, females, and both groups are shown in Table 7.

Table 7. List of 31 most upregulated and downregulated lipids in human serum of pancreatic cancer patients in comparison to healthy volunteers determined for both genders and separately for males and females.

| Lipid | All | Males | Females |
|---|---|---|---|
| HexCer d18:1/16:0; HexCer d18:1/15:1 (1OH) | up | up | |
| SM 34:1 | | up | |
| HexCer d18:1/24:1; HexCer d18:1/23:2 (1OH) | up | up | |
| PE 34:1; PE P-35:0 | | up | |
| PC 32:0; PC O-33:0 | up | up | |
| PE 36:4; PE P-37:3 | | up | |
| PC 34:1; PC P-35:0 | up | up | |
| PE 34:2; PE P-35:1 | | up | |
| PC P-34:0; PC 33:1 | | up | |
| DG 36:2 | up | up | |
| Cer d18:1/24:1; Cer d18:1/23:2 (1OH) | up | up | |
| CE 16:0 | up | | up |
| SM41:1 | down | down | down |
| SM 41:2 | down | down | down |
| PC 34:4; PC P-35:3 | down | down | |
| HexCer d18:1/24:0; HexCer d18:1/23:1 (1OH) | down | | down |
| PE P-36:3; PE 35:4 | down | | |
| PE P-38:4; PE 37:5 | down | down | down |
| PE P-38:5; PE 37:6 | down | down | |
| PE P-36:2; PE 35:3 | down | | down |
| SM 32:1 | down | | |
| HexCer d18:1/22:0; HexCer d18:1/21:1 (1OH) | down | | |
| PE P-38:3; PE 37:4 | down | | |
| PC P-38:2; PC 37:3 | down | | |
| PE P-40:5; PE 39:6 | down | down | |
| PE 36:0; PE P-38:6 | | down | |
| PE 38:0; PE P-40:6, PE O-39:0 | | down | |
| PE P-36:3; PE 35:4 | | down | down |
| PC P-34:1; PC 33:2 | | | down |
| PC P-36:2; PC 35:3 | | | down |
| DG 34:3 | | | down |

Table 8. Assignment of human male subjects with unknown classification based on 4 statistical models (#1 OPLS-DA of shotgun MS, #2 OPLS-DA of UHPSFC/MS, #3 OPLS-DA of all data, and #4 SVM of all data) and the average of all 4 models used for the final assignment (N = normal, T = tumor, T1, T2, T3, T4 = tumor stages (T1, T2 are early tumor stages), Tx = tumor but stage is not reported). Real state = health state/diagnosis confirmed by standard examination methods (imaging and/or biopsy).

| Sample No. | Final prediction | Real state | Model 1 | Model 2 | Model 3 | Model 4 | Average of 4 models |
|---|---|---|---|---|---|---|---|
| 21 | T | Tx | 1.00 | 1.00 | 1.00 | 0.99 | 1.00 |
| 29 | T | T4 | 0.99 | 1.00 | 1.00 | 1.00 | 1.00 |
| 40 | N | N | 0.19 | 0.23 | 0.23 | 0.08 | 0.18 |
| 44 | N | N | 0.15 | 0.36 | 0.18 | 0.12 | 0.20 |
| 46 | N | N | 0.26 | 0.22 | 0.26 | 0.13 | 0.22 |

(continued)

| Sample No. | Final prediction | Real state | Model 1 | Model 2 | Model 3 | Model 4 | Average of 4 models |
|---|---|---|---|---|---|---|---|
| 50 | N | N | 0.33 | 0.15 | 0.27 | 0.61 | 0.34 |
| 64 | (T) | T4 | 0.31 | 0.89 | 0.40 | 0.66 | 0.57 |
| 89 | T | Tx | 0.76 | 0.70 | 0.80 | 0.89 | 0.79 |
| 106 | T | T3 | 1.00 | 1.00 | 0.99 | 1.00 | 1.00 |
| 110 | T | T3 | 0.66 | 0.44 | 0.58 | 0.96 | 0.66 |
| 115 | N | N | 0.14 | 0.48 | 0.14 | 0.32 | 0.27 |
| 118 | N | N | 0.37 | 0.36 | 0.34 | 0.09 | 0.29 |
| 120 | N | N | 0.11 | 0.42 | 0.11 | 0.08 | 0.18 |
| 133 | T | Tx | 0.95 | 0.73 | 0.91 | 1.00 | 0.90 |
| 142 | T | T3 | 0.97 | 0.81 | 0.95 | 1.00 | 0.93 |
| 149 | N | T2 | 0.37 | 0.39 | 0.38 | 0.27 | 0.35 |
| 166 | N | N | 0.44 | 0.30 | 0.45 | 0.43 | 0.41 |
| 168 | N | N | 0.21 | 0.48 | 0.23 | 0.19 | 0.28 |
| 173 | N | N | 0.09 | 0.46 | 0.15 | 0.03 | 0.18 |
| 177 | (N) | N | 0.54 | 0.38 | 0.59 | 0.28 | 0.45 |
| 184 | T | T1 | 0.87 | 0.93 | 0.90 | 0.95 | 0.91 |
| 194 | T | Tx | 0.82 | 1.00 | 0.89 | 0.99 | 0.93 |
| 203 | T | T3 | 1.00 | 0.83 | 1.00 | 1.00 | 0.96 |
| 247 | T | Tx | 0.62 | 0.92 | 0.61 | 0.73 | 0.72 |
| 251 | T | T3 | 1.00 | 0.65 | 1.00 | 0.99 | 0.91 |
| 255 | T | T4 | 0.81 | 0.97 | 0.89 | 0.98 | 0.91 |
| 258 | T | Tx | 1.00 | 1.00 | 1.00 | 1.00 | 1.00 |
| 264 | T | T2 | 0.68 | 0.69 | 0.60 | 0.84 | 0.70 |
| 267 | T | Tx | 1.00 | 1.00 | 1.00 | 1.00 | 1.00 |
| 271 | T | T4 | 1.00 | 1.00 | 1.00 | 1.00 | 1.00 |
| 275 | T | T3 | 0.98 | 1.00 | 1.00 | 1.00 | 1.00 |
| 305 | T | T4 | 0.92 | 0.96 | 1.00 | 1.00 | 0.97 |
| 310 | T | Tx | 0.83 | 0.81 | 0.85 | 1.00 | 0.87 |
| 317 | T | Tx | 1.00 | 1.00 | 1.00 | 1.00 | 1.00 |
| 322 | T | T3 | 1.00 | 1.00 | 1.00 | 1.00 | 1.00 |
| 347 | T | T3 | 1.00 | 0.71 | 1.00 | 0.97 | 0.92 |
| 351 | T | T3 | 0.85 | 0.74 | 0.90 | 0.95 | 0.86 |
| 355 | T | Tx | 0.94 | | 0.73 | | 0.84 |

Table 9. Assignment of human female subjects with unknown classification based on 4 statistical models (#1 OPLS-DA of shotgun MS, #2 OPLS-DA of UHPSFC/MS, #3 OPLS-DA of all data, and #4 SVM of all data) and the average of all 4 models used for the final assignment (N = normal, T = tumor, T1, T2, T3, T4 = tumor stages, Tx = tumor but stage is not reported). Real state = health state/diagnosis confirmed by standard examination methods (imaging and/or biopsy).

| Sample No. | Final prediction | Real state | Model | Model 2 | Model 3 | Model 4 | Average of 4 models |
|---|---|---|---|---|---|---|---|
| 17 | T | T1 | 0.89 | 1.00 | 0.88 | 1.00 | 0.94 |
| 25 | T | T4 | 1.00 | 1.00 | 1.00 | 1.00 | 1.00 |
| 34 | T | T3 | 1.00 | 0.98 | 1.00 | 0.99 | 0.99 |
| 55 | T | T4 | 1.00 | 0.90 | 1.00 | 1.00 | 0.97 |
| 68 | T | Tx | 0.92 | 1.00 | 1.00 | 1.00 | 0.98 |
| 73 | T | T3 | 0.85 | 1.00 | 0.89 | 0.99 | 0.93 |
| 78 | T | Tx | 0.82 | 0.71 | 0.68 | 0.95 | 0.79 |

(continued)

| Sample No. | Final prediction | Real state | Model | Model 2 | Model 3 | Model 4 | Average of 4 models |
|---|---|---|---|---|---|---|---|
| 83 | T | T3 | 1.00 | 1.00 | 1.00 | 1.00 | 1.00 |
| 93 | T | T4 | 1.00 | 1.00 | 1.00 | 1.00 | 1.00 |
| 98 | T | T3 | 1.00 | 1.00 | 1.00 | 1.00 | 1.00 |
| 104 | N | Tx | 0.16 | 0.52 | 0.40 | 0.23 | 0.33 |
| 125 | T | T3 | 1.00 | 0.75 | 1.00 | 1.00 | 0.94 |
| 130 | T | T3 | 0.94 | 1.00 | 1.00 | 0.86 | 0.95 |
| 157 | T | T4 | 0.61 | 0.49 | 0.51 | 0.96 | 0.64 |
| 161 | T | Tx | 0.92 | 0.68 | 0.72 | 0.88 | 0.80 |
| 181 | T | Tx | 0.96 | 1.00 | 1.00 | 0.96 | 0.98 |
| 190 | T | T3 | 0.65 | 0.97 | 0.67 | 0.56 | 0.71 |
| 199 | N | T2 | 0.47 | 0.52 | 0.44 | 0.11 | 0.38 |
| 208 | T | T4 | 0.76 | 0.73 | 0.96 | 0.97 | 0.85 |
| 218 | T | T2 | 0.98 | 0.87 | 0.89 | 1.00 | 0.93 |
| 226 | N | N | 0.13 | 0.32 | 0.22 | 0.21 | 0.22 |
| 230 | N | N | 0.25 | 0.36 | 0.25 | 0.02 | 0.22 |
| 235 | N | N | 0.36 | 0.00 | 0.31 | 0.07 | 0.19 |
| 239 | (N) | N | 0.64 | 0.30 | 0.48 | 0.40 | 0.46 |
| 280 | (T) | T3 | 0.33 | 0.70 | 0.47 | 0.56 | 0.51 |
| 283 | N | N | 0.41 | 0.19 | 0.30 | 0.59 | 0.37 |
| 288 | N | N | 0.16 | 0.55 | 0.35 | 0.22 | 0.32 |
| 292 | N | N | 0.15 | 0.00 | 0.00 | 0.03 | 0.04 |
| 293 | N | N | 0.00 | 0.02 | 0.00 | 0.01 | 0.01 |
| 296 | N | N | 0.27 | 0.19 | 0.20 | 0.13 | 0.20 |
| 327 | N | N | 0.13 | 0.08 | 0.11 | 0.21 | 0.13 |
| 331 | T | N | 0.76 | 0.33 | 0.73 | 0.82 | 0.66 |
| 335 | N | N | 0.00 | 0.00 | 0.00 | 0.00 | 0.00 |
| 344 | T | Tx | 1.00 | 1.00 | 1.00 | 0.99 | 1.00 |
| 359 | T | Tx | 0.59 | 0.64 | 0.61 | 0.54 | 0.59 |

*Calculation of sample throughput for early screening and monitoring of therapy of pancreatic cancer patients based on the lipidomic analysis of human serum*

Calculation for 100 samples of human serum

Instrumentation:

- 1 MS system (shotgun MS, UHPSFC/MS, or MALDI-MS)
- laboratory for sample preparation in Biological Safety Level (BSL) 2 regime

Time:

Table 10. Time calculation of individual steps in our methodology for high-throughput lipidomic quantitation

| Step | Time [days] | Comments |
|---|---|---|
| Sample preparation (liquid/liquid extraction) | 1.5 | 10-12 hours for 1 person |

(continued)

| Step | Time [days] | Comments |
|---|---|---|
| Analysis (including continuous measurement control) | 2.5 | 2 injections = 40 samples/24 hours, 1 injection = 80 (possible 2. injection in case of positive results or ambiguity) |
| Data processing (noise reduction and data conversion) | 1 | In fact, it is 2.5 days, but it runs in parallel with the analysis |
| Quantitation using Microsoft Excel script | 0.75 | |
| Multivariate data analysis (MDA) | 0.25 | |
| Spare time for unexpected circumstances | 1 | |
| TOTAL | 7 | |

Calculation for 1000 samples:

$7 * 10 = 70$ working days - reduction due to some multiplexing and possible over weekend automated operation = ca. 3 months

How many samples per year for 1 MS system:

$$1000 \text{ (per 3 months)} * 4 = \text{ca. } 4000 \text{ samples/year}$$

Further automation of sample preparation, data processing, multiplexing of tasks, and shortening the analysis time could further increase the sample throughput at least two times.

Industrial Applicability

The method of the invention can be used for population screening of the whole population or selected population groups based on risk factors such as age, gender, body-mass-index, genetic predispositions, risk behavior, etc. The subjects having the probability of suffering from pancreatic cancer above a predetermined threshold can then be subjected to further examinations and tests (e.g., computer tomography or other advanced imaging methods). As the present screening method is non-invasive, can be performed in a high-throughput mode, and can detect early stages of pancreatic cancer, it is the first known method suitable for routine screening of pancreatic cancer. The analytical methodology is fully validated in line with recommendations of authoritative organizations, such Food and Drug Administration or European Medicines Agency (EMEA).

**Claims**

1. A method of diagnosing pancreatic cancer based on lipidomic analysis of a serum taken from the body of a human patient, **characterized in that** it comprises the steps of:

    - spiking of the sample with a set of internal standards comprising at least one internal standard for each lipid class present in the sample,
    - subsequently processing the sample by liquid-liquid lipidomic extraction,
    - measurement of the processed sample by a mass spectrometry method,
    - determining concentrations for at least 60, preferably at least 120 or at least 230, more preferably for all lipids present at a level above detection threshold of the mass spectrometry method, using the internal standards for the corresponding lipid classes for the quantitation, or using relative concentrations of lipids, or using ratios of concentrations and/or signal intensities,

wherein the lipids for which the concentrations are determined include the following lipids:

HexCer d18:1/16:0; HexCer d18:1/15:1 (1OH)
SM 34:1
HexCer d18:1/24:1; HexCer d18:1/23:2 (1OH)
PE 34:1; PE P-35:0
PC 32:0; PC O-33:0
PE 36:4; PE P-37:3
PC 34:1; PC P-35:0
PE 34:2; PE P-35:1
PC P-34:0; PC 33:1
DG 36:2
Cer d18:1/24:1; Cer d18:1/23:2 (1OH)
CE 16:0
SM 41:1
SM 41:2
PC 34:4; PC P-35:3
HexCer d18:1/24:0; HexCer d18:1/23:1 (1OH)
PE P-36:3; PE 35:4
PE P-38:4; PE 37:5
PE P-38:5; PE 37:6
PE P-36:2; PE 35:3
SM 32:1
HexCer d18:1/22:0; HexCer d18:1/21:1 (1OH)
PE P-38:3; PE 37:4
PC P-38:2; PC 37:3
PE P-40:5; PE 39:6
PE 36:0; PE P-38:6
PE 38:0; PE P-40:6, PE O-39:0
PE P-36:3; PE 35:4
PC P-34:1; PC 33:2

- statistical evaluation of the determined concentrations of the lipids, said statistical evaluation determining the level of probability of the patient suffering from pancreatic cancer based on the determined lipid concentrations compared to a cancerous pattern and a non-cancerous pattern, wherein the cancerous pattern and the non-cancerous pattern are determined by statistical analysis of the lipid concentrations for a group of known cancer samples and non-cancer samples, wherein the statistical evaluation is done separately for males and females.

2. The method according to claim 1, wherein the internal standards are exogenous lipid compounds, having the polar head structure typical for the relevant lipid class and containing fatty acyls with shorter chains than naturally occurring lipids, preferably chains 12:0 or 14:0, or fatty acyls with odd number of carbon atoms, preferably chains 17:0, 17:1 or 19:1, or the internal standards are isotopically labelled analogues of the lipids of the relevant lipid class, preferably D7-Chol, D7-CE 16:0; wherein the notation of lipid compounds is as follows: abbreviation of the class of the compounds, number of carbons: number of double bonds, preceded by information about isotopic labeling.

3. The method according to any one of the preceding claims, wherein a set of internal standards contains:

| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

wherein the notation of lipid compounds is as follows: abbreviation of the class of the compounds, number of carbons: number of double bonds, preceded by information about isotopic labeling.

4. The method according to any one of the preceding claims, wherein the mass spectrometry method is selected from shotgun mass spectrometry, ultrahigh-performance liquid chromatography - mass spectrometry, ultrahigh-performance supercritical fluid chromatography - mass spectrometry, and matrix-assisted laser desorption/ionization mass spectrometry.

5. The method according to any one of the preceding claims, wherein a pooled sample is prepared by mixing identical volumes of several samples which include samples from pancreatic cancer patients and healthy volunteers, and said pooled sample is used and processed in the same way as the measured samples; preferably the pooled sample is used for observing intra-day accuracy and intra-day precision, as well as inter-day accuracy and inter-day precision, and/or for determining the lower limit of quantitation and the upper limit of quantitation, and/or for quality control during measurements of sample set.

6. The method according to any one of the preceding claims, wherein the order of samples is randomized in sample measurement sequences.

7. The method according to any one of the preceding claims, wherein the step of determining concentrations for all lipids present at a level above lower limit of quantitation of the mass spectrometry method, using the internal standards for the corresponding lipid classes for the quantitation, comprises one or more of the following procedures and corrections:

   - isotopic correction,
   - zero filling procedure in which the signals of lipid species which are not detected for particular sample are replaced by 50 to 100 %, preferably by 70 to 100 %, more preferably by 80 to 90 %, or by 80 %, of the minimum concentration observed for said lipid species in all samples.

8. The method according to any one of the preceding claims, wherein the statistical evaluation involves

   - data pre-processing such as centering, scaling, and/or transformation;
   - principal component analysis (PCA) for identification of influential factors such as outliers or measurement failures,
   - discrimination analysis by orthogonal projections to latent structures discriminant analysis (OPLS-DA) for group separation of pancreatic cancer patients and healthy volunteers, performed for males and females separately,
   - assignment of the statistical parameters as well as the evaluation of the prediction power.

**Patentansprüche**

1. Ein Verfahren zur Diagnose von Bauchspeicheldrüsenkrebs mithilfe einer Lipidomanalyse eines Serums, das aus dem Körper eines menschlichen Patienten entnommen wurde, wobei das Verfahren **dadurch gekennzeichnet ist, dass** es die folgenden Schritte umfasst:

   - Dotierung der Probe mit einem Satz interner Standards, die mindestens einen internen Standard für jede in der Probe vorhandene Lipidklasse umfassen;
   - anschließende Verarbeitung der Probe durch lipidomische Flüssig-Flüssig-Extraktion,
   - Messung der verarbeiteten Probe mittels Massenspektrometrie,
   - Bestimmen von Konzentrationen für mindestens 60, vorzugsweise mindestens 120 oder mindestens 230, bevorzugter für alle Lipide, die auf einem Niveau oberhalb der Nachweisschwelle des Massenspektrometrieverfahrens vorhanden sind, unter Verwendung der internen Standards für die entsprechenden Lipidklassen für die Quantifizierung oder unter Verwendung relative Konzentrationen von Lipiden oder unter Verwendung von Verhältnissen von Konzentrationen und/oder Signalintensitäten,

   wobei die Lipide, für die die Konzentrationen bestimmt werden, die folgenden Lipide umfassen:

HexCer d18:1/16:0; HexCer d18:1/15:1 (1OH)

SM 34:1

HexCer d18:1/24:1; HexCer d18:1/23:2 (1OH)

PE 34:1; PE P-35:0

PC 32:0; PC O-33:0

PE 36:4; PE P-37:3

PC 34:1; PC P-35:0

PE 34:2; PE P-35:1

PC P-34:0; PC 33:1

DG 36:2

Cer d18:1/24:1; Cer d18:1/23:2 (1OH)

CE 16:0

SM 41:1

SM 41:2

PC 34:4; PC P-35:3

HexCer d18:1/24:0; HexCer d18:1/23:1 (1OH)

PE P-36:3; PE 35:4

PE P-38:4; PE 37:5

PE P-38:5; PE 37:6

PE P-36:2; PE 35:3

SM 32:1

HexCer d18:1/22:0; HexCer d18:1/21:1 (1OH)

PE P-38:3; PE 37:4

PC P-38:2; PC 37:3

PE P-40:5; PE 39:6

PE 36:0; PE P-38:6

PE 38:0; PE P-40:6, PE O-39:0

PE P-36:3; PE 35:4

PC P-34:1; PC 33:2

PC P-36:2; PC 35:3

DG 34:3

- statistische Auswertung der bestimmten Konzentrationen der Lipide, wobei die statistische Auswertung das Wahrscheinlichkeitsniveau des an Bauchspeicheldrüsenkrebs leidenden Patienten auf der Grundlage der bestimmten Lipidkonzentrationen im Vergleich zu einem Krebsmuster und einem nicht-krebsartigen Muster bestimmt, wobei das Krebsmuster und das nicht-krebsartige Muster wird durch statistische Analyse der Lipidkonzentrationen für eine Gruppe bekannter Krebsproben und nichtkrebsartiger Proben bestimmt, wobei die statistische Auswertung für Männer und Frauen getrennt durchgeführt wird.

2. Verfahren nach Anspruch 1, wobei die internen Standards exogene Lipidverbindungen sind, die für die relevante Lipidklasse typische polare Kopfstruktur aufweisen und Fettacyle mit kürzeren Ketten als natürlich vorkommende Lipide, vorzugsweise Ketten 12:0 oder 14:0, oder Fettacyle mit einer ungeraden Anzahl von Kohlenstoffatomen, vorzugsweise Ketten 17:0, 17:1 oder 19:1, enthalten; oder die internen Standards sind isotopenmarkierte Analoga der Lipide der relevanten Lipidklasse, vorzugsweise D7-Chol, D7-CE 16:0; wobei die Notation der Lipidverbindungen wie folgt lautet: Abkürzung der Klasse der Verbindungen, Anzahl der Kohlenstoffe:Anzahl der Doppelbindungen, vorangestellt von Informationen über die Isotopenmarkierung.

3. Verfahren nach einem der vorhergehenden Ansprüche, wobei ein Satz interner Standards enthält:

| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |

(fortgesetzt)

| | |
|---|---|
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

wobei die Notation der Lipidverbindungen wie folgt lautet: Abkürzung der Klasse der Verbindungen, Anzahl der Kohlenstoffe:Anzahl der Doppelbindungen, vorangestellt von Informationen über die Isotopenmarkierung.

4. Verfahren nach einem der vorhergehenden Ansprüche, wobei das Massenspektrometrieverfahren ausgewählt ist aus Schrotflintenmassenspektrometrie, Ultrahochleistungsflüssigchromatographie - Massenspektrometrie, ultrahochleistungsfähige überkritische Flüssigkeitschromatographie - Massenspektrometrie, und matrixunterstützter Laserdesorption/Ionisationsmassenspektrometrie.

5. Verfahren nach einem der vorhergehenden Ansprüche, wobei eine gepoolte Probe hergestellt wird, indem identische Volumina mehrerer Proben gemischt werden, die Proben von Bauchspeicheldrüsenkrebs und gesunden Freiwilligen enthalten, und die gepoolte Probe auf die gleiche Weise wie die gemessenen Proben verwendet und verarbeitet wird; vorzugsweise wird die gepoolte Probe verwendet zur Beobachtung der Intra-Day-Genauigkeit und Intra-Day-Präzision sowie der Inter-Day-Genauigkeit und Inter-Day-Präzision und/oder zur Bestimmung der unteren Bestimmungsgrenze und der oberen Bestimmungsgrenze und/oder zur Qualitätskontrolle während der Messung des Probensatzes.

6. Verfahren nach einem der vorhergehenden Ansprüche, wobei die Reihenfolge der Proben in Probenmesssequenzen randomisiert ist.

7. Verfahren nach einem der vorhergehenden Ansprüche, wobei der Schritt des Bestimmens der Konzentrationen für alle Lipide, die auf einem Niveau oberhalb der Nachweisschwelle des Massenspektrometrieverfahrens vorhanden sind, unter Verwendung der internen Standards für die entsprechenden Lipidklassen für die Quantifizierung, umfasst eines oder mehrere der folgenden Verfahren und Korrekturen:

- Isotopenkorrektur,
- Nullfüllverfahren, bei dem die Signale von Lipiden, die für eine bestimmte Probe nicht erfasst werden, um 50 bis 100%, vorzugsweise um 70 bis 100%, bevorzugter um 80 bis 90% oder um 80% der Mindestkonzentration beobachtet für diese Lipiden in allen Proben ersetzt werden.

8. Verfahren nach einem der vorhergehenden Ansprüche, wobei die statistische Auswertung umfasst

- Datenvorverarbeitung wie Zentrieren, Skalieren und/oder Transformieren;
- Hauptkomponentenanalyse (PCA) zur Identifizierung von Einflussfaktoren wie Ausreißern oder Messfehlern;
- Diskriminierungsanalyse durch orthogonale Projektionen zur Diskriminanzanalyse latenter Strukturen (OPLS-DA) zur Gruppentrennung von Bauchspeicheldrüsenkrebspatienten und gesunden Freiwilligen, getrennt für Männer und Frauen durchgeführt,
- Zuordnung der statistischen Parameter sowie Auswertung der Vorhersagekraft.

**Revendications**

1. Procédé de diagnostic du cancer du pancréas basé sur l'analyse lipidomique d'un sérum sanguin prélevé sur le corps d'un patient humain, **caractérisé en ce qu'**il comprend les étapes de:

- enrichissement de l'échantillon avec un ensemble d'étalons internes comprenant au moins un étalon interne pour chaque classe de lipides présente dans l'échantillon,
- traitement de l'échantillon par extraction lipidomique liquide-liquide,
- mesure de l'échantillon par une méthode de spectrométrie de masse,

- déterminer des concentrations pour au moins 60, de préférence au moins 120 ou au moins 230, plus préférentiellement pour tous les lipides présents à un niveau supérieur au seuil de détection de la méthode de spectrométrie de masse, en utilisant les standards internes correspondants aux classes de lipides pour la quantification, ou en utilisant des concentrations relatives de lipides, ou en utilisant des rapports de concentrations et/ou des intensités de signal,

où les lipides pour lesquels les concentrations sont déterminées comprennent les lipides suivants:

HexCer d18:1/16:0; HexCer d18:1/15:1 (1OH)
SM 34:1
HexCer d18:1/24:1; HexCer d18:1/23:2 (1OH)
PE 34:1; PE P-35:0
PC 32:0; PC O-33:0
PE 36:4; PE P-37:3
PC 34:1; PC P-35:0
PE 34:2; PE P-35:1
PC P-34:0; PC 33:1
DG 36:2
Cer d18:1/24:1; Cer d18:1/23:2 (1OH)
CE 16:0
SM 41:1
SM 41:2
PC 34:4; PC P-35:3
HexCer d18:1/24:0; HexCer d18:1/23:1 (1OH)
PE P-36:3; PE 35:4
PE P-38:4; PE 37:5
PE P-38:5; PE 37:6
PE P-36:2; PE 35:3
SM 32:1
HexCer d18:1/22:0; HexCer d18:1/21:1 (1OH)
PE P-38:3; PE 37:4
PC P-38:2; PC 37:3
PE P-40:5; PE 39:6
PE 36:0; PE P-38:6
PE 38:0; PE P-40:6, PE O-39:0
PE P-36:3; PE 35:4
PC P-34:1; PC 33:2
PC P-36:2; PC 35:3
DG 34:3

- évaluation statistique des concentrations déterminées des lipides, ladite évaluation statistique déterminant le niveau de probabilité du patient souffrant d'un cancer du pancréas sur la base des concentrations lipidiques déterminées par rapport à un motif cancéreux et un motif non-cancéreux, dans lequel le motif cancéreux et le motif non-cancéreux sont déterminés par analyse statistique des concentrations lipidiques pour un groupe d'échantillons cancéreux connus et d'échantillons non-cancéreux connus, l'évaluation statistique étant effectuée séparément pour les hommes et les femmes.

2. Procédé selon la revendication 1, où les standards internes sont des composés lipidiques exogènes, ayant la structure de tête polaire typique pour la classe lipidique concernée et contenant des acyles gras avec des chaînes plus courtes que les lipides naturels, de préférence des chaînes 12:0 ou 14:0, ou des acyles gras avec un nombre impair d'atomes de carbone, de préférence des chaînes 17:0, 17:1 ou 19:1, ou les standards internes sont des analogues isotopiquement marqués des lipides de la classe de lipides concernée, de préférence D7-Chol, D7-CE 16:0; où la notation des composés lipidiques est la suivante: abréviation de la classe des composés, nombre de carbones: nombre de doubles liaisons, précédées d'informations sur le marquage isotopique.

**3.** Procédé selon l'une quelconque des revendications précédentes, dans lequel un ensemble d'étalons internes contient:

| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

où la notation des composés lipidiques est la suivante: abréviation de la classe des composés, nombre de carbones: nombre de doubles liaisons, précédées d'informations sur le marquage isotopique.

**4.** Procédé selon l'une quelconque des revendications précédentes, où le procédé de spectrométrie de masse est choisi parmi la spectrométrie de masse shotgun, la chromatographie liquide ultra haute performance - spectrométrie de masse, la chromatographie fluide supercritique ultra haute performance - la spectrométrie de masse et spectrométrie de masse à la désorption/ionisation par laser assistée par matrice.

**5.** Procédé selon l'une quelconque des revendications précédentes, où un échantillon groupé est préparé en mélangeant des volumes identiques de plusieurs échantillons qui comprennent des échantillons de patients atteints d'un cancer du pancréas et de sujets sains, et ledit échantillon groupé est utilisé et traité de la même manière que les échantillons mesurés;
de préférence, l'échantillon groupé est utilisé pour observer l'exactitude intra-journalière et la précision intra-journalière, ainsi que l'exactitude inter-journalière et la précision inter-journalière, et/ou pour déterminer la limite inférieure de quantification et la limite supérieure de quantification, et/ou pour le contrôle de la qualité lors des mesures de l'ensemble d'échantillons.

**6.** Procédé selon l'une quelconque des revendications précédentes, où l'ordre des échantillons est randomisé dans des séquences de mesure d'échantillons.

**7.** Procédé selon l'une quelconque des revendications précédentes, où l'étape de détermination des concentrations pour tous les lipides présents à un niveau supérieur à la limite inférieure de quantification de la méthode de spectrométrie de masse, en utilisant les standards internes correspondants aux classes de lipides pour la quantification, comprend une ou plusieurs des procédures et corrections suivantes:

- correction isotopique,
- procédure de remplissage zéro dans laquelle les signaux des espèces lipidiques qui ne sont pas détectées pour un échantillon particulier sont remplacés par 50 à 100%, de préférence par 70 à 100%, plus préférablement par 80 à 90%, ou par 80%, de la concentration minimale observé pour lesdites espèces lipidiques dans tous les échantillons.

**8.** Procédé selon l'une quelconque des revendications précédentes, où l'évaluation statistique implique

- le prétraitement des données tel que le centrage, la mise à l'échelle et/ou la transformation;
- analyse en composantes principales (ACP) pour l'identification des facteurs influents tels que les valeurs aberrantes ou les échecs de mesure;
- analyse de discrimination par analyse discriminante aux projections orthogonales aux structures latentes (OPLS-DA) pour la séparation de groupe de patients atteints de cancer du pancréas et de sujets sains, réalisée séparément pour les hommes et les femmes;
- l'affectation des paramètres statistiques ainsi que l'évaluation de la puissance de prédiction.

Figure 1

Figure 2

## Figure 3

R2X[1] = 0.0817        R2X[XSide Comp. 1] = 0.524        Ellipse: Hotelling's T2 (95%)

## Figure 4

R2X[1] = 0.0966        R2X[XSide Comp. 1] = 0.563        Ellipse: Hotelling's T2 (95%)

## Figure 5

## Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130140452 A
- WO 2016207391 A
- US 20120202188 A

**Non-patent literature cited in the description**

- **E. CÍFKOVÁ ; M. HOLČAPEK ; M. LÍSA ; D. VRÁNA ; J. GATĚK ; B. MELICHAR.** *Anal. Bioanal. Chem.,* 2015, vol. 407, 991-1002
- **E. CÍFKOVÁ ; M. LÍSA ; R. HRSTKA ; D. VRÁNA ; J. GATĚK ; B. MELICHAR ; M. HOLČAPEK.** *Rapid Commun. Mass Spectrom.,* 2017, vol. 31, 253-263